# EUROPEAN PATENT SPECIFICATION

(11) **EP 3 337 813 B1**
(45) Date of publication and mention of the grant of the patent: **15.04.2020**
(21) Application number: 16760183.0
(22) Date of filing: 17.08.2016
(51) Int. Cl.: C07K 14/14, C07K 14/425, C12N 15/62, A61K 38/16, A61K 39/15, A61K 39/00, A61K 39/12, C07K 14/005, C12N 15/82

(54) **SYNTHETIC BTV VP2 FUSION PROTEIN**
SYNTHETISCHES BTV-VP2-FUSIONSPROTEIN
PROTÉINE DE FUSION VP2 DU VFCO SYNTHÉTIQUE

(30) Priority: 18.08.2015 GB 201514648
(43) Date of publication of application: 27.06.2018
(73) Proprietor: University of Cape Town, Cape Town 7701 (ZA)
(72) Inventor: RYBICKI, Edward Peter, 7405 Cape Town (ZA); MEYERS, Ann Elizabeth, 7700 Plumstead (ZA)
(74) Representative: Appleyard Lees IP LLP
(86) International application number: PCT/IB2016/054918
(87) International publication number: WO 2017/029612

(56) References cited:
- WO-A1-2006/056483
- MOHD JAAFAR FAUZIAH ET AL: "Immunisation with bacterial expressed VP2 and VP5 of bluetongue virus (BTV) protect [alpha]/[beta] interferon-receptor knock-out (IFNAR-/-) mice from homologous", VACCINE, vol. 32, no. 32, 2 June 2014 (2014-06-02), pages 4059-4067, XP028873705, ISSN: 0264-410X, DOI: 10.1016/J.VACCINE.2014.05.056
- CAPOCEFALO A ET AL: "Expression and secretion of Bluetongue virus serotype 8 (BTV-8)VP2 outer capsid protein by mammalian cells", JOURNAL OF VIROLOGICAL METHODS, ELSEVIER BV, NL, vol. 169, no. 2, 1 November 2010 (2010-11-01), pages 420-424, XP027338756, ISSN: 0166-0934 [retrieved on 2010-08-10]
- ANDREW J. CONLEY ET AL: "Protein body-inducing fusions for high-level production and purification of recombinant proteins in plants", PLANT BIOTECHNOLOGY JOURNAL, vol. 9, no. 4, 1 May 2011 (2011-05-01), pages 419-433, XP055006552, ISSN: 1467-7644, DOI: 10.1111/j.1467-7652.2011.00596.x
- MARK WHITEHEAD ET AL: "Human papillomavirus (HPV) type 16 E7 protein bodies cause tumour regression in mice", BMC CANCER, BIOMED CENTRAL, LONDON, GB, vol. 14, no. 1, 24 May 2014 (2014-05-24), page 367, XP021187563, ISSN: 1471-2407, DOI: 10.1186/1471-2407-14-367
- Sandiswa Mbewana ET AL: "Production of H5N1 Influenza Virus Matrix Protein 2 Ectodomain Protein Bodies in Tobacco Plants and in Insect Cells as a Candidate Universal Influenza Vaccine", Frontiers in bioengineering and biotechnology, 8 December 2015 (2015-12-08), page 197, XP055307930, Switzerland DOI: 10.3389/fbioe.2015.00197 Retrieved from the Internet: URL:https://www.ncbi.nlm.nih.gov/pmc/artic les/PMC4672040/pdf/fbioe-03-00197.pdf [retrieved on 2016-10-05]

## Description

### BACKGROUND OF THE INVENTION

This invention relates to a second generation plant-produced bluetongue virus (BTV) candidate vaccine. The vaccine comprises VP2 fusion protein, comprising of a BTV VP2 polypeptide which is fused to a peptide which induces the formation of protein bodies in plant cells. The invention specifically relates to the fusion proteins described herein, methods of producing the fusion proteins in plant cells and pharmaceutical compositions comprising the fusion proteins. More specifically, the invention relates to a BTV vaccine comprising the fusion proteins of the invention and which allow for the distinction between infected and vaccinated animals (DIVA).

The fusion protein of the invention is a plant-produced particulate vaccine comprising a BTV VP2 protein which presents virus-neutralising epitopes in protein bodies. The fusion proteins of the invention may be used to protect subjects against a single BTV serotype, or it can be adapted to produce a plant-produced particulate vaccine that represents several different BTV serotypes, this may be achieved by mixing fusion proteins which present VP2 of different BTV serotypes in a single pharmaceutical composition.

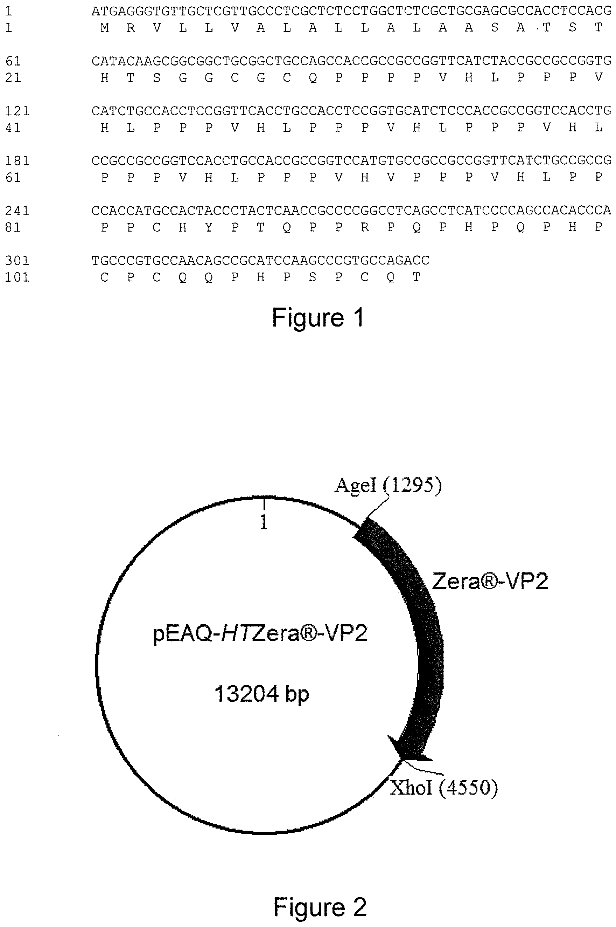

The BTV structural protein VP2 is the major immunogenic determinant of BTV. The present inventors have developed a novel BTV vaccine which presents a serotype-specific VP2 antigenic determinant of BTV to the immune system of a subject and presents it in a particulate form. This significantly increases the extent of the immune response in a subject. The inventors have fused a gene sequence which encodes a signal sequence called Zera® to a plant codon optimised VP2 gene sequence. Zera® is a peptide sequence generated from the maize γ-zein sequence (Figure 1) which has been described as being sufficient to induce retention of recombinant proteins in protein bodies called StorPro® organelles (ERA Biotech, Spain). This phenomenon has been shown with the fusion of several different proteins fused to Zera® including E7 (Whitehead et al., 2014), enhanced green fluorescent protein (eGFP) (Rybicki laboratory), DsRed (Joseph et al., 2012), enhanced cyan fluorescent protein (eCFP) (Torrent et al., 2009a), human growth hormone (hGH) (Llompart et al., 2010), calcitonin and epidermal growth factor (EGF) (Torrent et al., 2009b), xylanase (Llop-Tous et al., 2011). The formation of protein bodies allows for better accumulation of the fusion proteins within a plant host cell and it also simplifies the purification process of the fusion protein as the product is particulate. The fusion protein of the invention is also considerably more immunogenic than the subunit VP2 polypeptide alone.

The particulate fusion proteins of the invention are considered excellent immunogens as they lack the viral genome and they have also been shown to stimulate both the humoral and cellular arms of the immune system. Further, the Zera® peptide is also believed to have an adjuvanting effect. There are a number of other advantages to using particles as vaccine candidates. Their administration precludes the co-administration of an adjuvant for the induction of a strong antibody response, thus reducing the vaccine dose costs. In the case of animal vaccines, the Zera® sequence can be used as a marker for vaccination (Liu et al., 2012), allowing for the distinction between infected and vaccinated animals (DIVA) - this is a very important requirement in areas affected by disease outbreaks, such as in the EU where vaccination has taken place.

The fusion proteins of the present invention are also safe to produce, and further the production method is relatively cost effective. The recombinant vaccine of the invention should abrogate the need for high biosafety levels during manufacture. This is due to the fact that production of a recombinant vaccine in plants will negate the requirement for high biosafety levels for handling, thus significantly lowering the risks and the cost of its production compared to present live attenuated or whole-virus killed vaccines. The use of a recombinant protein, as opposed to live virus, also accelerates the production rate since the protein bodies can be made transiently in plants within 9 days of infiltration.

The existing vaccine against BTV which is used in South Africa consists of field strains of BTV attenuated through serial passage in embryonated chicken eggs and BHK-21 cells. The vaccine consists of 3 bottles (A, B and C) each containing 5 different serotypes (A - 1, 4, 6, 12 and 14; B - 3, 8, 9, 10 and 11; C - 2, 5, 7, 13 and 19). The existing vaccine does not represent all of the BTV serotypes circulating in South Africa, which total 22. The reason for this is that the missing serotypes (serotypes 15, 16, 18, 22-26) do not cause severe pathogenicity in sheep.

The current production process using eggs and cell culture is costly and the inclusion of so many different serotypes also adds substantially to the cost of manufacturing the existing vaccine. The use of live virus also makes it difficult to differentiate infected from vaccinated animals (DIVA).

Mohd Jaafar Fauziah et al., Vaccine, Vol.32, no.32, 2 June 2-14, pages 4059-4067 discloses the expression of bluetongue virus VP2 protein as a fusion protein with glutathione S-transferase in a bacterial cell system.

Capocefalo A et al., Journal of Virological methods, Elsevier BV, NL, vol. 169, no.2, 1 November 2016, pages 420-424 discloses a further expression system for the production of VP2 peptides, namely a mammalian expression system.

Andrew J Conley et al., Plant Biotechnology Journal, vol. 9, no.4, 1 May 2011 pages 419-433 discloses that maize γ-zein protein can induce the formation of protein storage bodies, facilitating the recovery of fused proteins.

Mark Whitehead et al., BMC Cancer, BioMed central, Vol. 14, no.1, page 367 describes Zera® fused to HPV 16E7SH protein.

The present invention describes production of VP2 fusion proteins, which are capable of forming protein bodies, the protein bodies of which are particulate in nature.

### SUMMARY OF THE INVENTION

The present invention relates to insoluble fusion proteins comprising a BTV VP2 polypeptide fused to a maize γ-zein peptide, nucleic acids encoding the insoluble fusion proteins, vaccine compositions comprising the insoluble fusion proteins and methods for producing the insoluble fusion proteins.

According to a first aspect of the invention there is provided for an insoluble fusion protein comprising a bluetongue virus VP2 polypeptide or a derivative thereof which is linked to a maize γ-zein peptide sequence or a derivative thereof.

In one embodiment of the invention the bluetongue virus VP2 polypeptide or its derivative is selected from the group consisting of a VP2 polypeptide of the serotype: BTV-1, BTV-2, BTV-3, BTV-4, BTV-5, BTV-6, BTV-7, BTV-8, BTV-9, BTV-10, BTV-11, BTV-12, BTV-13, BTV-14 and BTV-22. In a further part of the disclosure the VP2 polypeptide may be selected from the group consisting of a VP2 polypeptide of the serotype: BTV-15, BTV-16, BTV-17, BTV-18, BTV-19, BTV-20, BTV-21, BTV-23, BTV-24, BTV-25 and BTV-26.

In another embodiment of the invention the maize γ-zein peptide sequence or derivative thereof comprises a sequence of SEQ ID NO:2 or an amino acid sequence having at least 90% sequence identity to the sequence of SEQ ID NO:2.

In yet another part of the disclosure, the polypeptide sequence of the bluetongue virus VP2 or its derivative is selected from the group consisting of BTV-1 (SEQ ID NO:5), BTV-2 (SEQ ID NO:6), BTV-3 (SEQ ID NO:7), BTV-4 (SEQ ID NO:8), BTV-5 (SEQ ID NO:9), BTV-6 (SEQ ID NO:10), BTV-7 (SEQ ID NO:11), BTV-8 (SEQ ID NO:12), BTV-9 (SEQ ID NO:13), BTV-10 (SEQ ID NO:14), BTV-11 (SEQ ID NO:15), BTV-12 (SEQ ID NO:16), BTV-13 (SEQ ID NO:17), BTV-14 (SEQ ID NO:18), BTV-15 (SEQ ID NO:19), BTV-16 (SEQ ID NO:20), BTV-17 (SEQ ID NO:21), BTV-18 (SEQ ID NO:22), BTV-19 (SEQ ID NO:23), BTV-20 (SEQ ID NO:24), BTV-21 (SEQ ID NO:25), BTV-22 (SEQ ID NO:26), BTV-23 (SEQ ID NO:27), BTV-24 (SEQ ID NO:28), BTV-25 (SEQ ID NO:29), BTV-26 (SEQ ID NO:30) and BTV-27 (SEQ ID NO:35) or a codon optimised sequence having at least 90% sequence identity thereto.

It will be appreciated by those of skill in the art that the insoluble fusion protein of the invention is capable of forming a protein body, as a result of the properties of the zein peptide. The protein body formed from the insoluble fusion protein of the invention presents the antigenic epitopes of the VP2 polypeptide to the immune system, consequently illiciting an immune response. Accordingly, it will further be appreciated that the protein bodies of the invention are particulate in nature and are thus effective immunogens for eliciting immune responses against BTV.

In one embodiment of the invention the fusion protein is expressed in and recovered from a plant or plant cell. It will however be appreciated that any suitable host cell may be used to express the fusion proteins of the invention.

A second aspect of the invention provides for nucleic acid molecules encoding the insoluble fusion proteins of the invention. In some embodiments, the nucleic acid molecules of the invention may be operably linked to regulatory sequences in such a way as to permit gene expression thereof when the appropriate molecules are bound to the regulatory sequences. Such operably linked sequences may be in the form of vectors or expression constructs that can be transformed or transfected into host cells for expression. It will be appreciated by those of skill in the art that any suitable vector can be used for this purpose.

It will be appreciated that expression vectors comprising the nucleic acid molecules encoding the insoluble fusion proteins and expression cassettes comprising the nucleic acid molecules encoding the insoluble fusion proteins also fall within the scope of an embodiment of the invention.

A further aspect of the invention provides for a vaccine composition comprising the insoluble fusion proteins of the invention and a pharmaceutically acceptable diluent or excipient, wherein the vaccine composition is capable of eliciting a protective immune response against bluetongue virus. Preferably the protective immune response is a cellular or humoral immune response.

In one embodiment the insoluble fusion protein may be present in an oil in water emulsion vehicle.

In a preferred embodiment the vaccine composition comprises a combination of at least two insoluble fusion proteins from different BTV serotypes, such as insoluble fusion proteins comprising a VP2 polypeptide or derivative thereof selected from the group consisting of BTV-1, BTV-2, BTV-3, BTV-4, BTV-5, BTV-6, BTV-7, BTV-8, BTV-9, BTV-10, BTV-11, BTV-12, BTV-13, BTV-14, BTV-15, BTV-16, BTV-17, BTV-18, BTV-19, BTV-20, BTV-21, BTV-22, BTV-23, BTV-24, BTV-25 and BTV-26.

In one embodiment the vaccine composition is for use in inducing an immune response against bluetongue virus.

Also described is a DNA vaccine composition comprising a cassette containing a promoter and a polynucleotide encoding the fusion protein of the invention. Alternatively, the DNA vaccine may comprise the expression vectors described herein. The cassette or expression vector containing the nucleic acid molecules encoding the fusion proteins of the invention may be mixed together with pharmaceutically acceptable diluents or excipients. The nucleic acid molecules encoding the fusion proteins are preferably linked to regulatory sequences that allow for the expression of the fusion proteins in a cell. Preferably, the cell is an animal cell.

A further aspect of the invention relates to the use of the insoluble fusion proteins described herein in the manufacture of a vaccine for use in a method of preventing bluetongue virus infection in a subject, the method comprising administering a therapeutically effective amount of the vaccine to the subject.

According to yet another aspect of the present invention there is provided for a method of producing the insoluble fusion proteins of the invention in a plant or plant cell, wherein the method comprises the steps of:
(i) transforming or infiltrating a plant cell with an expression vector which contains a nucleic acid molecule of the invention;
(ii) expressing the insoluble fusion protein in the plant cell; and
(iii) recovering the insoluble fusion protein from the plant cell.

It will be appreciated that the method of producing the insoluble fusion protein may be either by transient expression of the protein or by stably expressing the protein in the plant cell.

### BRIEF DESCRIPTION OF THE FIGURES

Non-limiting embodiments of the invention will now be described by way of example only and with reference to the following figures:
**Figure 1****:** Zera® signal sequence (339 bases (SEQ ID NO:1); 113 amino acids (SEQ ID NO:2)
**Figure 2****:** pEAQ-HTZera®-VP2 plasmid map
**Figure 3****:** Zera®-VP2 detected in the pellet after purification using α-VP2R (a), BTV-8 sheep serum (b) and BTV-10 guinea pig produced serum (c).
**Figure 4****:** Zera®-VP2 detected in the crude extract after probing with 1:2000 BTV-8 sheep serum.
**Figure 5****:** Serum titration of Zera®-VP2 vaccinated mice
**Figure 6****:** A TEM of a leaf section infiltrated with only infiltration medium at 17000x magnification. B TEM of a leaf section showing electron dense Zera®-VP2 protein bodies (PB) at 7 dpi (5700x magnification). CPT: chloroplast; CW: cell wall; ER: endoplasmic reticulum; CYT: cytoplasm.
**Figure 7****:** A schematic of the BTV particle showing the four structural proteins, the transcriptase complex and the dsRNA genome (Mertens et al., 2004).
**Figure 8****:** Western blot analysis of Zera® BTV-2 VP2 (124.2kDa) expression of in *N. benthamiana* extracted on day 5. Nitrocellulose membrane probed with anti-Zera® (lane 1-3) and sheep anti-BTV-8 (lane 5-6) (A). Positive control (plant-produced BTV-8 VP2, lane 1 and 6), plants infiltrated with pEAQ-HT Zera® BTV-2 VP2 (lane 2 and 5) and negative control (lane 3). Nitrocellulose membrane probed rabbit anti-BTV-8 VP2 primary antibody (B). Negative control (lane 1), plant infiltrated with pEAQ-HT BTV-2 VP2 (lane 2), positive control (plant-produced BTV-8 VP2, lane 3).
**Figure 9****:** Immunoblot of purified Zera® BTV-2 VP2 (124.2 kDa). Primary antibody used in this immunoblot was directed against the Zera® tag. A 1:10 and 1:100 dilution were prepared of the purified sample.
**Figure 10****:** Coomassie stain of purified Zera® BTV-2 VP2 (124.2 kDa). A final centrifugation step was also included to clarify any insoluble protein in the sample for comparison. A 1:10 and 1:100 dilution were prepared of the purified sample.

### DETAILED DESCRIPTION OF THE INVENTION

The present invention will now be described more fully hereinafter with reference to the accompanying drawings, in which some, but not all embodiments of the invention are shown.

The invention as described should not be limited to the specific embodiments disclosed. Although specific terms are employed herein, they are used in a generic and descriptive sense only and not for purposes of limitation.

As used throughout this specification and in the claims which follow, the singular forms "a", "an" and "the" include the plural form, unless the context clearly indicates otherwise.

The terminology and phraseology used herein is for the purpose of description and should not be regarded as limiting. The use of the terms "comprising", "containing", "having" and "including" and variations thereof used herein, are meant to encompass the items listed thereafter and equivalents thereof as well as additional items.

By "bluetongue" or "BT" is meant a virus belonging to a group of approximately 26 related but genetically distinct "serotypes". The virus may also be referred to herein as "bluetongue virus" or "BTV".

BTV is a double-stranded ribonucleic acid (dsRNA) virus that causes an insect-borne, infectious non-contagious disease of both domesticated and wild ruminants; it is the type species of the genus *Orbivirus* that is classified into the family *Reoviridae. Reoviridae* is one of the largest families of virus that includes major human pathogens, such as rotavirus, as well as pathogens of insects, reptiles, fish, plants and fungi (Mertens et al., 2004). *Orbiviruses* differ from other members of the *Reoviridae* family in that they can multiply in both arthropod and vertebrate cells, causing severe disease and high mortality. BTV is transmitted between its hosts by *Culicoides* spp., causing disease in ruminants worldwide.

Virus protein (VP) 2 is the most variable of the BTV capsid proteins and contains the epitopes involved in virus neutralisation and serotype determination (DeMaula et al., 2000, Huismans and Erasmus, 1981). Twenty seven distinct serotypes of BTV have been identified based on neutralisation activity of VP2 as well as with BTV specific real time reverse transcriptase polymerase chain reaction (RT-PCR). Each serotype shows variation that is associated with the geographical origins of the virus from around the world. Molecular studies on BTV isolates from different geographic regions have further divided BTV into two major topotypes, namely the eastern and western lineages (Maan et al., 2012, Maan et al., 2010).

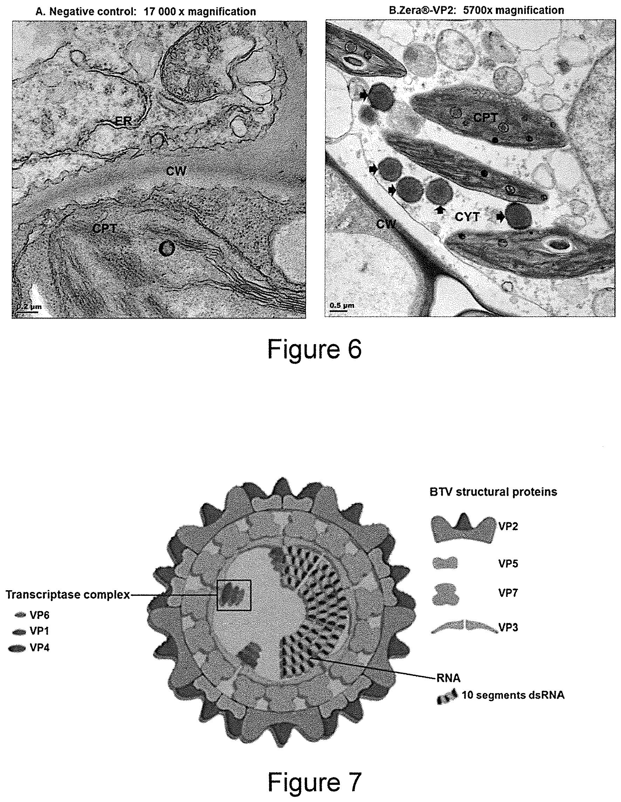

The BTV genome is a double-stranded circular dsRNA surrounded by a protein capsid. BTV can replicate in both wild and domestic ruminants as well as some species of deer. Replication takes place in both the host and the *Culicoides* insect vector. BTV virions are complex three-layered icosahedral structures that are ∼80 nanometer (nm) in diameter. The virions are composed of a core of ten segments of dsRNA encapsulated by seven structural proteins (four major and three minor proteins) that are arranged into three distinct layers (Figure 7).

The three minor proteins (viral protein (VP) 1, VP4 and VP6) are enclosed by the subcore that is made up of VP3. The core-surface layer consists of VP7. The outer capsid is composed of major proteins VP2 and VP5 which is laid onto the foundation provided by the core. The minor proteins together with the genomic RNA form the virus replication complex, whereas the four major proteins make up the capsid of the virus. In addition to the structural proteins BTV has four non-structural (NS) proteins (NS1, NS2, NS3 and NS3a) which are involved in virus replication and assembly in BTV-infected cells.

The fusion proteins and compositions according to the invention may be used to treat BTV infection or conditions associated with BTV infection. BTV can infect all known species of domestic and wild ruminants. Severe disease usually occurs in the fine-wool and mutton breeds of sheep as well as some species of deer. BTV infection of cattle, goats and wild ruminant species is mostly asymptomatic or subclinical. In BTV endemic areas BTV-infected sheep develop only mild or no obvious disease. The bluetongue after which the disease is named is seen only in serious clinical cases.

Onset of the disease in sheep is typically characterised by high fever lasting 5-7 days. Clinical signs of disease can include fever, depression, excessive salivation, nasal discharge, facial oedema, hyperaemia and ulceration of the oral mucosa, coronitis, lameness and death. Abortion can occur in pregnant animals as well as teratogenic defects in calves. The severity of clinical disease and mortality rate is influenced by the breed and age of the animal as well as the virus strain that causes the infection. In acute phases of BT, clinical signs in sheep are mainly associated with damage to microvascular endothelial cells.

After recovery from BT animals may suffer from a number of long-lasting secondary effects, such as reductions in milk production, weight loss, wool brake and temporary infertility.

Pathogenesis of BTV infection is similar in sheep and cattle as well as other species of ruminants. After an animal gets infected with BTV, through the bite of a *Culicoides* vector, the virus will travel to the regional lymph node where initial replication takes place. The virus then spreads throughout the body to a variety of tissues, where replication occurs mainly in mononuclear phagocytic and endothelial cells.

Viraemia is cell associated and can be prolonged in domestic ruminants. During viraemia BTV is associated with all blood cells, but late in the course of infection the virus is mostly associated with the erythrocytes. The longer lifespan of erythrocytes facilitate prolonged infection of ruminants, as well as the infection of the haematophagous insect vectors that feed on viraemic ruminants (Barratt-Boyes et al., 1995). Infectious virus can co-circulate for several weeks with high neutralising antibody titres, the maximum period of viraemia in sheep is about 50 days and in cattle about 100 days.

By "condition associated with BTV infection" is meant any condition, disease or disorder that has been correlated with the presence of an existing BTV infection, in includes secondary effects, such as reductions in milk production, weight gain, wool brake and temporary infertility.

A compound according to the invention includes, without limitation, a fusion protein including the amino acid sequence of a VP2 protein, fused to a maize γ-zein peptide sequence. It will be appreciated by those skill in the art that the VP2 protein or derivative thereof may be selected from any of the BTV serotypes, but particularly BTV-1, BTV-2, BTV-3, BTV-4, BTV-5, BTV-6, BTV-7, BTV-8, BTV-9, BTV-10, BTV-11, BTV-12, BTV-13, BTV-14 and BTV-22. It will further be appreciated that the VP2 protein or derivative thereof may be selected from a polypeptide selected from the group consisting of any one of SEQ ID NO:5 to SEQ ID NO:30 or SEQ ID NO:35 or a codon-optimised polynucleotide sequence encoding a polypeptide selected from the group consisting of any one of SEQ ID NO:5 to SEQ ID NO:30 or SEQ ID NO:35 and having at least 90% sequence identity thereto.

A "protein," "peptide" or "polypeptide" is any chain of two or more amino acids, including naturally occurring or non-naturally occurring amino acids or amino acid analogues, irrespective of post-translational modification (e.g., glycosylation or phosphorylation).

An "antigen" is a compound, composition, or substance that can stimulate the production of antibodies and/or a CD4+ or CD8+ T cell response in an animal, including compositions that are injected or absorbed into an animal. An antigen reacts with the products of specific humoral or cellular immunity, including those induced by heterologous immunogens.

The terms "nucleic acid" or "nucleic acid molecule" encompass both ribonucelotides (RNA) and deoxyribonucleotides (DNA), including cDNA, genomic DNA, and synthetic DNA. The nucleic acid may be double-stranded or single-stranded. Where the nucleic acid is single-stranded, the nucleic acid may be the sense strand or the antisense strand. A nucleic acid molecule may be any chain of two or more covalently bonded nucleotides, including naturally occurring or non-naturally occurring nucleotides, or nucleotide analogs or derivatives. By "RNA" is meant a sequence of two or more covalently bonded, naturally occurring or modified ribonucleotides. The term "DNA" refers to a sequence of two or more covalently bonded, naturally occurring or modified deoxyribonucleotides. By "cDNA" is meant a complementary or copy DNA produced from an RNA template by the action of RNA-dependent DNA polymerase (reverse transcriptase).

Accordingly, a "cDNA clone" refers to a duplex DNA sequence which is complementary to an RNA molecule of interest, and which is carried in a cloning vector. The term "complementary" refers to two nucleic acids molecules, e.g., DNA or RNA, which are capable of forming Watson-Crick base pairs to produce a region of double-strandedness between the two nucleic acid molecules. It will be appreciated by those of skill in the art that each nucleotide in a nucleic acid molecule need not form a matched Watson-Crick base pair with a nucleotide in an opposing complementary strand to form a duplex. One nucleic acid molecule is thus "complementary" to a second nucleic acid molecule if it hybridizes, under conditions of high stringency, with the second nucleic acid molecule. A nucleic acid molecule according to the invention includes both complementary molecules.

In some embodiments, a fusion protein of the invention may include, without limitation, a polypeptide including an amino acid sequence substantially identical to the amino acid sequence a fusion protein comprising BTV VP2 protein, linked to a γ-zein peptide. Another embodiment of the invention includes, without limitation, nucleic acid molecules encoding the aforementioned fusion protein.

As used herein a "substantially identical" sequence is an amino acid or nucleotide sequence that differs from a reference sequence only by one or more conservative substitutions, or by one or more non-conservative substitutions, deletions, or insertions located at positions of the sequence that do not destroy or substantially reduce the antigenicity of the expressed fusion protein or of the polypeptide encoded by the nucleic acid molecule. Alignment for purposes of determining percent sequence identity can be achieved in various ways that are within the knowledge of those with skill in the art. These include using, for instance, computer software such as ALIGN, Megalign (DNASTAR), CLUSTALW or BLAST software. Those skilled in the art can readily determine appropriate parameters for measuring alignment, including any algorithms needed to achieve maximal alignment over the full length of the sequences being compared. In one embodiment of the invention there is provided for a polypeptide or polynucleotide sequence that has at least about 80% sequence identity, at least about 90% sequence identity, or even greater sequence identity, such as about 95%, about 96%, about 97%, about 98% or about 99% sequence identity to the sequences described herein.

Alternatively, or additionally, two nucleic acid sequences may be "substantially identical" if they hybridize under high stringency conditions. The "stringency" of a hybridisation reaction is readily determinable by one of ordinary skill in the art, and generally is an empirical calculation which depends upon probe length, washing temperature, and salt concentration. In general, longer probes required higher temperatures for proper annealing, while shorter probes require lower temperatures. Hybridisation generally depends on the ability of denatured DNA to re-anneal when complementary strands are present in an environment below their melting temperature. A typical example of such "stringent" hybridisation conditions would be hybridisation carried out for 18 hours at 65°C with gentle shaking, a first wash for 12 min at 65°C in Wash Buffer A (0.5% SDS; 2XSSC), and a second wash for 10 min at 65°C in Wash Buffer B (0.1% SDS; 0.5% SSC).

In one embodiment, the VP2 polynucleotide sequences may be "naturally occurring" or "native" that is they are isolated from a natural source rather than artificially produced. Sources of such native polynucleotides may include, biological samples, such as biopsies, blood, mucus, oral, plasma, semen, serum, urine, etc.) obtained from an infected subject or from another source.

In an alternative part of the disclosure, the fusion proteins may be prepared by, for instance, inserting, deleting or replacing amino acid residues at any position of the VP2 polypeptide sequences and/or, for instance inserting, deleting or replacing nucleic acids at any position of the nucleic acid molecule encoding a VP2 polypeptide from any BTV serotype.

Those skilled in the art will appreciate that polypeptides, peptides or peptide analogues can be synthesised using standard chemical techniques, for instance, by automated synthesis using solution or solid phase synthesis methodology. Automated peptide synthesisers are commercially available and use techniques known in the art. Polypeptides, peptides and peptide analogues can also be prepared from their corresponding nucleic acid molecules using recombinant DNA technology.

In some embodiments, the nucleic acid molecules of the invention may be operably linked to other sequences. By "operably linked" is meant that the nucleic acid molecules encoding the fusion proteins of the invention and regulatory sequences are connected in such a way as to permit expression of the fusion proteins when the appropriate molecules are bound to the regulatory sequences. Such operably linked sequences may be contained in vectors or expression constructs which can be transformed or transfected into host cells for expression. It will be appreciated that any vector can be used for the purposes of expressing the fusion proteins of the invention.

The term "recombinant" means that something has been recombined. When used with reference to a nucleic acid construct the term refers to a molecule that comprises nucleic acid sequences that are joined together or produced by means of molecular biological techniques. The term "recombinant" when used in reference to a protein or a polypeptide refers to a protein or polypeptide molecule which is expressed from a recombinant nucleic acid construct created by means of molecular biological techniques. Recombinant nucleic acid constructs may include a nucleotide sequence which is ligated to, or is manipulated to become ligated to, a nucleic acid sequence to which it is not ligated in nature, or to which it is ligated at a different location in nature. Accordingly, a recombinant nucleic acid construct indicates that the nucleic acid molecule has been manipulated using genetic engineering, i.e. by human intervention. Recombinant nucleic acid constructs may be introduced into a host cell by transformation. Such recombinant nucleic acid constructs may include sequences derived from the same host cell species or from different host cell species.

The term "vector" refers to a means by which polynucleotides or gene sequences can be introduced into a cell. There are various types of vectors known in the art including plasmids, viruses, bacteriophages and cosmids. Generally polynucleotides or gene sequences are introduced into a vector by means of a cassette. The term "cassette" refers to a polynucleotide or gene sequence that is expressed from a vector, for example, the polynucleotide or gene sequences encoding the fusion proteins of the invention. A cassette generally comprises a gene sequence inserted into a vector, which in some embodiments, provides regulatory sequences for expressing the polynucleotide or gene sequences. In other embodiments, the vector provides the regulatory sequences for the expression of the fusion protein. In further embodiments, the vector provides some regulatory sequences and the nucleotide or gene sequence provides other regulatory sequences. "Regulatory sequences" include but are not limited to promoters, transcription termination sequences, enhancers, splice acceptors, donor sequences, introns, ribosome binding sequences, poly(A) addition sequences, and/or origins of replication.

The fusion proteins or compositions of the invention can be provided either alone or in combination with other compounds (for example, nucleic acid molecules, small molecules, peptides, or peptide analogues), in the presence of a liposome, an adjuvant, or any carrier, such as a pharmaceutically acceptable carrier and in a form suitable for administration to mammals, for example, humans, cattle, sheep, etc.

As used herein a "pharmaceutically acceptable carrier" or "excipient" includes any and all antibacterial and antifungal agents, coatings, dispersion media, solvents, isotonic and absorption delaying agents, and the like that are physiologically compatible. A "pharmaceutically acceptable carrier" may include a solid or liquid filler, diluent or encapsulating substance which may be safely used for the administration of the fusion protein or vaccine composition to a subject. The pharmaceutically acceptable carrier can be suitable for intramuscular, intraperitoneal, intravenous, oral or sublingual administration. Pharmaceutically acceptable carriers include sterile aqueous solutions, dispersions and sterile powders for the preparation of sterile solutions. The use of media and agents for the preparation of pharmaceutically active substances is well known in the art. Where any conventional media or agent is incompatible with the active compound, use thereof in the pharmaceutical compositions of the invention is not contemplated. Supplementary active compounds can also be incorporated into the compositions.

Suitable formulations or compositions to administer the fusion proteins and compositions to subjects suffering from BTV infection or subjects which are presymptomatic for a condition associated with BTV infection fall within the scope of the invention. Any appropriate route of administration may be employed, such as, parenteral, intravenous, subcutaneous, intramuscular, intracranial, intraorbital, ophthalmic, intraventricular, intracapsular, intraspinal, intrathecal, intracistemal, intraperitoneal, intranasal, aerosol, topical, or oral administration.

As used herein the term "subject" includes both wild and domestic ruminants.

For vaccine formulations, an effective amount of the fusion proteins or compositions of the invention can be provided, either alone or in combination with other compounds, with immunological adjuvants, for example, aluminium hydroxide dimethyldioctadecylammonium hydroxide or Freund's incomplete adjuvant. The fusion proteins or compositions of the invention may also be linked with suitable carriers and/or other molecules, such as bovine serum albumin or keyhole limpet hemocyanin in order to enhance immunogenicity.

In some parts of the disclosure, the fusion proteins or compositions according to the invention may be provided in a kit, optionally with a carrier and/or an adjuvant, together with instructions for use.

An "effective amount" of a compound according to the invention includes a therapeutically effective amount, immunologically effective amount, or a prophylactically effective amount. A "therapeutically effective amount" refers to an amount effective, at dosages and for periods of time necessary, to achieve the desired therapeutic result, such as treatment of BTV infection or a condition associated with such infection. The outcome of the treatment may for example be measured by a decrease in BTV viremia, inhibition of viral gene expression, delay in development of a pathology associated with BTV infection, stimulation of the immune system, or any other method of determining a therapeutic benefit. A therapeutically effective amount of a compound may vary according to factors such as the disease state, age, sex, and weight of the individual, and the ability of the compound to elicit a desired response in the individual. Dosage regimens may be adjusted to provide the optimum therapeutic response. A therapeutically effective amount is also one in which any toxic or detrimental effects of the compound are outweighed by the therapeutically beneficial effects.

The dosage of any of the fusion proteins or compositions of the present invention will vary depending on the symptoms, age and body weight of the subject, the nature and severity of the disorder to be treated or prevented, the route of administration, and the form of the composition. Any of the compositions of the invention may be administered in a single dose or in multiple doses. The dosages of the compositions of the invention may be readily determined by techniques known to those of skill in the art or as taught herein.

By "immunogenically effective amount" is meant an amount effective, at dosages and for periods of time necessary, to achieve a desired immune response, including a cellular and/or humoral response. The desired immune response may include stimulation or elicitation of an immune response, for instance a T cell response.

A "prophylactically effective amount" refers to an amount effective, at dosages and for periods of time necessary, to achieve a desired prophylactic result, such as prevention of onset of a condition associated with BTV infection. Typically, a prophylactic dose is used in subjects prior to or at an earlier stage of disease, so that a prophylactically effective amount may be less than a therapeutically effective amount.

Dosage values may vary with the severity of the condition to be alleviated. For any particular subject, specific dosage regimens may be adjusted over time according to the individual need and the judgment of the person administering or supervising the administration of the fusion proteins or compositions of the invention. Dosage ranges set forth herein are exemplary only and do not limit the dosage ranges that may be selected. The amount of active compound(s) in the composition may vary according to factors such as the disease state, age, sex, and weight of the individual. Dosage regimens may be adjusted to provide the optimum therapeutic response. For example, a single dose may be administered, or multiple doses may be administered over time. It may be advantageous to formulate the compositions in dosage unit forms for ease of administration and uniformity of dosage.

The term "preventing", when used in relation to an infectious disease, or other medical disease or condition, is well understood in the art, and includes administration of a composition which reduces the frequency of or delays the onset of symptoms of a condition in a subject relative to a subject which does not receive the composition. Prevention of a disease includes, for example, reducing the number of diagnoses of the infection in a treated population versus an untreated control population, and/or delaying the onset of symptoms of the infection in a treated population versus an untreated control population.

The term "prophylactic or therapeutic" treatment is well known to those of skill in the art and includes administration to a subject of one or more of the compositions of the invention. If the composition is administered prior to clinical manifestation of the unwanted condition (e.g., disease or other unwanted state of the subject) then the treatment is prophylactic, i.e., it protects the host against developing the unwanted condition, whereas if it is administered after manifestation of the unwanted condition, the treatment is therapeutic (i.e., it is intended to diminish, ameliorate, or stabilize the existing unwanted condition or side effects thereof).

Toxicity and therapeutic efficacy of compositions of the invention may be determined by standard pharmaceutical procedures in cell culture or using experimental animals, such as by determining the LD50 and the ED50. Data obtained from the cell cultures and/or animal studies may be used to formulating a dosage range for use in a subject. The dosage of any composition of the invention lies preferably within a range of circulating concentrations that include the ED50 but which has little or no toxicity. The dosage may vary within this range depending upon the dosage form employed and the route of administration utilized. For compositions of the present invention, the therapeutically effective dose may be estimated initially from cell culture assays.

The antigenic epitopes of the VP2 polypeptide are encapsulated in the protein body. It appears that the recombinant fusion proteins associate with each other through hydrophobic interactions (8 repeats of the PPVHL domain as well as disulphide bond formation by the 6 cysteine residues embedding the hydrophobic domain). It would appear that interactions between the Zera® domains and the ER membrane induces budding off of the protein bodies. It is surmised that the protein bodies of the invention induce an immune response which results in the protein bodies being broken up and consequently resulting in the immune system being exposed to the encapsulated VP2 polypeptides.

Also disclosed is a method of treating an infection in a subject comprising administering to a subject in need thereof a therapeutically effective amount of the fusion proteins or compositions of the present invention.

The following example is offered by way of illustration and not by way of limitation.

### EXAMPLE 1

BTV-8 VP2 was *Nicotiana* sp. codon-optimised by Geneart (SEQ ID NO:3) and cloned into the plant expression vector pTRAkc-rbcs1-cTP to yield pTRAkc-rbcs1-cTPVP2co. A multiepitope sequence was excised from a pre-existing construct (pEAQ-HTZera®-VP2ep) in our laboratory using the restriction enzymes *Mlu*I and *Xho*I to yield a pEAQ- HTZera® backbone. VP2 was restriction enzyme-digested from pTRAkc-rbcs1-cTPVP2co using the *Mlu*I and *Xho*I sites and subcloned into the pEAQ-HTZera® backbone. This yielded a construct with Zera® linked to the 5' terminus of VP2 yielding pEAQ-HTZera®-VP2 (Figure 2). The resulting construct was sequenced (SEQ ID NO:4) and transformed into *A. tumefaciens* LBA4404.

For agro-infiltration, 10 ml cultures of the recombinant pEAQ-HTZera®-VP2 were grown up in LB containing magnesium sulphate (2 mM), rifampicin (50 µg/ml) and kanamycin (30 µg/ml) at 27°C overnight with agitation at 200 rpm. A 10^{th} of the volume was transferred to 100 ml induction medium (LB, 10 mM MES, pH 5.6) containing the same concentration of antibiotics as well as 20 µM acetosyringone. The culture was incubated overnight at 27°C with agitation at 200 rpm and then centrifuged at 4000 rpm to pellet the cells. The cell pellet was resuspended in 5 ml infiltration medium (10 mM MES, 10 mM MgCl₂, 3% sucrose, pH 5.6) supplemented with 200 µM acetosyringone and incubated at room temperature for 2 h. The culture was diluted to an OD₆₀₀ of 1.5 and syringe-infiltrated into the abaxial surfaces of six-week-old *N. benthamiana* plants.

At 7 dpi the leaves were either frozen at -80°C or prepared for density gradient centrifugation. Freshly harvested leaves were immediately cut up into fine pieces and homogenized thoroughly in five volumes ice cold buffer PBP3 (100 mM Tris pH 8, 50 mM KCI, 6 mM MgCl₂, 10 mM EDTA and 0.4 M NaCl) with 10% sucrose and 1x Complete Mini, EDTA-free protease inhibitor cocktail (Roche). The crude plant extract was incubated at 4°C with shaking, after which it was clarified through four layers of Miracloth™ (Merck). The crude extract was centrifuged at 4000 rpm for 10 min at 4°C. The clarified crude plant sap was overlayed onto 5 ml of a 42% sucrose cushion (prepared in buffer PBP3) and centrifuged for 2 h at 79000x g in a SW 32 Ti Rotor (Beckman). The pellet was resuspended in 300 µl buffer PBP3 containing 10% sucrose. The resuspended pellet was washed 11 times by centrifugation at 6000 rpm for 5 min, collecting the supernatant (SNT and w1 to w10) and resuspending the pellet in 500 µl PBP3 buffer containing 10% sucrose. The wash fractions and the final pellet were analysed on dotblots.

Dotblots were carried out on fractions to detect the presence of the protein. For the dotblots, 5 µl of the Zera®-VP2 wash fractions and the final pellet was pipetted onto nitrocellulose membranes and left to dry completely. The membranes were probed with 1:2000 dilutions of α-VP2R (rabbit raised antibody against *E*. *coli-*expressed wild type VP2), BTV-8 sheep serum (containing antibodies to BTV-8 VLPs) and BTV-10 guinea pig produced serum (containing antibodies to BTV-10 virus). The membranes were subsequently probed with a 1:5000 dilution of anti-rabbit alkaline phosphatase-conjugated secondary antibody (Sigma-Aldrich #A3687), 1:10000 dilution of anti-goat/sheep alkaline phosphatase-conjugated secondary antibody (Sigma-Aldrich #A8062) or 1:5000 dilution of alkaline phosphatase-conjugated anti-guinea-pig antibody (Sigma-Aldrich #A5062), respectively. Detection was performed with 5-bromo-4-chloro-3-indoxyl-phosphate (BCIP) and nitroblue tetrazolium (NBT) phosphatase substrate (BCIP/NBT 1-component, KPL).

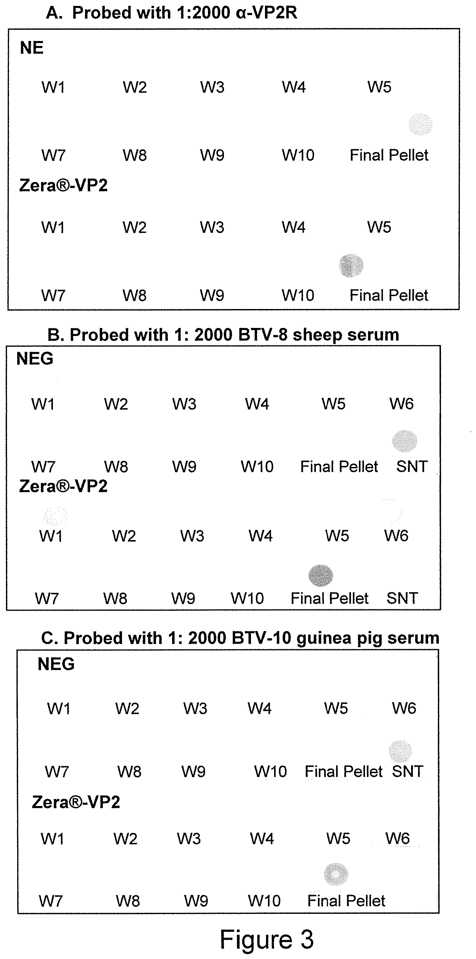

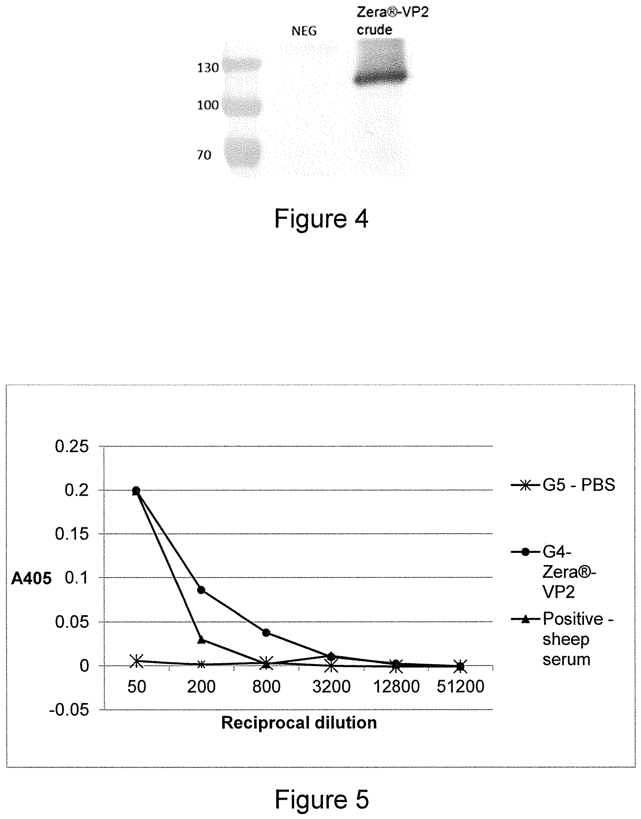

The dotblots (Figure 3) using the different sera indicated that the majority of Zera®-VP2 remained insoluble in the pellet as visualised by a dark precipitate (final pellet) with little to no protein detected in the wash fractions (the first wash (SNT) seemed to include most of the chlorophyll from the crude extract and there was no dark precipitate in w1 to w10). Zera®-VP2 was insoluble and very stable after purification. Samples of crude extract and purified protein (pellet) were separated by denaturing SDS-PAGE to detect the size of the protein. The purified protein could not be denatured with DTT or SDS to analyse with SDS-PAGE, however the crude extract could be sufficiently denatured and a single band of the expected size of ∼120 kDa was detected on western blots (Figure 4).

The insolubility of the Zera®-VP2ep fusion protein could not be predicted: this may be an advantage, as insoluble antigens work well in providing a "depot effect" for slow leakage of antigen into the circulation for long-term immune stimulation.

The purified preparation of Zera®-VP2 was used to inoculate Balb/c mice together with BTV-8 VLPs. This was as a control to test their ability to elicit an antibody response in mice (AEC# 011-016) which would give an indication that the particles were immunogenic. The mouse study was performed in duplicate (exp 1 and exp 2).

Each experiment consisted of a total of 20 mice that were divided into 4 groups containing 5 mice each.

| | |
|---|---|
| Group1: | Inoculated with BTV-8 VLP vaccine |
| Group2: | Inoculated with BTV-8 VLP vaccine with Freund's adjuvant (Incomplete) |
| Group3: | Inoculated with Zera®-VP2 vaccine |
| Group4: | Inoculated with PBS negative control |

Three days before vaccination all the mice were pre-bled and serum was harvested in order to determine baseline antibody levels. At Day 0 all the mice were vaccinated subcutaneously with 10 µg of the appropriate antigen with/without Freund's adjuvant (Incomplete) and at day 28 the mice received booster vaccinations with 10 µg antigen. The experiments ended at day 56 with the collection of blood from the mice via cardiac puncture. The serum obtained from these animal experiments was analysed using indirect enzyme linked immunosorbent assay (ELISA).

A 96-well Maxisorp® microtitre plate (Nunc) was coated with 100 µL/well (1µg made up to 100 µL in coating buffer [10mM Tris, pH 8.5]) of total soluble wild type BTV-8 VP2 produced in *E*. *coli* and incubated overnight at 4°C. The plates were blocked for 2 hours at room temperature with blocking buffer (5% non-fat dry milk in 1x TBS, pH 7.5 [50 mM Tris, 150 mM NaCl]) after which it was washed 4x with 1x TST buffer (1xTBS [pH 7.5], 0.05% Tween®20).

Sera from mice vaccinated with the same vaccine were pooled (5 mice / vaccine) for analysis. Sera were diluted in blocking buffer in a 4-fold series in triplicate ranging from a 1:50 dilution to 1:51200. Mouse sera from the mice vaccinated with PBS served as a negative control. Positive control wells contained sheep serum produced against BTV-8 VLPs (Thuenemann et al., 2013) and blank wells with no antibody were included for background control. A volume of 100 µL of the diluted sera was added to each well and incubated for 2 hours at room temperature after which the plates were washed 4x with 1x TST buffer. A 100 µL volume of goat anti-mouse IgG alkaline phosphatase conjugate (1:10000, Sigma) and monoclonal anti-goat/sheep IgG alkaline phosphatase conjugate (1:10000, Sigma) diluted in blocking buffer was added to wells containing mouse or sheep serum, respectively and the plates incubated for 1 hour at 37°C. Plates were washed 4x with 1x TBS (pH 9) buffer and 200 µL SIGMAFAST™ p-Nitrophenyl phosphate (pNPP, Sigma) was added to each well. The plates were developed in the dark for 30 minutes after which the absorbance was read at 405 nm on a BIO-TEK® Powerwave XS microtitre plate reader.

Initial results of the mouse sera collected from mice vaccinated with Zera®-VP2 indicate that the mice did produce antibodies against this vaccine and that the antibodies bind to wild type VP2 produced in *E*. *coli* (Figure 5).

Leaves infiltrated with the above construct were also sectioned and embedded in resin for TEM. The pEAQ-HTZera®-VP2 construct was cultured as described previously and syringe infiltrated into the abaxial spaces of six-week old *N*. *benthamiana* plants. At 7 dpi a whole leaf was picked from the infiltrated plant and embedded: A 3cm x 3cm piece was cut out with a scalpel blade in the presence of 2.5% gluteraldehyde (25% gluteraldehyde diluted in 0.1 M phosphate buffer [pH 7.4]). The leaf sample was soaked in 2.5% gluteraldehyde for 6 hours after which it was cut into 1mm x 3mm fragments, also in the presence of 2.5% gluteraldehyde. The leaf fragments were left in 2.5% gluteraldehyde overnight at 4°C. The following morning the leaf fragments were washed 3 times, 5 minutes for each wash, in 0.1 M phosphate buffer (pH 7.4). The leaf fragments were fixed for one hour in one part 2% osmium tetroxide and one part 0.2 M phosphate buffer (pH 7.4) after which it was washed twice for 5 minutes each with 0.1 M phosphate buffer (pH 7.4) followed with two washes of 5 min each with water.

After washing the leaf fragments were sequentially dehydrated. The leaf fragments were incubated for 5 minutes each in 30%, 50%, 70%, 80%, 90% and 95% ethanol. The fragments were incubated for 10 minutes in 100% ethanol; this step was repeated twice. After the ethanol dehydrations series the leaf fragments were further dehydrated by 10 minute incubation in 100% acetone, repeated twice. The leaf fragments were mixed overnight in 1:1 acetone:Spurr's resin.

The following day half of the 1:1 acetone:Spurr's resin mixture was removed (after centrifugation) and replaced with 100% Spurr's resin to yield a 1:3 acetone:Spurr's resin mixture. The sample was mixed for four hours at room temperature, after which the acetone/resin mixture was removed and replaced with 100% Spurr's resin. The leaf fragments were incubated in 100% Spurr's resin for three days at 4°C. The 100% Spurr's resin was replaced with fresh resin and incubated for four hours at room temperature after which the resin was replaced again and incubated overnight at room temperature. The following morning the samples were embedded and incubated for 24 hours at 60°C.

The embedded leaf samples were cut into ultrathin sections with a diamond knife and collected onto copper grids. The copper grids were stained with uranyl acetate for 10 minutes after which they were washed five times, 15 seconds each, with water. The grids were blotted dry and transferred to lead citrate for 10 minutes after which the grids were washed with water and blotted dry. Grids were viewed using the Technai G2 transmission electron microscope.

Figure 6A is a negative control, showing a section of leaf which was infiltrated with infiltration medium. TEM of the leaf sections expressing Zera®-VP2 show the presence of electron dense protein bodies in the cytoplasm (Figure 6B) ranging from 0.65 to 0.80 µm in size. These structures are not present in the negative control.

### EXAMPLE 2

Zera® was fused to a consensus BTV serotype 2 VP2 sequence (derived from 40 protein sequences downloaded from GenBank) resulting in a construct having a nucleotide sequence of SEQ ID NO:31, and encoding a fusion protein of SEQ ID NO:32. Zera was also fused to a consensus BTV serotype 4 VP2 sequence (derived from 25 protein sequences downloaded from GenBank) resulting in a construct having the nucleotide sequence of SEQ ID NO:33, and encoding a fusion protein of SEQ ID NO:34. Both Zera-VP2 fusions (representing serotypes 2 and 4) were successfully subcloned into the plant expression vector pEAQ-HT to generate pEAQ-HT-Zera-BTV2-VP2 and pEAQ-HT-Zera-BTV4-VP2 and into the pRIC3.0 vector. The pRIC3.0 constructs for both the Zera® BTV2 and BTV4 VP2 genes appear to be toxic to the cells.

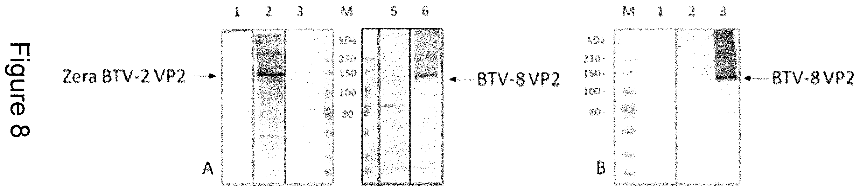

Recombinant pEAQ-HT constructs were transformed into *Agrobacterium* as follows: Six-week-old *N. benthamiana* plants were infiltrated with recombinant *Agrobacterium* strains (LBA 4404) at an OD₆₀₀ of 1.50 and containing the pEAQ-HT Zera® BTV-2 VP2 plasmid. Total plant protein was extracted on day 5 post infiltration and separated using SDS-PAGE. The proteins were then transferred to nitrocellulose membrane and probed with sheep anti-BTV-8, rabbit anti-BTV-8 VP2 and anti-Zera® primary antibody (Figure 8). The Zera® BTV-2 VP2 protein was successfully detected using the anti-Zera® antibody (Figure 8, lane A2). However as with the BTV-2 VP2, the sheep anti-BTV-8 and rabbit anti-BTV-8 VP2 failed to detect the Zera BTV-2 VP2 protein (Figure 8, lane A5 and B2). In contrast both the sheep anti-serum and rabbit anti-BTV-8 VP2 reacted strongly with a plant-produced BTV-8 VP2 positive control as expected.

An optimal optical density (1.5) at which to infiltrate *Agrobacterium* was determined as well as optimal time for harvesting (5dpi). After optimisation, proteins were expressed and small scale production and purification of Zera® protein bodies commenced using density gradient centrifugation.

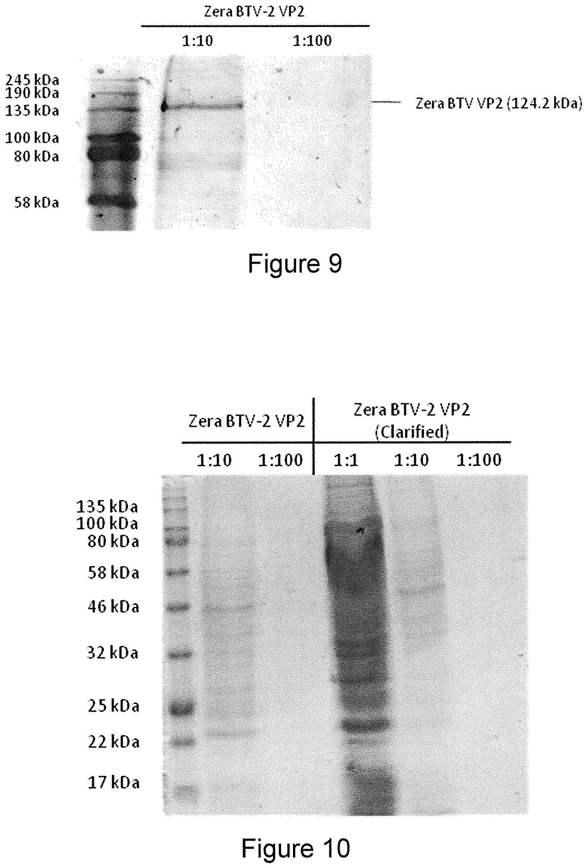

Preliminary results show that the Zera® BTV-2 VP2 protein was present in purified fractions as detected by immunoblotting (Figure 9) but looks as if there are other impurities which have co-purified with the protein bodies as seen by SDS-PAGE Coomassie staining (Figure 10). In addition, infiltrated leaf sections have been prepared and stained in order to look for protein bodies.

### REFERENCES

Barratt-Boyes, S. M., et al. (1995) Vet Immunol Immunopathol, 45, 73-84.
Capocefalo, A., et al (2010) Journal of Virological Methods, 169, 420-424.
Demaula, C. D., et al. (2000) Virus Res, 67, 59-66.
Huismans, H. & Erasmus, B. (1981) Onderstepoort J Vet Res, 48, 51 - 58.
Jaafar, F.M., et al (2014) Vaccine, 32, 4059-4067.
Joseph, M., et al. (2012) BMC Biol., 12, 36.
Liu, F., et al. (2012) Research in Veterinary Science, 93, 553-559.
Llompart, B., et al. (2010) Process Biochemistry, 45, 1816-1820.
Llop-Tous, I., et al. (2011) PloS ONE, 6, e19474.
Maan, N. S., et al. (2012) PloS ONE, 7, 32601.
Maan, S., et al. (2010) PloS ONE, 5, e10323.
Mertens, P. P. & Diprose, J. (2004) Virus Res, 101, 29-43.
Thuenemann, E. C., et al (2013) Current Pharmaceutical Design, 19, 5564-5573.
Torrent, M., et al. (2009a) BMC Biol., 7, 5.
Torrent, M., et al. (2009b) Methods in Molecular Biology, 483, 193-208.

## Claims

1. An insoluble fusion protein comprising a bluetongue virus VP2 polypeptide linked to a maize γ-zein peptide sequence.

2. The insoluble fusion protein of claim 1, wherein the bluetongue virus VP2 polypeptide is selected from the group consisting of a VP2 polypeptide of the serotype: BTV-1, BTV-2, BTV-3, BTV-4, BTV-5, BTV-6, BTV-7, BTV-8, BTV-9, BTV-10, BTV-11, BTV-12, BTV-13, BTV-14 and BTV-22.

3. The insoluble fusion protein of claim 1 or 2, wherein the maize γ-zein peptide sequence comprises a sequence of SEQ ID NO:2 or an amino acid sequence having at least 90% sequence identity to the sequence of SEQ ID NO:2.

4. The insoluble fusion protein of any one of claims 1 to 3, wherein the fusion protein forms a protein body.

5. The insoluble fusion protein of any one of claims 1 to 4, wherein the fusion protein is expressed in and recovered from a plant.

6. A nucleic acid encoding the insoluble fusion protein of any one of claims 1 to 5.

7. A vaccine composition comprising an insoluble fusion protein of any one of claims 1 to 5 and a pharmaceutically acceptable diluent or excipient, wherein said vaccine composition is capable of eliciting a protective immune response against bluetongue virus.

8. The vaccine composition of claim 7, wherein the insoluble fusion protein is present in an oil in water emulsion vehicle.

9. The vaccine composition of claims 7 or 8, containing a combination of insoluble fusion proteins of different serotypes.

10. The vaccine composition of any one of claims 7 to 9, for use in inducing an immune response against bluetongue virus.

11. Use of an insoluble fusion protein of any one of claims 1 to 4 in the manufacture of a vaccine for use in a method of preventing bluetongue virus infection in a subject, comprising administering a therapeutically effective amount of the vaccine to the subject.

12. An expression vector comprising the nucleic acid of claim 6.

13. A method of producing an insoluble fusion protein of any one of claims 1 to 5 in a plant, the method comprising the steps of:
(i) transforming or infiltrating a plant cell with the expression vector of claim 12;
(ii) expressing the insoluble fusion protein in the plant cell; and
(iii) recovering the insoluble fusion protein from the plant cell.

## Patentansprüche

1. Unlösliches Fusionsprotein, umfassend ein Blauzungenvirus-VP2-Polypeptid, das an eine Mais-γ-Zein-Peptidsequenz gebunden ist.

2. Unlösliches Fusionsprotein nach Anspruch 1, wobei das Blauzungenvirus-VP2-Polypeptid ausgewählt ist aus der Gruppe bestehend aus einem VP2-Polypeptid des Serotyps: BTV-1, BTV-2, BTV-3, BTV-4, BTV-5, BTV-6, BTV-7, BTV-8, BTV-9, BTV-10, BTV-11, BTV-12, BTV-13, BTV-14 und BTV-22.

3. Unlösliches Fusionsprotein nach Anspruch 1 oder 2, wobei die Mais-γ-Zein-Peptidsequenz eine Sequenz von SEQ ID NO: 2 oder eine Aminosäuresequenz mit mindestens 90% Sequenzidentität zu der Sequenz von SEQ ID NO: 2 umfasst.

4. Unlösliches Fusionsprotein nach einem der Ansprüche 1 bis 3, wobei das Fusionsprotein einen Proteinkörper bildet.

5. Unlösliches Fusionsprotein nach einem der Ansprüche 1 bis 4, wobei das Fusionsprotein in einer Pflanze exprimiert und daraus gewonnen wird.

6. Nukleinsäure, die das unlösliche Fusionsprotein nach einem der Ansprüche 1 bis 5 codiert.

7. Impfstoffzusammensetzung, umfassend ein unlösliches Fusionsprotein nach einem der Ansprüche 1 bis 5 und ein pharmazeutisch annehmbares Verdünnungsmittel oder einen pharmazeutisch annehmbaren Exzipienten, wobei die Impfstoffzusammensetzung zum Auslösen einer schützenden Immunantwort gegen Blauzungenvirus in der Lage ist.

8. Impfstoffzusammensetzung nach Anspruch 7, wobei das unlösliche Fusionsprotein in einem Öl-in-Wasser-Emulsionsvehikel vorliegt.

9. Impfstoffzusammensetzung nach Anspruch 7 oder 8, enthaltend eine Kombination aus unlöslichen Fusionproteinen verschiedener Serotypen.

10. Impfstoffzusammensetzung nach einem der Ansprüche 7 bis 9 zur Verwendung beim Induzieren einer Immunantwort gegen Blauzungenvirus.

11. Verwendung eines unlöslichen Fusionsproteins nach einem der Ansprüche 1 bis 4 bei der Herstellung eines Impfstoffs zur Verwendung in einem Verfahren zum Verhindern einer Blauzungenvirus-Infektion in einem Subjekt, umfassend das Verabreichen einer therapeutisch wirksamen Menge des Impfstoffs an das Subjekt.

12. Expressionsvektor, umfassend die Nukleinsäure nach Anspruch 6.

13. Verfahren zur Herstellung eines unlöslichen Fusionsproteins nach einem der Ansprüche 1 bis 5 in einer Pflanze, wobei das Verfahren die folgenden Schritte umfasst:
(i) Transformieren oder Infiltrieren einer Pflanzenzelle mit dem Expressionsvektor nach Anspruch 12;
(ii) Exprimieren des unlöslichen Fusionsproteins in der Pflanzenzelle; und
(iii) Gewinnen des unlöslichen Fusionsproteins aus der Pflanzenzelle.

## Revendications

1. Protéine de fusion insoluble comprenant un polypeptide VP2 du virus de la fièvre catarrhale du mouton lié à une séquence peptidique de γ-zéine de maïs.

2. Protéine de fusion insoluble selon la revendication 1, dans laquelle le polypeptide VP2 du virus de la fièvre catarrhale du mouton est choisi dans le groupe constitué d'un polypeptide VP2 de sérotype : BTV-1, BTV-2, BTV-3, BTV-4, BTV-5, BTV-6, BTV-7, BTV-8, BTV-9, BTV-10, BTV-11, BTV-12, BTV-13, BTV-14 et BTV-22.

3. Protéine de fusion insoluble selon la revendication 1 ou 2, dans laquelle la séquence peptidique de γ-zéine de maïs comprend une séquence de SEQ ID NO : 2 ou une séquence d'acides aminés ayant au moins 90 % d'identité de séquence avec la séquence de SEQ ID NO : 2.

4. Protéine de fusion insoluble selon l'une quelconque des revendications 1 à 3, la protéine de fusion formant un corps protéique.

5. Protéine de fusion insoluble selon l'une quelconque des revendications 1 à 4, la protéine de fusion étant exprimée dans une plante et récupérée à partir de celle-ci.

6. Acide nucléique codant pour la protéine de fusion insoluble selon l'une quelconque des revendications 1 à 5.

7. Composition de vaccin comprenant une protéine de fusion insoluble selon l'une quelconque des revendications 1 à 5 et un diluant ou excipient pharmaceutiquement acceptable, ladite composition de vaccin pouvant induire une réponse immunitaire protectrice contre le virus de la fièvre catarrhale du mouton.

8. Composition de vaccin selon la revendication 7, dans laquelle la protéine de fusion insoluble est présente dans un véhicule de type émulsion huile dans eau.

9. Composition de vaccin selon les revendications 7 ou 8, contenant une combinaison de protéines de fusion insolubles de différents sérotypes.

10. Composition de vaccin selon l'une quelconque des revendications 7 à 9, pour utilisation dans l'induction d'une réponse immunitaire contre le virus de la fièvre catarrhale du mouton.

11. Utilisation d'une protéine de fusion insoluble selon l'une quelconque des revendications 1 à 4 dans la fabrication d'un vaccin pour utilisation dans un procédé de prévention d'une infection par le virus de la fièvre catarrhale du mouton chez un sujet, comprenant l'administration d'une quantité thérapeutiquement efficace du vaccin au sujet.

12. Vecteur d'expression comprenant l'acide nucléique selon la revendication 6.

13. Procédé de production d'une protéine de fusion insoluble selon l'une quelconque des revendications 1 à 5 dans une plante, le procédé comprenant les étapes de :
(i) transformation ou infiltration d'une cellule de plante avec le vecteur d'expression selon la revendication 12 ;
(ii) expression de la protéine de fusion insoluble dans la cellule de plante ; et
(iii) récupération de la protéine de fusion insoluble à partir de la cellule de plante.


### SEQUENCE LISTING

<110> University of Cape Town
<120> Synthetic BTV VP2 Fusion Protein
<130> PA159218/P
<160> 35
<170> PatentIn version 3.5
<210> 1
   <211> 339
   <212> DNA
   <213> Artificial sequence
<220>
   <223> Zera Signal Sequence
<400> 1
   atgagggtgt tgctcgttgc cctcgctctc ctggctctcg ctgcgagcgc cacctccacg      60
   catacaagcg gcggctgcgg ctgccagcca ccgccgccgg ttcatctacc gccgccggtg     120
   catctgccac ctccggttca cctgccacct ccggtgcatc tcccaccgcc ggtccacctg     180
   ccgccgccgg tccacctgcc accgccggtc catgtgccgc cgccggttca tctgccgccg     240
   ccaccatgcc actaccctac tcaaccgccc cggcctcagc ctcatcccca gccacaccca     300
   tgcccgtgcc aacagccgca tccaagcccg tgccagacc                            339
<210> 2
   <211> 113
   <212> PRT
   <213> Artificial sequence
<220>
   <223> Zera Signal Peptide
<400> 2
   Met Arg Val Leu Leu Val Ala Leu Ala Leu Leu Ala Leu Ala Ala Ser
   1               5                   10                  15
   Ala Thr Ser Thr His Thr Ser Gly Gly Cys Gly Cys Gln Pro Pro Pro
               20                  25                  30
   Pro Val His Leu Pro Pro Pro Val His Leu Pro Pro Pro Val His Leu
           35                  40                  45
   Pro Pro Pro Val His Leu Pro Pro Pro Val His Leu Pro Pro Pro Val
       50                  55                  60
   His Leu Pro Pro Pro Val His Val Pro Pro Pro Val His Leu Pro Pro
   65                  70                  75                  80
   Pro Pro Cys His Tyr Pro Thr Gln Pro Pro Arg Pro Gln Pro His Pro
                   85                  90                  95
   Gln Pro His Pro Cys Pro Cys Gln Gln Pro His Pro Ser Pro Cys Gln
               100                 105                 110
   Thr
<210> 3
   <211> 2886
   <212> DNA
   <213> Artificial sequence
<220>
   <223> Codon optimised BTV-8 VP2
<400> 3
   atggaagaac tcgctatccc aatctacact aacgttttcc cagctgagct tcttgatgga      60
   tacgattaca tcatcgatgt ttcttctagg gttgaagaag aaggcgacga gccagttaag     120
   aggcatgatg tgactgagat tcctaggaac tccatgttcg atatcaagga tgagcacatc     180
   agggatgcta ttatctacaa gccagtgaac aacgatggat acgttttgcc aagggtgctc     240
   gatattactc tcaaggcttt cgatgatagg aagcgtgttg ttcttaacga tggacactct     300
   gagtttcaca ctaagactaa ctgggtgcag tggatgattg atgatgctat ggatgtgcag     360
   ccactcaaag ttgatattgc tcacactagg tctaggattt ctcacgctct cttcaattgc     420
   actgtgaggc ttcattctaa gaaggctgat actgcttctt atcacgttga gccagtggaa     480
   attgaatctt ggggatgcaa tcacacttgg ctttctagga ttcaccacct tgttaacgtt     540
   gagcttttcc actgttctca agaggctgct tacactctta agccaactta caagatcatt     600
   tccaacgctg aaagggcttc tacttccgat tctttcaacg gaactatgat cgagcttggt     660
   aggaatcatc agatccagat gggagatcaa ggataccaga agcttaaaga gggacttgtt     720
   caggttagaa tcgagggaaa gactccactt gttatccagg aagagatcac tgctctcaac     780
   aagattcgtg agcaatggat tgctaggaat ttcgatcagc gtgagattaa ggttttggat     840
   ttgtgcaggc ttctttcaac tatcggtagg aagatgtgca atactgaaga ggaacctaag     900
   aacgaggctg atctttctgt taagttccag atggaactcg atgagatttt caggccagga     960
   aataacgaga ggactaacat tatgggaggt ggagttcata gaaagaacga ggacaggttc    1020
   tacgtgctca ttatgattgc tgcttccgat acaaacaagg gaaggatttg gtggtctaat    1080
   ccatatccat gtcttagggg tgctcttatt gctgctgagg ttcagcttgg agatgtgtac    1140
   aaccttctcc gtaattggtt ccaatggtct gttaggccaa cttacgttcc atacgatagg    1200
   aacagagagt ccgataagta catctactcc aggatcaacc ttttcgattc tactcttagg    1260
   ccaggtgata agattgtgca ctgggagtac aagcttttga acgaggttag agaggtgtca 1320
   atcaacaagg gaaacgagtg tgatttgttc ccagaggatg aagagttcac tactaagttc 1380
   catgaggcta ggtatactga gatgaagaac cagattattc agtctggatg gaaccagagg 1440
   gatttcaaga tgcacaagat ccttgaggat ggtgctaacg tgctcacaat cgattttgag 1500
   aaggatgctc acattggaac tggatctgct ctttctctcc ctgattacta caacaagtgg 1560
   attatcgctc caatgttcaa cgctaagctc aggattactg aggttgtgat tggaactgct 1620
   cacactgatg atccagctgt tggaagatct gctaaggctt tcactcacga tccattcgat 1680
   cttcaaaggt actgtctcgc taggtattat gatgttaggc caggaatgat gggtagagct 1740
   ttgtctaagc agcagaatat gtcctccatg actgataagc tttccaagca agaggattac 1800
   gctggaattg tgtcaagaag gcttgagtac aaagagagag agaaccgttg tcttactgag 1860
   actgctcagt acgttttcga aaagacttgc ctctacgttt tggagcttct ttctaggcat 1920
   acaatgccat ctgaggattc tgaagtgact ttcgagcacc caactattga tccatctgtg 1980
   gatatcgaga cttggaagat cattgatgtg tcccagctca ttatcttcgt gttcgattac 2040
   cttttcgaga accgtaagat cgttagggat acaactgaag ctaggtggac tcttttcaag 2100
   attaggtctg aggttggaag ggctaggatt gatgctatcg agatgacttt tccaaggttc 2160
   ggaaggatgc ttaggaatgc ttctcaggct aagatcaacc aggatatcgc ttgccttaac 2220
   ttccttccac tcctcttcat tatcggtgat aacatctctt acgctcatag gcaatggtct 2280
   attccagtgc ttctttacgc tcacgatatc aggattatcc cacttgaggt tggagcttac 2340
   aataacaggt tcggacttac ttcttacctt gagtacatgg ctttcttccc atcttatgct 2400
   actagggtgg caaagattga tgagtccatc aaagagtgcg ctattgctat ggctgagttc 2460
   tacatgaaca ctgatattca ctctggatcc gtgatgtcta acgtgatcac tactaagagg 2520
   cttctctacg agacttacct tgcttctctt tgcggtggat actctgatgg acttctctgg 2580
   tacttgccaa ttactcaccc atctaagtgc cttgttgctt tcgaagttgc tgatgatgtt 2640
   gtgccacttt ctgttagaag agagaggatt ctttctaggt tcccactttc ttcaaggcac 2700
   gtgaagggaa ttgctcttat ttccgtggat aggaaccaga aagtttctgt tcagactgag 2760
   ggaattgtga ctcataggct ctgcaagaag aaccttctca agtacgtttg tgatgtgatc 2820
   ctcttcaagt tctctggtca cgttttcgga aacgatgaga tgcttactaa gctcctcaac 2880
   gtttga 2886
<210> 4
   <211> 13204
   <212> DNA
   <213> Artificial sequence
<220>
   <223> pEAQ-HTZera-VP2
<400> 4
   cctgtggttg gcatgcacat acaaatggac gaacggataa accttttcac gcccttttaa      60
   atatccgatt attctaataa acgctctttt ctcttaggtt tacccgccaa tatatcctgt     120
   caaacactga tagtttgtga accatcaccc aaatcaagtt ttttggggtc gaggtgccgt     180
   aaagcactaa atcggaaccc taaagggagc ccccgattta gagcttgacg gggaaagccg     240
   gcgaacgtgg cgagaaagga agggaagaaa gcgaaaggag cgggcgccat tcaggctgcg     300
   caactgttgg gaagggcgat cggtgcgggc ctcttcgcta ttacgccagc tggcgaaagg     360
   gggatgtgct gcaaggcgat taagttgggt aacgccaggg ttttcccagt cacgacgttg     420
   taaaacgacg gccagtgaat tgttaattaa gaattcgagc tccaccgcgg aaacctcctc     480
   ggattccatt gcccagctat ctgtcacttt attgagaaga tagtggaaaa ggaaggtggc     540
   tcctacaaat gccatcattg cgataaagga aaggccatcg ttgaagatgc ctctgccgac     600
   agtggtccca aagatggacc cccacccacg aggagcatcg tggaaaaaga agacgttcca     660
   accacgtctt caaagcaagt ggattgatgt gatatctcca ctgacgtaag ggatgacgca     720
   caatcccact atccttcgca agacccttcc tctatataag gaagttcatt tcatttggag     780
   aggtattaaa atcttaatag gttttgataa aagcgaacgt ggggaaaccc gaaccaaacc     840
   ttcttctaaa ctctctctca tctctcttaa agcaaacttc tctcttgtct ttcttgcgtg     900
   agcgatcttc aacgttgtca gatcgtgctt cggcaccagt acaacgtttt ctttcactga     960
   agcgaaatca aagatctctt tgtggacacg tagtgcggcg ccattaaata acgtgtactt    1020
   gtcctattct tgtcggtgtg gtcttgggaa aagaaagctt gctggaggct gctgttcagc    1080
   cccatacatt acttgttacg attctgctga ctttcggcgg gtgcaatatc tctacttctg    1140
   cttgacgagg tattgttgcc tgtacttctt tcttcttctt cttgctgatt ggttctataa    1200
   gaaatctagt attttctttg aaacagagtt ttcccgtggt tttcgaactt ggagaaagat    1260
   tgttaagctt ctgtatattc tgcccaaatt cgcgaccggt atgagggtgt tgctcgttgc    1320
   cctcgctctc ctggctctcg ctgcgagcgc cacctccacg catacaagcg gcggctgcgg    1380
   ctgccagcca ccgccgccgg ttcatctacc gccgccggtg catctgccac ctccggttca    1440
   cctgccacct ccggtgcatc tcccaccgcc ggtccacctg ccgccgccgg tccacctgcc    1500
   accgccggtc catgtgccgc cgccggttca tctgccgccg ccaccatgcc actaccctac    1560
   tcaaccgccc cggcctcagc ctcatcccca gccacaccca tgcccgtgcc aacagccgca    1620
   tccaagcccg tgccagccat gggaagcggc ggcgaaaacg cgtatggaag aactcgctat    1680
   cccaatctac actaacgttt tcccagctga gcttcttgat ggatacgatt acatcatcga    1740
   tgtttcttct agggttgaag aagaaggcga cgagccagtt aagaggcatg atgtgactga    1800
   gattcctagg aactccatgt tcgatatcaa ggatgagcac atcagggatg ctattatcta 1860
   caagccagtg aacaacgatg gatacgtttt gccaagggtg ctcgatatta ctctcaaggc 1920
   tttcgatgat aggaagcgtg ttgttcttaa cgatggacac tctgagtttc acactaagac 1980
   taactgggtg cagtggatga ttgatgatgc tatggatgtg cagccactca aagttgatat 2040
   tgctcacact aggtctagga tttctcacgc tctcttcaat tgcactgtga ggcttcattc 2100
   taagaaggct gatactgctt cttatcacgt tgagccagtg gaaattgaat cttggggatg 2160
   caatcacact tggctttcta ggattcacca ccttgttaac gttgagcttt tccactgttc 2220
   tcaagaggct gcttacactc ttaagccaac ttacaagatc atttccaacg ctgaaagggc 2280
   ttctacttcc gattctttca acggaactat gatcgagctt ggtaggaatc atcagatcca 2340
   gatgggagat caaggatacc agaagcttaa agagggactt gttcaggtta gaatcgaggg 2400
   aaagactcca cttgttatcc aggaagagat cactgctctc aacaagattc gtgagcaatg 2460
   gattgctagg aatttcgatc agcgtgagat taaggttttg gatttgtgca ggcttctttc 2520
   aactatcggt aggaagatgt gcaatactga agaggaacct aagaacgagg ctgatctttc 2580
   tgttaagttc cagatggaac tcgatgagat tttcaggcca ggaaataacg agaggactaa 2640
   cattatggga ggtggagttc atagaaagaa cgaggacagg ttctacgtgc tcattatgat 2700
   tgctgcttcc gatacaaaca agggaaggat ttggtggtct aatccatatc catgtcttag 2760
   gggtgctctt attgctgctg aggttcagct tggagatgtg tacaaccttc tccgtaattg 2820
   gttccaatgg tctgttaggc caacttacgt tccatacgat aggaacagag agtccgataa 2880
   gtacatctac tccaggatca accttttcga ttctactctt aggccaggtg ataagattgt 2940
   gcactgggag tacaagcttt tgaacgaggt tagagaggtg tcaatcaaca agggaaacga 3000
   gtgtgatttg ttcccagagg atgaagagtt cactactaag ttccatgagg ctaggtatac 3060
   tgagatgaag aaccagatta ttcagtctgg atggaaccag agggatttca agatgcacaa 3120
   gatccttgag gatggtgcta acgtgctcac aatcgatttt gagaaggatg ctcacattgg 3180
   aactggatct gctctttctc tccctgatta ctacaacaag tggattatcg ctccaatgtt 3240
   caacgctaag ctcaggatta ctgaggttgt gattggaact gctcacactg atgatccagc 3300
   tgttggaaga tctgctaagg ctttcactca cgatccattc gatcttcaaa ggtactgtct 3360
   cgctaggtat tatgatgtta ggccaggaat gatgggtaga gctttgtcta agcagcagaa 3420
   tatgtcctcc atgactgata agctttccaa gcaagaggat tacgctggaa ttgtgtcaag 3480
   aaggcttgag tacaaagaga gagagaaccg ttgtcttact gagactgctc agtacgtttt 3540
   cgaaaagact tgcctctacg ttttggagct tctttctagg catacaatgc catctgagga 3600
   ttctgaagtg actttcgagc acccaactat tgatccatct gtggatatcg agacttggaa 3660
   gatcattgat gtgtcccagc tcattatctt cgtgttcgat taccttttcg agaaccgtaa 3720
   gatcgttagg gatacaactg aagctaggtg gactcttttc aagattaggt ctgaggttgg 3780
   aagggctagg attgatgcta tcgagatgac ttttccaagg ttcggaagga tgcttaggaa 3840
   tgcttctcag gctaagatca accaggatat cgcttgcctt aacttccttc cactcctctt 3900
   cattatcggt gataacatct cttacgctca taggcaatgg tctattccag tgcttcttta 3960
   cgctcacgat atcaggatta tcccacttga ggttggagct tacaataaca ggttcggact 4020
   tacttcttac cttgagtaca tggctttctt cccatcttat gctactaggg tggcaaagat 4080
   tgatgagtcc atcaaagagt gcgctattgc tatggctgag ttctacatga acactgatat 4140
   tcactctgga tccgtgatgt ctaacgtgat cactactaag aggcttctct acgagactta 4200
   ccttgcttct ctttgcggtg gatactctga tggacttctc tggtacttgc caattactca 4260
   cccatctaag tgccttgttg ctttcgaagt tgctgatgat gttgtgccac tttctgttag 4320
   aagagagagg attctttcta ggttcccact ttcttcaagg cacgtgaagg gaattgctct 4380
   tatttccgtg gataggaacc agaaagtttc tgttcagact gagggaattg tgactcatag 4440
   gctctgcaag aagaaccttc tcaagtacgt ttgtgatgtg atcctcttca agttctctgg 4500
   tcacgttttc ggaaacgatg agatgcttac taagctcctc aacgtttgac tcgaggcctt 4560
   taactctggt ttcattaaat tttctttagt ttgaatttac tgttattcgg tgtgcatttc 4620
   tatgtttggt gagcggtttt ctgtgctcag agtgtgttta ttttatgtaa tttaatttct 4680
   ttgtgagctc ctgtttagca ggtcgtccct tcagcaagga cacaaaaaga ttttaatttt 4740
   attaaaaaaa aaaaaaaaaa agaccgggaa ttcgatatca agcttatcga cctgcagatc 4800
   gttcaaacat ttggcaataa agtttcttaa gattgaatcc tgttgccggt cttgcgatga 4860
   ttatcatata atttctgttg aattacgtta agcatgtaat aattaacatg taatgcatga 4920
   cgttatttat gagatgggtt tttatgatta gagtcccgca attatacatt taatacgcga 4980
   tagaaaacaa aatatagcgc gcaaactagg ataaattatc gcgcgcggtg tcatctatgt 5040
   tactagatct ctagagtctc aagcttggcg cgccagcttg gcgtaatcat ggtcatagct 5100
   gttgcgatta agaattcgag ctcggtaccc ccctactcca aaaatgtcaa agatacagtc 5160
   tcagaagacc aaagggctat tgagactttt caacaaaggg taatttcggg aaacctcctc 5220
   ggattccatt gcccagctat ctgtcacttc atcgaaagga cagtagaaaa ggaaggtggc 5280
   tcctacaaat gccatcattg cgataaagga aaggctatca ttcaagatgc ctctgccgac 5340
   agtggtccca aagatggacc cccacccacg aggagcatcg tggaaaaaga agacgttcca 5400
   accacgtctt caaagcaagt ggattgatgt gacatctcca ctgacgtaag ggatgacgca 5460
   caatcccact atccttcgca agacccttcc tctatataag gaagttcatt tcatttggag     5520
   aggacagccc aagcttcgac tctagaggat ccccttaaat cgatatggaa cgagctatac     5580
   aaggaaacga cgctagggaa caagctaaca gtgaacgttg ggatggagga tcaggaggta 5640
   ccacttctcc cttcaaactt cctgacgaaa gtccgagttg gactgagtgg cggctacata 5700
   acgatgagac gaattcgaat caagataatc cccttggttt caaggaaagc tggggtttcg 5760
   ggaaagttgt atttaagaga tatctcagat acgacaggac ggaagcttca ctgcacagag 5820
   tccttggatc ttggacggga gattcggtta actatgcagc atctcgattt ttcggtttcg 5880
   accagatcgg atgtacctat agtattcggt ttcgaggagt tagtatcacc gtttctggag 5940
   ggtctcgaac tcttcagcat ctctgtgaga tggcaattcg gtctaagcaa gaactgctac 6000
   agcttgcccc aatcgaagtg gaaagtaatg tatcaagagg atgccctgaa ggtactgaga 6060
   ccttcgaaaa agaaagcgag taaggggagc tcgaattcgc tgaaatcacc agtctctctc 6120
   tacaaatcta tctctctcta ttttctccat aaataatgtg tgagtagttt cccgataagg 6180
   gaaattaggg ttcttatagg gtttcgctca tgtgttgagc atataagaaa cccttagtat 6240
   gtatttgtat ttgtaaaata cttctatcaa taaaatttct aattcctaaa accaaaatcc 6300
   agtactaaaa tccagatctc ctaaagtccc tatagatctt tgtcgtgaat ataaaccaga 6360
   cacgagacga ctaaacctgg agcccagacg ccgttcgaag ctagaagtac cgcttaggca 6420
   ggaggccgtt agggaaaaga tgctaaggca gggttggtta cgttgactcc cccgtaggtt 6480
   tggtttaaat atgatgaagt ggacggaagg aaggaggaag acaaggaagg ataaggttgc 6540
   aggccctgtg caaggtaaga agatggaaat ttgatagagg tacgctacta tacttatact 6600
   atacgctaag ggaatgcttg tatttatacc ctataccccc taataacccc ttatcaattt 6660
   aagaaataat ccgcataagc ccccgcttaa aaattggtat cagagccatg aataggtcta 6720
   tgaccaaaac tcaagaggat aaaacctcac caaaatacga aagagttctt aactctaaag 6780
   ataaaagatg gcgcgtggcc ggcctacagt atgagcggag aattaaggga gtcacgttat 6840
   gacccccgcc gatgacgcgg gacaagccgt tttacgtttg gaactgacag aaccgcaacg 6900
   ttgaaggagc cactcagccg cgggtttctg gagtttaatg agctaagcac atacgtcaga 6960
   aaccattatt gcgcgttcaa aagtcgccta aggtcactat cagctagcaa atatttcttg 7020
   tcaaaaatgc tccactgacg ttccataaat tcccctcggt atccaattag agtctcatat 7080
   tcactctcaa tccaaataat ctgcaccgga tctggatcgt ttcgcatgat tgaacaagat 7140
   ggattgcacg caggttctcc ggccgcttgg gtggagaggc tattcggcta tgactgggca 7200
   caacagacaa tcggctgctc tgatgccgcc gtgttccggc tgtcagcgca ggggcgcccg 7260
   gttctttttg tcaagaccga cctgtccggt gccctgaatg aactgcagga cgaggcagcg 7320
   cggctatcgt ggctggccac gacgggcgtt ccttgcgcag ctgtgctcga cgttgtcact 7380
   gaagcgggaa gggactggct gctattgggc gaagtgccgg ggcaggatct cctgtcatct     7440
   caccttgctc ctgccgagaa agtatccatc atggctgatg caatgcggcg gctgcatacg     7500
   cttgatccgg ctacctgccc attcgaccac caagcgaaac atcgcatcga gcgagcacgt 7560
   actcggatgg aagccggtct tgtcgatcag gatgatctgg acgaagagca tcaggggctc 7620
   gcgccagccg aactgttcgc caggctcaag gcgcgcatgc ccgacggcga tgatctcgtc 7680
   gtgacccatg gcgatgcctg cttgccgaat atcatggtgg aaaatggccg cttttctgga 7740
   ttcatcgact gtggccggct gggtgtggcg gaccgctatc aggacatagc gttggctacc 7800
   cgtgatattg ctgaagagct tggcggcgaa tgggctgacc gcttcctcgt gctttacggt 7860
   atcgccgctc ccgattcgca gcgcatcgcc ttctatcgcc ttcttgacga gttcttctga 7920
   gcgggactct ggggttcgaa atgaccgacc aagcgacgcc caacctgcca tcacgagatt 7980
   tcgattccac cgccgccttc tatgaaaggt tgggcttcgg aatcgttttc cgggacgccg 8040
   gctggatgat cctccagcgc ggggatctca tgctggagtt cttcgcccac gggatctctg 8100
   cggaacaggc ggtcgaaggt gccgatatca ttacgacagc aacggccgac aagcacaacg 8160
   ccacgatcct gagcgacaat atgatcgcgg cgtccacatc aacggcgtcg gcggcgactg 8220
   cccaggcaag accgagatgc accgcgatat cttgctgcgt tcggatattt tcgtggagtt 8280
   cccgccacag acccggatga tccccgatcg ttcaaacatt tggcaataaa gtttcttaag 8340
   attgaatcct gttgccggtc ttgcgatgat tatcatataa tttctgttga attacgttaa 8400
   gcatgtaata attaacatgt aatgcatgac gttatttatg agatgggttt ttatgattag 8460
   agtcccgcaa ttatacattt aatacgcgat agaaaacaaa atatagcgcg caaactagga 8520
   taaattatcg cgcgcggtgt catctatgtt actagatcgg gactgtaggc cggccctcac 8580
   tggtgaaaag aaaaaccacc ccagtacatt aaaaacgtcc gcaatgtgtt attaagttgt 8640
   ctaagcgtca atttgtttac accacaatat atcctgccac cagccagcca acagctcccc 8700
   gaccggcagc tcggcacaaa atcaccactc gatacaggca gcccatcagt ccgggacggc 8760
   gtcagcggga gagccgttgt aaggcggcag actttgctca tgttaccgat gctattcgga 8820
   agaacggcaa ctaagctgcc gggtttgaaa cacggatgat ctcgcggagg gtagcatgtt 8880
   gattgtaacg atgacagagc gttgctgcct gtgatcaaat atcatctccc tcgcagagat 8940
   ccgaattatc agccttctta ttcatttctc gcttaaccgt gacagagtag acaggctgtc 9000
   tcgcggccga ggggcgcagc ccctgggggg gatgggaggc ccgcgttagc gggccgggag 9060
   ggttcgagaa gggggggcac cccccttcgg cgtgcgcggt cacgcgcaca gggcgcagcc 9120
   ctggttaaaa acaaggttta taaatattgg tttaaaagca ggttaaaaga caggttagcg 9180
   gtggccgaaa aacgggcgga aacccttgca aatgctggat tttctgcctg tggacagccc 9240
   ctcaaatgtc aataggtgcg cccctcatct gtcagcactc tgcccctcaa gtgtcaagga 9300
   tcgcgcccct catctgtcag tagtcgcgcc cctcaagtgt caataccgca gggcacttat 9360
   ccccaggctt gtccacatca tctgtgggaa actcgcgtaa aatcaggcgt tttcgccgat     9420
   ttgcgaggct ggccagctcc acgtcgccgg ccgaaatcga gcctgcccct catctgtcaa     9480
   cgccgcgccg ggtgagtcgg cccctcaagt gtcaacgtcc gcccctcatc tgtcagtgag     9540
   ggccaagttt tccgcgaggt atccacaacg ccggcggccg cggtgtctcg cacacggctt     9600
   cgacggcgtt tctggcgcgt ttgcagggcc atagacggcc gccagcccag cggcgagggc     9660
   aaccagcccg gtgagcgtcg gaaaggcgct cggtcttgcc ttgctcgtcg gtgatgtaca     9720
   ctagtcgctg gctgctgaac ccccagccgg aactgacccc acaaggccct agcgtttgca      9780
   atgcaccagg tcatcattga cccaggcgtg ttccaccagg ccgctgcctc gcaactcttc     9840
   gcaggcttcg ccgacctgct cgcgccactt cttcacgcgg gtggaatccg atccgcacat     9900
   gaggcggaag gtttccagct tgagcgggta cggctcccgg tgcgagctga aatagtcgaa     9960
   catccgtcgg gccgtcggcg acagcttgcg gtacttctcc catatgaatt tcgtgtagtg    10020
   gtcgccagca aacagcacga cgatttcctc gtcgatcagg acctggcaac gggacgtttt    10080
   cttgccacgg tccaggacgc ggaagcggtg cagcagcgac accgattcca ggtgcccaac    10140
   gcggtcggac gtgaagccca tcgccgtcgc ctgtaggcgc gacaggcatt cctcggcctt    10200
   cgtgtaatac cggccattga tcgaccagcc caggtcctgg caaagctcgt agaacgtgaa    10260
   ggtgatcggc tcgccgatag gggtgcgctt cgcgtactcc aacacctgct gccacaccag    10320
   ttcgtcatcg tcggcccgca gctcgacgcc ggtgtaggtg atcttcacgt ccttgttgac    10380
   gtggaaaatg accttgtttt gcagcgcctc gcgcgggatt ttcttgttgc gcgtggtgaa    10440
   cagggcagag cgggccgtgt cgtttggcat cgctcgcatc gtgtccggcc acggcgcaat    10500
   atcgaacaag gaaagctgca tttccttgat ctgctgcttc gtgtgtttca gcaacgcggc    10560
   ctgcttggcc tcgctgacct gttttgccag gtcctcgccg gcggtttttc gcttcttggt    10620
   cgtcatagtt cctcgcgtgt cgatggtcat cgacttcgcc aaacctgccg cctcctgttc    10680
   gagacgacgc gaacgctcca cggcggccga tggcgcgggc agggcagggg gagccagttg    10740
   cacgctgtcg cgctcgatct tggccgtagc ttgctggacc atcgagccga cggactggaa    10800
   ggtttcgcgg ggcgcacgca tgacggtgcg gcttgcgatg gtttcggcat cctcggcgga    10860
   aaaccccgcg tcgatcagtt cttgcctgta tgccttccgg tcaaacgtcc gattcattca    10920
   ccctccttgc gggattgccc cgactcacgc cggggcaatg tgcccttatt cctgatttga    10980
   cccgcctggt gccttggtgt ccagataatc caccttatcg gcaatgaagt cggtcccgta    11040
   gaccgtctgg ccgtccttct cgtacttggt attccgaatc ttgccctgca cgaataccag    11100
   cgaccccttg cccaaatact tgccgtgggc ctcggcctga gagccaaaac acttgatgcg    11160
   gaagaagtcg gtgcgctcct gcttgtcgcc ggcatcgttg cgccacatct aggtactaaa    11220
   acaattcatc cagtaaaata taatatttta ttttctccca atcaggcttg atccccagta 11280
   agtcaaaaaa tagctcgaca tactgttctt ccccgatatc ctccctgatc gaccggacgc 11340
   agaaggcaat gtcataccac ttgtccgccc tgccgcttct cccaagatca ataaagccac 11400
   ttactttgcc atctttcaca aagatgttgc tgtctcccag gtcgccgtgg gaaaagacaa 11460
   gttcctcttc gggcttttcc gtctttaaaa aatcatacag ctcgcgcgga tctttaaatg 11520
   gagtgtcttc ttcccagttt tcgcaatcca catcggccag atcgttattc agtaagtaat 11580
   ccaattcggc taagcggctg tctaagctat tcgtataggg acaatccgat atgtcgatgg 11640
   agtgaaagag cctgatgcac tccgcataca gctcgataat cttttcaggg ctttgttcat 11700
   cttcatactc ttccgagcaa aggacgccat cggcctcact catgagcaga ttgctccagc 11760
   catcatgccg ttcaaagtgc aggacctttg gaacaggcag ctttccttcc agccatagca 11820
   tcatgtcctt ttcccgttcc acatcatagg tggtcccttt ataccggctg tccgtcattt 11880
   ttaaatatag gttttcattt tctcccacca gcttatatac cttagcagga gacattcctt 11940
   ccgtatcttt tacgcagcgg tatttttcga tcagtttttt caattccggt gatattctca 12000
   ttttagccat ttattatttc cttcctcttt tctacagtat ttaaagatac cccaagaagc 12060
   taattataac aagacgaact ccaattcact gttccttgca ttctaaaacc ttaaatacca 12120
   gaaaacagct ttttcaaagt tgttttcaaa gttggcgtat aacatagtat cgacggagcc 12180
   gattttgaaa ccacaattat gggtgatgct gccaacttac tgatttagtg tatgatggtg 12240
   tttttgaggt gctccagtgg cttctgtttc tatcagctgt ccctcctgtt cagctactga 12300
   cggggtggtg cgtaacggca aaagcaccgc cggacatcag cgctatctct gctctcactg 12360
   ccgtaaaaca tggcaactgc agttcactta caccgcttct caacccggta cgcaccagaa 12420
   aatcattgat atggccatga atggcgttgg atgccgggca acagcccgca ttatgggcgt 12480
   tggcctcaac acgattttac gtcacttaaa aaactcaggc cgcagtcggt aactatgcgg 12540
   tgtgaaatac cgcacagatg cgtaaggaga aaataccgca tcaggcgctc ttccgcttcc 12600
   tcgctcactg actcgctgcg ctcggtcgtt cggctgcggc gagcggtatc agctcactca 12660
   aaggcggtaa tacggttatc cacagaatca ggggataacg caggaaagaa catgtgagca 12720
   aaaggccagc aaaaggccag gaaccgtaaa aaggccgcgt tgctggcgtt tttccatagg 12780
   ctccgccccc ctgacgagca tcacaaaaat cgacgctcaa gtcagaggtg gcgaaacccg 12840
   acaggactat aaagatacca ggcgtttccc cctggaagct ccctcgtgcg ctctcctgtt 12900
   ccgaccctgc cgcttaccgg atacctgtcc gcctttctcc cttcgggaag cgtggcgctt 12960
   tctcatagct cacgctgtag gtatctcagt tcggtgtagg tcgttcgctc caagctgggc 13020
   tgtgtgcacg aaccccccgt tcagcccgac cgctgcgcct tatccggtaa ctatcgtctt 13080
   gagtccaacc cggtaagaca cgacttatcg ccactggcag caggtaacct cgcgcataca 13140
   gccgggcagt gacgtcatcg tctgcgcgga aatggacggg cccccggcgc cagatctggg 13200
   gaac 13204
<210> 5
   <211> 961
   <212> PRT
   <213> Artificial sequence
<220>
   <223> BTV-1 VP2 consensus sequence
<400> 5
   Met Asp Glu Leu Gly Ile Pro Val Tyr Lys Arg Gly Phe Pro Glu His
   1 5 10 15
   Leu Leu Arg Gly Tyr Glu Phe Ile Ile Asp Val Gly Thr Lys Ile Glu
   20 25 30
   Ser Val Gly Gly Arg His Asp Val Thr Lys Ile Pro Glu Met Asn Ala
   35 40 45
   Tyr Asp Ile Lys Gln Glu Ser Ile Arg Thr Ala Leu Trp Tyr Asn Pro
   50 55 60
   Ile Arg Asn Asp Gly Phe Val Leu Pro Arg Val Leu Asp Ile Thr Leu
   65 70 75 80
   Arg Gly Tyr Asp Glu Arg Arg Ala Val Val Glu Ser Thr Arg His Lys
   85 90 95
   Ser Phe His Thr Asn Asp Gln Trp Val Gln Trp Met Met Lys Asp Ser
   100 105 110
   Met Asp Ala Gln Pro Leu Lys Val Gly Leu Asp Asp Gln Ser Arg Asn
   115 120 125
   Val Ala His Ser Leu His Asn Cys Val Val Lys Ile Asp Ser Lys Lys
   130 135 140
   Ala Asp Thr Met Ser Tyr His Val Glu Pro Ile Glu Asp Ala Ser Lys
   145 150 155 160
   Gly Cys Leu His Thr Arg Thr Met Met Trp Asn His Leu Val Arg Ile
   165 170 175
   Glu Thr Phe His Ala Ala Gln Glu Val Ala Tyr Thr Leu Lys Pro Thr
   180 185 190
   Tyr Asp Ile Val Val His Ala Glu Arg Arg Asp Arg Ser Gln Pro Phe
           195                 200                 205
   Arg Pro Gly Asp Gln Thr Leu Ile Asn Phe Gly Arg Gly Gln Lys Val
       210                 215                 220
   Thr Met Asn His Asn Ser Tyr Asp Lys Met Val Glu Gly Leu Ala His
   225                 230                 235                 240
   Leu Val Ile Arg Gly Lys Thr Pro Glu Val Ile Arg Asp Asp Ile Ala
                   245                 250                 255
   Ser Leu Asp Glu Ile Cys Asn Arg Trp Ile Gln Ser Arg His Asp Pro
               260                 265                 270
   Gly Glu Ile Lys Ala Tyr Glu Leu Cys Lys Ile Leu Ser Thr Ile Gly
           275                 280                 285
   Arg Lys Val Leu Asp Arg Glu Lys Glu Pro Glu Asp Glu Ala Ser Leu
       290                 295                 300
   Ser Ile Arg Phe Gln Glu Ala Ile Asp Asn Lys Phe Arg Gln His Asp
   305                 310                 315                 320
   Pro Glu Arg Leu Lys Ile Phe Glu His Arg Asn Gln Arg Arg Asp Glu
                   325                 330                 335
   Asp Arg Phe Tyr Ile Leu Leu Met Ile Ala Ala Ser Asp Thr Phe Asn
               340                 345                 350
   Thr Arg Val Trp Trp Ser Asn Pro Tyr Pro Cys Leu Arg Gly Thr Leu
           355                 360                 365
   Ile Ala Ser Glu Thr Lys Leu Gly Asp Val Tyr Ser Met Met Arg Ser
       370                 375                 380
   Trp Tyr Asp Trp Ser Val Arg Pro Thr Tyr Thr Pro Tyr Glu Lys Thr
   385                 390                 395                 400
   Arg Glu Gln Glu Lys Tyr Ile Tyr Gly Arg Val Asn Leu Phe Asp Phe
                   405                 410                 415
   Val Ala Glu Pro Gly Ile Lys Ile Val His Trp Glu Tyr Arg Leu Asn
               420                 425                 430
   His Ser Thr Arg Glu Ile Thr Tyr Ala Gln Gly Asn Pro Cys Asp Leu
           435                 440                 445
   Tyr Pro Glu Asp Asp Asp Val Ile Val Thr Lys Phe Asp Asp Val Ala
   450 455 460
   Tyr Gly Gln Met Ile Asn Glu Met Ile Asn Gly Gly Trp Asn Gln Glu
   465 470 475 480
   Gln Phe Lys Met His Lys Ile Leu Lys Ser Glu Gly Asn Val Leu Thr
   485 490 495
   Ile Asp Phe Glu Lys Asp Ala Lys Leu Thr Thr Asn Glu Gly Val Thr
   500 505 510
   Met Pro Glu Tyr Phe Asn Lys Trp Ile Ile Ala Pro Met Phe Asn Ala
   515 520 525
   Lys Leu Arg Ile Lys His Glu Glu Ile Ala Gln Arg Gln Ser Asp Asp
   530 535 540
   Pro Met Val Lys Arg Thr Leu Ser Pro Ile Thr Ala Asp Pro Ile Glu
   545 550 555 560
   Leu Gln Arg Leu Thr Leu Ala Arg Phe Tyr Asp Ile Arg Pro Ala Leu
   565 570 575
   Arg Gly Gln Ala Leu Ser Arg Gln Gln Ala Gln Ser Thr Tyr Asp Glu
   580 585 590
   Glu Ile Ser Lys Arg Gln Asp Tyr Ala Glu Ile Leu Lys Arg Arg Gly
   595 600 605
   Ile Val Gln Ile Pro Lys Lys Pro Cys Pro Thr Val Thr Ala Gln Tyr
   610 615 620
   Thr Leu Glu Arg Tyr Ala Leu Phe Ile Ile Asn Ile Leu Gln Gln His
   625 630 635 640
   Val Val Arg Asp Cys Asp Glu Glu Ala Ile Tyr Glu His Pro Lys Ala
   645 650 655
   Asp His Glu Leu Glu Ile Phe Gly Glu Ser Ile Val Asp Ile Ser Gln
   660 665 670
   Val Ile Ile Leu Val Phe Asp Leu Ile Phe Glu Arg Arg Arg Arg Val
   675 680 685
   Arg Asp Val Tyr Glu Ser Arg Tyr Ile Ile Ala Arg Ile Arg Arg Met
       690                 695                 700
   Arg Gly Lys Glu Arg Leu Asn Val Ile Ala Glu Phe Phe Pro Thr Tyr
   705                 710                 715                 720
   Gly Gly Leu Leu Asn Gly Leu Asn Ser Ala Thr Val Val Gln Asp Ile
                   725                 730                 735
   Met Tyr Leu Asn Phe Leu Pro Leu Tyr Phe Leu Val Gly Asp Asn Met
               740                 745                 750
   Ile Tyr Ser His Arg Gln Trp Ser Ile Pro Leu Leu Leu Tyr Thr His
           755                 760                 765
   Glu Val Met Val Val Pro Leu Glu Val Gly Ser Tyr Asn Asp Arg Cys
       770                 775                 780
   Gly Leu Ile Ala Tyr Leu Glu Tyr Met Val Phe Phe Pro Ser Lys Ala
   785                 790                 795                 800
   Ile Arg Phe Ser Lys Leu Asn Glu Ala Gln Pro Lys Ile Ala Arg Glu
                   805                 810                 815
   Met Leu Lys Tyr Tyr Ala Asn Thr Thr Val Tyr Asp Gly Gly Val Asn
               820                 825                 830
   Tyr Asn Val Val Thr Thr Lys Gln Leu Leu Tyr Glu Thr Tyr Leu Ala
           835                 840                 845
   Ser Leu Cys Gly Gly Ile Ser Asp Gly Ile Val Trp Tyr Leu Pro Ile
       850                 855                 860
   Thr His Pro Asn Lys Cys Ile Val Ala Ile Glu Val Ser Asp Glu Arg
   865                 870                 875                 880
   Val Pro Ala Ser Ile Arg Ala Gly Arg Ile Arg Leu Arg Phe Pro Leu
                   885                 890                 895
   Ser Ala Arg His Leu Lys Gly Val Val Ile Ile Gln Ile Asp Glu Glu
               900                 905                 910
   Gly Glu Phe Thr Val Tyr Ser Glu Gly Ile Val Ser His Arg Val Cys
           915                 920                 925
   Lys Lys Asn Leu Leu Lys Tyr Met Cys Asp Ile Ile Leu Leu Lys Phe
       930                 935                 940
   Ser Gly His Val Phe Gly Asn Asp Glu Met Leu Thr Lys Leu Leu Asn
   945 950 955 960
   Val
<210> 6
   <211> 962
   <212> PRT
   <213> Artificial sequence
<220>
   <223> BTV-2 VP2 consensus sequence
<220>
   <221> misc_feature
   <222> (156)..(156)
   <223> Xaa can be any naturally occurring amino acid
<220>
   <221> misc_feature
   <222> (180)..(180)
   <223> Xaa can be any naturally occurring amino acid
<220>
   <221> misc_feature
   <222> (399)..(399)
   <223> Xaa can be any naturally occurring amino acid
<220>
   <221> misc_feature
   <222> (508)..(508)
   <223> Xaa can be any naturally occurring amino acid
<220>
   <221> misc_feature
   <222> (567)..(567)
   <223> Xaa can be any naturally occurring amino acid
<220>
   <221> misc_feature
   <222> (921)..(921)
   <223> Xaa can be any naturally occurring amino acid
<400> 6
   Met Asp Glu Leu Gly Ile Pro Ile Tyr Gly Arg Asn Tyr Pro Glu His
   1               5                   10                  15
   Leu Leu Lys Gly Tyr Glu Phe Leu Ile Asn Thr Gly Val Lys Tyr Pro
               20                  25                  30
   Ser Gln Gly Gly Arg His Asp Val Ser Lys Ile Pro Glu Met Phe Ala
           35                  40                  45
   Tyr Asp Ile Lys Asp Glu Gly Ile Arg Glu Ala Leu Lys Tyr Met Pro
       50                  55                  60
   Thr Arg Asn Asp Gly Val Val Leu Pro Arg Ile Val Asp Ile Ser Leu
   65                  70                  75                  80
   Lys Gly Tyr Asp Ile Arg Lys Ser Val Ile Glu Ala Asn Lys Ser Asn
                   85                  90                  95
   Ser Phe His Thr Asp Thr Asn Trp Val Gln Trp Met Ile Lys Asp Ser
               100                 105                 110
   Met Asp Gln Gln Pro Leu Lys Ile Ser Ile Asp Glu Glu His Ser Arg
           115                 120                 125
   Val Val His Ser Leu Phe Asn Cys Gln Val Lys Ile Asp Ala Lys Lys
       130                 135                 140
   Ala Asp Thr Leu Ser Tyr His Leu Glu Ala Ile Xaa Asp Ala Glu Lys
   145                 150                 155                 160
   Ala Cys Leu His Thr Arg Gly Gln Leu Cys Asn His Leu Ala Arg Met
                   165                 170                 175
   Asp Leu Leu Xaa Ala Ala Gln Glu Ile Ala Tyr Ala Ile Lys Pro Thr
               180                 185                 190
   Tyr Gln Leu Ile Val His Ser Glu Arg Ala Ser Thr Ser Asp Asn Phe
           195                 200                 205
   Glu Leu Gly Arg Gln Asp Val Ile Thr Leu Arg Arg Gly His Arg Val
       210                 215                 220
   Gln Met Gly Asp Glu Ala Tyr Thr Lys Leu Met Glu Arg Leu Val Arg
   225                 230                 235                 240
   Leu Thr Val Gln Gly Asn Val Pro Arg Lys Ile Gln Ser Glu Ile Glu
                   245                 250                 255
   Gln Leu Glu Ala Ile Arg Thr Arg Trp Ala Thr Gly Arg Tyr Asp Pro
               260                 265                 270
   Ala His Ile Asn Ser Gln Asp Leu Cys Arg Ile Leu Ser Arg Ile Gly
           275                 280                 285
   Arg Ile Met Leu Asp Gln Glu Ala Glu Pro Val Asp Glu Asp Ser Leu
       290                 295                 300
   Ser Leu Arg Phe Gln Arg Ala Leu Asp Glu Lys Phe Arg Leu Asn Asp
   305 310 315 320
   Ser Glu Arg Asn Lys Ile Phe Glu Pro Lys Ser His Arg Lys Asp Glu
   325 330 335
   Asp Arg Phe Tyr Val Leu Leu Ala Ile Ala Ala Ser Asp Thr Tyr Asn
   340 345 350
   Ser Arg Ile Trp Trp Ser Asn Pro Tyr Pro Cys Leu Arg Gly Thr Leu
   355 360 365
   Ile Ala Ala Glu Thr Lys Leu Gly Asp Val Tyr Phe Thr Leu Arg Ser
   370 375 380
   Trp Tyr Asp Trp Ser Val Arg Ser Ser Tyr Val Pro Arg Glu Xaa Glu
   385 390 395 400
   Arg Glu Thr Glu Lys Tyr Ile Phe Ser Lys Ile Asn Leu Phe Asp Tyr
   405 410 415
   Glu Ala Gly Pro Ser Ser Lys Val Ile His Trp Glu Tyr Gln Leu Tyr
   420 425 430
   Lys Arg Glu Arg Val Val Thr Leu Glu Arg Gly Asn Pro Cys Asp Leu
   435 440 445
   Tyr Pro Asp Glu Asp Asp Glu Val Ile Ile Thr Lys Phe Asp Asp Ala
   450 455 460
   Lys Tyr Ser Glu Met Val Gly Glu Ile Ile Asp Gly Gly Trp Asn Asp
   465 470 475 480
   Glu Glu Phe Lys Met Tyr Lys Leu Leu Gln Glu Lys Gly Asn Val Leu
   485 490 495
   Thr Ile Asp Phe Glu Lys Asp Thr Lys Leu Asn Xaa Thr Ser Glu Val
   500 505 510
   Val Leu Pro Asp Tyr Tyr Gly Lys Trp Ile Val Ala Pro Met Phe Asn
   515 520 525
   Ser Lys Met Arg Ile Ile Glu Thr Glu Ile Ala Thr Asn Lys Ser Asp
   530 535 540
   Asp Pro Met Ile Lys Arg Thr Leu Lys Pro Met Thr Asp Asp Pro Val
   545 550 555 560
   Glu Leu Gln Arg Tyr Thr Xaa Ala Arg Tyr Tyr Asp Ile Arg Pro Gly
                   565                 570                 575
   Leu Met Gly Arg Ser Leu Asn Arg Thr Gln Thr Gln Ser Thr Phe Asp
               580                 585                 590
   Ala Lys Val Ser Glu Leu Ser Asp Tyr Glu Lys Val Val Ser Arg Phe
           595                 600                 605
   Gly Val Ile Lys Lys Pro Thr Arg Pro Cys Val Thr Leu Thr Gly Arg
       610                 615                 620
   Tyr Ile Leu Glu Lys Tyr Ser Leu Leu Leu Ile Asp Ile Leu Lys Tyr
   625                 630                 635                 640
   His Thr Glu Val Glu Glu Asn Pro Gln Glu Glu Phe Thr His Pro Arg
                   645                 650                 655
   Ile Asp Ser Gln Phe Lys Phe Asn Gly Asn Thr Leu Ser Asp Leu Asn
               660                 665                 670
   Gln Thr Val Val Phe Ile Val Asp Tyr Leu His Glu Lys Arg Asn Tyr
           675                 680                 685
   Val Arg Ser Val Tyr Glu Ala Arg Tyr Ile Ile Ser Arg Ile Arg Ser
       690                 695                 700
   Ser Thr Gly Ala Ala Arg Met Ser Ile Leu Glu Phe Tyr Phe Pro Thr
   705                 710                 715                 720
   Phe Ala Arg Leu Ile Ser Asn Ala Arg Glu Pro Thr Tyr Val Lys Asp
                   725                 730                 735
   Leu Met Ala Leu Asn Phe Leu Pro Leu Leu Phe Ile Val Gly Asp Asn
               740                 745                 750
   Met Ile Tyr Lys His Arg Gln Trp Ser Ile Pro Leu Leu Leu Tyr Thr
           755                 760                 765
   Asp Arg Val Lys Val Ile Pro Leu Glu Val Gly Ser Ser Asn Asn Arg
       770                 775                 780
   Gln Gly Leu Val Ser Tyr Leu Glu Tyr Met Phe Phe Phe Pro Ser Leu
   785                 790                 795                 800
   Ala Asp Arg Thr Ser Lys Val Asp Glu Ser Met Ile Lys Val Ser Lys
                   805                 810                 815
   Glu Met Val Asn Tyr Tyr Met Lys Thr Thr Ile Ser Glu Gly Gly Val
               820                 825                 830
   Asn Leu Asn Val Val Ser Thr Lys Ser Leu Leu Tyr Asp Ile Tyr Leu
           835                 840                 845
   Ser Ser Val Cys Gly Gly Val Ser Asp Gly Val Val Trp Tyr Leu Pro
       850                 855                 860
   Ile Thr His Pro Tyr Lys Cys Val Val Ala Ile Glu Val Cys Asp Asp
   865                 870                 875                 880
   Arg Val Pro Ala Arg Leu Arg Cys Asp Arg Leu Arg Leu Arg Phe Pro
                   885                 890                 895
   Leu Ser Ala Gln His Leu Lys Gly Ile Val Val Ile Gln Ile Asn Glu
               900                 905                 910
   Glu Gly Gly Phe Asp Val Tyr Thr Xaa Gly Ile Val Thr His Arg Val
           915                 920                 925
   Cys Lys Lys Ser Leu Leu Lys His Val Cys Asp Ile Val Leu Leu Lys
       930                 935                 940
   Phe His Gly His Val Phe Gly Asn Asp Glu Met Leu Thr Lys Leu Leu
   945                 950                 955                 960
   Asn Val
<210> 7
   <211> 959
   <212> PRT
   <213> Artificial sequence
<220>
   <223> BTV-3 VP2 consensus sequence
<220>
   <221> misc_feature
   <222> (34)..(34)
   <223> Xaa can be any naturally occurring amino acid
<220>
   <221> misc_feature
   <222> (56)..(56)
   <223> Xaa can be any naturally occurring amino acid
<220>
   <221> misc_feature
   <222> (117)..(117)
   <223> Xaa can be any naturally occurring amino acid
<220>
   <221> misc_feature
   <222> (156)..(156)
   <223> Xaa can be any naturally occurring amino acid
<220>
   <221> misc_feature
   <222> (205)..(205)
   <223> Xaa can be any naturally occurring amino acid
<220>
   <221> misc_feature
   <222> (249)..(249)
   <223> Xaa can be any naturally occurring amino acid
<220>
   <221> misc_feature
   <222> (255)..(255)
   <223> Xaa can be any naturally occurring amino acid
<220>
   <221> misc_feature
   <222> (302)..(302)
   <223> Xaa can be any naturally occurring amino acid
<220>
   <221> misc_feature
   <222> (383)..(383)
   <223> Xaa can be any naturally occurring amino acid
<220>
   <221> misc_feature
   <222> (404)..(404)
   <223> Xaa can be any naturally occurring amino acid
<220>
   <221> misc_feature
   <222> (417)..(417)
   <223> Xaa can be any naturally occurring amino acid
<220>
   <221> misc_feature
   <222> (421)..(422)
   <223> Xaa can be any naturally occurring amino acid
<220>
   <221> misc_feature
   <222> (437)..(437)
   <223> Xaa can be any naturally occurring amino acid
<220>
   <221> misc_feature
   <222> (455)..(455)
   <223> Xaa can be any naturally occurring amino acid
<220>
   <221> misc_feature
   <222> (477)..(477)
   <223> Xaa can be any naturally occurring amino acid
<220>
   <221> misc_feature
   <222> (488)..(488)
   <223> Xaa can be any naturally occurring amino acid
<220>
   <221> misc_feature
   <222> (490)..(490)
   <223> Xaa can be any naturally occurring amino acid
<220>
   <221> misc_feature
   <222> (553)..(553)
   <223> Xaa can be any naturally occurring amino acid
<220>
   <221> misc_feature
   <222> (585)..(585)
   <223> Xaa can be any naturally occurring amino acid
<220>
   <221> misc_feature
   <222> (588)..(588)
   <223> Xaa can be any naturally occurring amino acid
<220>
   <221> misc_feature
   <222> (597)..(597)
   <223> Xaa can be any naturally occurring amino acid
<220>
   <221> misc_feature
   <222> (601)..(601)
   <223> Xaa can be any naturally occurring amino acid
<220>
   <221> misc_feature
   <222> (608)..(608)
   <223> Xaa can be any naturally occurring amino acid
<220>
   <221> misc_feature
   <222> (633)..(633)
   <223> Xaa can be any naturally occurring amino acid
<220>
   <221> misc_feature
   <222> (647)..(647)
   <223> Xaa can be any naturally occurring amino acid
<220>
   <221> misc_feature
   <222> (663)..(663)
   <223> Xaa can be any naturally occurring amino acid
<220>
   <221> misc_feature
   <222> (727)..(727)
   <223> Xaa can be any naturally occurring amino acid
<220>
   <221> misc_feature
   <222> (750)..(750)
   <223> Xaa can be any naturally occurring amino acid
<220>
   <221> misc_feature
   <222> (761)..(761)
   <223> Xaa can be any naturally occurring amino acid
<220>
   <221> misc_feature
   <222> (763)..(763)
   <223> Xaa can be any naturally occurring amino acid
<220>
   <221> misc_feature
   <222> (771)..(771)
   <223> Xaa can be any naturally occurring amino acid
<220>
   <221> misc_feature
   <222> (799)..(799)
   <223> Xaa can be any naturally occurring amino acid
<220>
   <221> misc_feature
   <222> (803)..(803)
   <223> Xaa can be any naturally occurring amino acid
<220>
   <221> misc_feature
   <222> (809)..(810)
   <223> Xaa can be any naturally occurring amino acid
<220>
   <221> misc_feature
   <222> (816)..(816)
   <223> Xaa can be any naturally occurring amino acid
<220>
   <221> misc_feature
   <222> (821)..(821)
   <223> Xaa can be any naturally occurring amino acid
<220>
   <221> misc_feature
   <222> (824)..(824)
   <223> Xaa can be any naturally occurring amino acid
<220>
   <221> misc_feature
   <222> (872)..(872)
   <223> Xaa can be any naturally occurring amino acid
<220>
   <221> misc_feature
   <222> (896)..(896)
   <223> Xaa can be any naturally occurring amino acid
<220>
   <221> misc_feature
   <222> (914)..(914)
   <223> Xaa can be any naturally occurring amino acid
<220>
   <221> misc_feature
   <222> (930)..(930)
   <223> Xaa can be any naturally occurring amino acid
<220>
   <221> misc_feature
   <222> (940)..(940)
   <223> Xaa can be any naturally occurring amino acid
<400> 7
   Met Glu Glu Leu Val Ile Pro Val Ile Ser Arg Gln Phe Asp Lys Lys
   1               5                   10                  15
   Leu Val Gly Arg Tyr Asp Tyr Val Ile Glu Leu Ala Glu Pro Glu Gln
               20                  25                  30
   Asp Xaa Trp Thr Asn His Asp Val Thr Gln Ile Pro Asp Arg Arg Met
           35                  40                  45
   Phe Asp Val Ala Gln Gln Gly Xaa Arg Glu Ala Ile Ile Tyr Lys Pro
       50                  55                  60
   Leu Asp Asn Asp Gly Glu Val Leu Pro Arg Ile Leu Asp Met Ser Ile
   65                  70                  75                  80
   Ala Cys Tyr Asp Met Arg Lys Thr Met Met Lys Lys Glu Gly Val Asp
                   85                  90                  95
   Phe Val Ser Asn Thr Lys Trp Leu Glu Trp Met Ile Gln Asp Ser Met
               100                 105                 110
   Asp Val Gln Pro Xaa Arg Val Gln Met Lys Glu Asp His Ser Thr Val
           115                 120                 125
   Gln Tyr Asp Met Phe Ser Ala Lys Val His Ile Asp Ser Arg Lys Ala
       130                 135                 140
   Asp Thr Thr Ser Tyr His Ala Val Ala Val Glu Xaa Lys Ala Glu Arg
   145                 150                 155                 160
   Lys Cys Cys His Val His Thr Asp Val Trp Asn Ser Met Val Arg Asn
                   165                 170                 175
   His Leu Phe Asn Thr Ala Gln Glu Ser Cys Tyr Thr Phe Lys Gln Thr
               180                 185                 190
   Tyr Glu Leu Ile Val Asn Ser Glu Arg Leu Ser Pro Xaa Glu Glu Phe
           195                 200                 205
   Arg Val Gly Ala Pro Gln Phe His Thr Ile Gln Arg Asn His Arg Met
       210                 215                 220
   Arg Leu Gly Asp Asn Ala Tyr Asp Lys Phe Leu Lys Gly Leu Val Gln
   225                 230                 235                 240
   Leu Arg Val Ser Gly Thr Thr Pro Xaa Lys Ile Arg Asp Glu Xaa Ala
                   245                 250                 255
   Ala Leu Asp Val Ile Arg Asp Asn Trp Ile Arg Gly Ser Phe Asp Arg
               260                 265                 270
   Ser His Ile Lys Ser Leu Glu Leu Cys Arg Leu Leu Ser Ser Ile Gly
           275                 280                 285
   Arg Lys Met Val Asn Met Glu Glu Glu Pro Lys Asp Glu Xaa Asp Leu
       290                 295                 300
   Ser Val Lys Phe Gln Phe Lys Leu Asp Glu Lys Phe Ser Pro Asn Asp
   305                 310                 315                 320
   Ser Glu Arg Asn Val Ile Phe Thr Ser Lys Thr His Arg Thr Asn Glu
                   325                 330                 335
   Asp Arg Phe Tyr Val Leu Leu Val Ile Ala Ala Ser Asp Thr Asn Asn
               340                 345                 350
   Gly Arg Val Trp Trp Ser Asn Pro Tyr Pro Cys Leu Arg Gly Ala Leu
           355                 360                 365
   Ile Ala Ala Glu Cys Lys Leu Gly Asp Val Tyr His Thr Leu Xaa Ser
       370                 375                 380
   Lys Tyr Glu Trp Ser Val Arg Pro Thr Tyr Lys Pro Arg Asp Leu Glu
   385                 390                 395                 400
   Arg Glu Arg Xaa Lys Tyr Val Val Gly Arg Val Asn Leu Phe Asp Leu
                   405                 410                 415
   Xaa Gly Glu Pro Xaa Xaa Lys Val Ile His Trp Glu Tyr Glu Leu Ile
               420                 425                 430
   Ser Pro Met Tyr Xaa Val Ser Asn His Lys Gly Asn His Cys Asp Leu
           435                 440                 445
   Tyr Pro Asp Asp Val Glu Xaa Thr Thr Lys Phe Asn Glu Asp Arg Tyr
       450                 455                 460
   Arg Glu Met Ile Gln Ser Val Ile Asp Asp Gly Trp Xaa Gln Lys Asn
   465                 470                 475                 480
   Phe Lys Met Tyr Lys Ile Leu Xaa Glu Xaa Gly Asn Pro Leu Leu Tyr
                   485                 490                 495
   Asp Leu Glu Lys Asp Ile Lys Leu Asp Ser Arg Ser Gln Val Val Phe
               500                 505                 510
   Pro Ser Tyr Tyr Asn Lys Trp Thr Tyr Ala Pro Met Phe Asn Ala Arg
           515                 520                 525
   Val Lys Pro Cys Asp Val Glu Leu Ala Glu Arg Lys Asn Asp Asp Pro
       530                 535                 540
   Phe Val Lys Arg Thr Leu Lys Pro Xaa Lys Ala Asp Cys Val Asp Leu
   545                 550                 555                 560
   Leu Arg Tyr His Met Ser His Tyr Tyr Asp Leu Arg Pro Ser Leu Lys
                   565                 570                 575
   Gly Val Ser Leu Ser Asn Lys Gln Xaa Pro Ser Xaa Ile His Gln Ala
               580                 585                 590
   Leu Ala Arg Asp Xaa Leu Tyr Ser Xaa Leu Leu Lys Arg Arg Asp Xaa
           595                 600                 605
   Asp Leu Asp Tyr Ser Ser Pro Cys Pro Ile Val Thr Asn Tyr Phe Leu
       610                 615                 620
   Leu Glu Lys Phe His Ile Leu Ile Xaa Thr Ile Met Glu Lys His Tyr
   625                 630                 635                 640
   Trp Glu Leu Asp Asp Ser Xaa Asp Val Tyr Glu Phe Pro Lys Ile Asp
                   645                 650                 655
   Ala Ser Ala Phe Glu Val Xaa Gly Thr Leu Tyr Asp Val Ser Gln Thr
               660                 665                 670
   Ile Val His Met Tyr Asp Arg Phe Phe Glu Lys Arg Arg Val Leu Arg
           675                 680                 685
   Ser Ile Asp Glu Ser Arg Trp Ile Leu His Leu Ile Arg Ile Ser Gln
       690                 695                 700
   Gly Arg Glu Arg Leu Glu Val Ile Glu Arg Phe Phe Pro Asn Tyr Gly
   705                 710                 715                 720
   Lys Ala Met Arg Gln Arg Xaa Phe Lys Lys Val Arg Asp Ile Met Phe
                   725                 730                 735
   Leu Asn Phe Leu Pro Phe Phe Phe Leu Thr Gly Asp Asn Xaa Ser Tyr
               740                 745                 750
   Glu His Arg Gln Trp Ser Ile Pro Xaa Ile Xaa Tyr Ala Asp Lys Leu
           755                 760                 765
   Arg Ile Xaa Pro Val Glu Val Gly Ala Tyr Tyr Asn Arg Phe Gly Val
       770                 775                 780
   Ala Cys Ile Leu Glu Leu Leu Asn Phe Phe Pro Ser Tyr Glu Xaa Arg
   785                 790                 795                 800
   Glu Glu Xaa Leu Glu Glu Asp Ile Xaa Xaa Cys Ala Asp Ala Ile Xaa
                   805                 810                 815
   Asn Phe Tyr Leu Xaa Thr Thr Xaa Ser Asn Gly Gly Val Gln Thr Ser
               820                 825                 830
   Ile Val Ser Thr Lys Ala Leu Leu Tyr Glu Met Tyr Leu Ser Ser Ile
           835                 840                 845
   Cys Gly Gly Tyr Ser Glu Gly Val Leu Trp Tyr Leu Pro Ile Thr His
       850                 855                 860
   Pro Val Lys Cys Leu Val Ala Xaa Glu Val Ser Asp Ala Leu Val Gly
   865                 870                 875                 880
   Ala Asp Val Arg Ile Asp Lys Ile Lys Arg Arg Phe Pro Leu Ser Xaa
                   885                 890                 895
   Lys His Leu Lys Gly Ile Val Gln Ile Ser Ile His Pro Asn Arg Thr
               900                 905                 910
   Phe Xaa Val Met Thr Cys Gly Ile Val Arg His Lys Val Cys Lys Lys
           915                 920                 925
   Thr Xaa Leu Lys His Arg Cys Asp Val Ile Leu Xaa Gln Thr Pro Gly
       930                 935                 940
   Tyr Val Phe Gly Asn Asp Glu Leu Leu Thr Lys Leu Leu Asn Ile
   945                 950                 955
<210> 8
   <211> 952
   <212> PRT
   <213> Artificial sequence
<220>
   <223> BTV-4 VP2 consensus sequence
<220>
   <221> misc_feature
   <222> (155)..(155)
   <223> Xaa can be any naturally occurring amino acid
<220>
   <221> misc_feature
   <222> (179)..(179)
   <223> Xaa can be any naturally occurring amino acid
<220>
   <221> misc_feature
   <222> (398)..(398)
   <223> Xaa can be any naturally occurring amino acid
<220>
   <221> misc_feature
   <222> (507)..(507)
   <223> Xaa can be any naturally occurring amino acid
<220>
   <221> misc_feature
   <222> (566)..(566)
   <223> Xaa can be any naturally occurring amino acid
<220>
   <221> misc_feature
   <222> (920)..(920)
   <223> Xaa can be any naturally occurring amino acid
<400> 8
   Met Lys Leu Gly Ile Pro Ile Tyr Gly Arg Asn Tyr Pro Glu His Leu
   1               5                   10                  15
   Leu Lys Gly Tyr Glu Phe Leu Ile Asn Thr Gly Val Lys Tyr Pro Ser
               20                  25                  30
   Gln Gly Gly Arg His Asp Val Ser Lys Ile Pro Glu Met Phe Ala Tyr
           35                  40                  45
   Asp Ile Lys Asp Glu Gly Ile Arg Glu Ala Leu Lys Tyr Met Pro Thr
       50                  55                  60
   Arg Asn Asp Gly Val Val Leu Pro Arg Ile Val Asp Ile Ser Leu Lys
   65                  70                  75                  80
   Gly Tyr Asp Ile Arg Lys Ser Val Ile Glu Ala Asn Lys Ser Asn Ser
                   85                  90                  95
   Phe His Thr Asp Thr Asn Trp Val Gln Trp Met Ile Lys Asp Ser Met
               100                 105                 110
   Asp Gln Gln Pro Leu Lys Ile Ser Ile Asp Glu Glu His Ser Arg Val
           115                 120                 125
   Val His Ser Leu Phe Asn Cys Gln Val Lys Ile Asp Ala Lys Lys Ala
       130                 135                 140
   Asp Thr Leu Ser Tyr His Leu Glu Ala Ile Xaa Asp Ala Glu Lys Ala
   145                 150                 155                 160
   Cys Leu His Thr Arg Gly Gln Leu Cys Asn His Leu Ala Arg Met Asp
                   165                 170                 175
   Leu Leu Xaa Ala Ala Gln Glu Ile Ala Tyr Ala Ile Lys Pro Thr Tyr
               180                 185                 190
   Gln Leu Ile Val His Ser Glu Arg Ala Ser Thr Ser Asp Asn Phe Glu
           195                 200                 205
   Leu Gly Arg Gln Asp Val Ile Thr Leu Arg Arg Gly His Arg Val Gln
       210                 215                 220
   Met Gly Asp Glu Ala Tyr Thr Lys Leu Met Glu Arg Leu Val Arg Leu
   225                 230                 235                 240
   Thr Val Gln Gly Asn Val Pro Arg Lys Ile Gln Ser Glu Ile Glu Gln
                   245                 250                 255
   Leu Glu Ala Ile Arg Thr Arg Trp Ala Thr Gly Arg Tyr Asp Pro Ala
               260                 265                 270
   His Ile Asn Ser Gln Asp Leu Cys Arg Ile Leu Ser Arg Ile Gly Arg
           275                 280                 285
   Ile Met Leu Asp Gln Glu Ala Glu Pro Val Asp Glu Asp Ser Leu Ser
       290                 295                 300
   Leu Arg Phe Gln Arg Ala Leu Asp Glu Lys Phe Arg Leu Asn Asp Ser
   305                 310                 315                 320
   Glu Arg Asn Lys Ile Phe Glu Pro Lys Ser His Arg Lys Asp Glu Asp
                   325                 330                 335
   Arg Phe Tyr Val Leu Leu Ala Ile Ala Ala Ser Asp Thr Tyr Asn Ser
               340                 345                 350
   Arg Ile Trp Trp Ser Asn Pro Tyr Pro Cys Leu Arg Gly Thr Leu Ile
           355                 360                 365
   Ala Ala Glu Thr Lys Leu Gly Asp Val Tyr Phe Thr Leu Arg Ser Trp
       370                 375                 380
   Tyr Asp Trp Ser Val Arg Ser Ser Tyr Val Pro Arg Glu Xaa Glu Arg
   385                 390                 395                 400
   Glu Thr Glu Lys Tyr Ile Phe Ser Lys Ile Asn Leu Phe Asp Tyr Glu
                   405                 410                 415
   Ala Gly Pro Ser Ser Lys Val Ile His Trp Glu Tyr Gln Leu Tyr Lys
               420                 425                 430
   Arg Glu Arg Val Val Thr Leu Glu Arg Gly Asn Pro Cys Asp Leu Tyr
           435                 440                 445
   Pro Asp Glu Asp Asp Glu Val Ile Ile Thr Lys Phe Asp Asp Ala Lys
       450                 455                 460
   Tyr Ser Glu Met Val Gly Glu Ile Ile Asp Gly Gly Trp Asn Asp Glu
   465                 470                 475                 480
   Glu Phe Lys Met Tyr Lys Leu Leu Gln Glu Lys Gly Asn Val Leu Thr
                   485                 490                 495
   Ile Asp Phe Glu Lys Asp Thr Lys Leu Asn Xaa Thr Ser Glu Val Val
               500                 505                 510
   Leu Pro Asp Tyr Tyr Gly Lys Trp Ile Val Ala Pro Met Phe Asn Ser
           515                 520                 525
   Lys Met Arg Ile Ile Glu Thr Glu Ile Ala Thr Asn Lys Ser Asp Asp
       530                 535                 540
   Pro Met Ile Lys Arg Thr Leu Lys Pro Met Thr Asp Asp Pro Val Glu
   545                 550                 555                 560
   Leu Gln Arg Tyr Thr Xaa Ala Arg Tyr Tyr Asp Ile Arg Pro Gly Leu
                   565                 570                 575
   Met Gly Arg Ser Leu Asn Arg Thr Gln Thr Gln Ser Thr Phe Asp Ala
               580                 585                 590
   Lys Val Ser Glu Leu Ser Asp Tyr Glu Lys Val Val Ser Arg Phe Gly
           595                 600                 605
   Val Ile Lys Lys Pro Thr Arg Pro Cys Val Thr Leu Thr Gly Arg Tyr
       610                 615                 620
   Ile Leu Glu Lys Tyr Ser Leu Leu Leu Ile Asp Ile Leu Lys Tyr His
   625                 630                 635                 640
   Thr Glu Val Glu Glu Asn Pro Gln Glu Glu Phe Thr His Pro Arg Ile
                   645                 650                 655
   Asp Ser Gln Phe Lys Phe Asn Gly Asn Thr Leu Ser Asp Leu Asn Gln
               660                 665                 670
   Thr Val Val Phe Ile Val Asp Tyr Leu His Glu Lys Arg Asn Tyr Val
           675                 680                 685
   Arg Ser Val Tyr Glu Ala Arg Tyr Ile Ile Ser Arg Ile Arg Ser Ser
       690                 695                 700
   Thr Gly Ala Ala Arg Met Ser Ile Leu Glu Phe Tyr Phe Pro Thr Phe
   705                 710                 715                 720
   Ala Arg Leu Ile Ser Asn Ala Arg Glu Pro Thr Tyr Val Lys Asp Leu
                   725                 730                 735
   Met Ala Leu Asn Phe Leu Pro Leu Leu Phe Ile Val Gly Asp Asn Met
               740                 745                 750
   Ile Tyr Lys His Arg Gln Trp Ser Ile Pro Leu Leu Leu Tyr Thr Asp
           755                 760                 765
   Arg Val Lys Val Ile Pro Leu Glu Val Gly Ser Ser Asn Asn Arg Gln
       770                 775                 780
   Gly Leu Val Ser Tyr Leu Glu Tyr Met Phe Phe Phe Pro Ser Leu Ala
   785                 790                 795                 800
   Asp Arg Thr Ser Lys Val Asp Glu Ser Met Ile Lys Val Ser Lys Glu
                   805                 810                 815
   Met Val Asn Tyr Tyr Met Lys Thr Thr Ile Ser Glu Gly Gly Val Asn
               820                 825                 830
   Leu Asn Val Val Ser Thr Lys Ser Leu Leu Tyr Asp Ile Tyr Leu Ser
           835                 840                 845
   Ser Val Cys Gly Gly Val Ser Asp Gly Val Val Trp Tyr Leu Pro Ile
   850 855 860
   Thr His Pro Tyr Lys Cys Val Val Ala Ile Glu Val Cys Asp Asp Arg
   865 870 875 880
   Val Pro Ala Arg Leu Arg Cys Asp Arg Leu Arg Leu Arg Phe Pro Leu
   885 890 895
   Ser Ala Gln His Leu Lys Gly Ile Val Val Ile Gln Ile Asn Glu Glu
   900 905 910
   Gly Gly Phe Asp Val Tyr Thr Xaa Gly Ile Val Thr His Arg Val Cys
   915 920 925
   Lys Lys Ser Leu Leu Lys His Val Cys Asp Ile Val Leu Leu Lys Phe
   930 935 940
   His Gly His Val Phe Gly Asn Asp
   945 950
<210> 9
   <211> 955
   <212> PRT
   <213> Artificial sequence
<220>
   <223> BTV-5 VP2 consensus sequence
<400> 9
   Met Asp Glu Phe Gly Val Pro Ile Phe Arg Ser Ser Gln Val Pro His
   1               5                   10                  15
   His Ile Leu Glu Gly Tyr Asp Ile Lys Val Asp Leu Ser Asp Arg Val
               20                  25                  30
   Val Gln Glu Asp Gly Arg His Asp Pro Asn Lys Leu Lys Glu Arg Lys
           35                  40                  45
   Thr Ile Asp Val Pro Asn Gly Glu Ala Arg Ser Asp Leu Asn Tyr Asn
       50                  55                  60
   Pro Thr Asp Asn Glu Gly Asn Gln Leu Pro Arg Ala Leu Asp Ile Ala
   65                  70                  75                  80
   Leu Cys Ala Arg Leu Thr Arg Lys Ser Phe Asn Thr Asn Ser Gly Val
                   85                  90                  95
   Thr Phe His Thr Asp His Lys Trp Met Glu Trp Met Leu Ser Asp Ala
               100                 105                 110
   Met Asp Val Gln Pro Leu Arg Ile Asp Leu Ala Ala Gln Asn Glu Tyr
           115                 120                 125
   Ala Lys Cys Asp Leu Phe Asn Ser Ser Val Tyr Val Arg Lys Lys Tyr
       130                 135                 140
   Val Asp Ala Ile Ser Tyr Arg Tyr Ile Ser Val Glu Asp Asp Ser Lys
   145                 150                 155                 160
   Gly Cys Asn His Thr Arg Val Tyr Ser Val Asn His Leu Ile Asn Cys
                   165                 170                 175
   Gly Leu Tyr Asn Val Ala Gln Glu Cys Ala Tyr Ala Phe Lys Asp Thr
               180                 185                 190
   Asn Thr Leu Ile Val Asn Ser Gln Arg Glu Thr Thr Ser Glu Pro Phe
           195                 200                 205
   Gln Pro Gly Asn Pro Lys Ile Gly Ser Leu Gly Arg Arg Ala Arg Val
       210                 215                 220
   Asp Met Ala Asp Pro Gly Tyr Pro Leu Phe Arg Ala Gly Leu Leu Gln
   225                 230                 235                 240
   Ile Ile Val Asn Gly Ala Ile Pro Ala Asp Ile Arg Ser Glu Met Asp
                   245                 250                 255
   Arg Leu Asn Gln Ile Arg Glu Ala Trp Lys Arg Asp Lys Asn Thr Arg
               260                 265                 270
   Glu Val Arg Ala Leu Asp Leu Cys Val Leu Leu Ser Lys Ile Gly Arg
           275                 280                 285
   Ile Lys Met Asn Met Glu Glu Glu Pro Lys Asp Glu Gly Val Met Ser
       290                 295                 300
   Leu Arg Phe Gln Gly Lys Ile Asp Ala Met Phe Phe Ser Glu Asn Thr
   305                 310                 315                 320
   Glu Lys Thr Asn Ile Met Arg Asp Gly Ser Gly Arg Thr Asp Asp Glu
                   325                 330                 335
   Arg Phe Tyr Ala Leu Leu Leu Ile Cys Ala Thr Asp Ala Phe Ser Arg
               340                 345                 350
   Arg Ile Trp Arg Thr Asn Pro Tyr Pro Cys Leu Arg Gly Thr Leu Ile
   355 360 365
   Ala Ala Glu Cys Val Leu Gly Asp Pro Tyr Lys Thr Leu Arg Arg Lys
   370 375 380
   Phe Asp Trp Ser Val Arg His Ala Gly Asp Lys Met Leu Glu Gly Ser
   385 390 395 400
   Ser Tyr Val Phe Thr Arg Ile Asn Leu Phe Asp Thr Asp Lys Ala Pro
   405 410 415
   Gly Ser Arg Val Ile His Trp Ile Gln Glu Thr Leu Pro Val Ile Lys
   420 425 430
   Thr Thr Trp Ala Glu Gly Cys Ser Leu Ala Asp Glu Ala Ser Asp Asp
   435 440 445
   His Leu His Cys Lys Ile Asp His Ala Gln Tyr Arg Asp Leu Val Ser
   450 455 460
   Arg Val Ile Asn Gly Gly Trp Asp Gln Glu Asn Phe Lys Ile His Lys
   465 470 475 480
   Leu Phe Val Glu Arg Gly Asn Ile Phe Leu Met Asp Phe Glu Lys Asp
   485 490 495
   Ala Lys Ile Thr Thr Gln Ser Glu Val Glu Tyr Pro Tyr Tyr Tyr Asp
   500 505 510
   Thr Trp Ile Tyr Ala Pro Ile Phe Ser Thr Arg Tyr Lys Ile Thr Glu
   515 520 525
   Thr Glu Ile Ala Asn Ala Lys Ser Leu Asp Pro Ala Ile Lys Arg Thr
   530 535 540
   Leu Ala Pro Leu Ser Asp Asp Pro Val Ala Leu Gln Leu Arg Ala Val
   545 550 555 560
   Gly His Leu Tyr Asp Thr Arg Pro Ala Ile Met Gly Gln Thr Leu Ser
   565 570 575
   Pro Arg Gln Asn Gln Thr Ala Leu His Lys Thr Leu Leu Asp Asp Pro
   580 585 590
   Glu Asn Asp Arg Tyr Tyr Glu Ala Arg Ser Ile Lys Lys Val Asp Asn
   595 600 605
   Pro Cys Ser Val His Tyr Thr Ser Ser Phe Ile Leu Gln Lys Ile Ser
       610                 615                 620
   Glu Leu Val Leu Ser Leu Met Ile Tyr His Ile Glu His Asp Trp Glu
   625                 630                 635                 640
   Pro Leu Lys Gln Tyr Thr His Pro Glu Ile Ser Thr Glu Phe Gly Glu
                   645                 650                 655
   Ile Ser Cys Glu Ile Cys Asp Ile Ser Gln Leu Thr Val Leu Leu Ile
               660                 665                 670
   Asp Leu Phe Phe Glu Asn Arg Arg Thr Val Arg Pro Ser Glu Glu Thr
           675                 680                 685
   Arg Tyr Ile Ile His Gln Ile Arg Glu Ser Arg Gly Asp Gly Arg Leu
       690                 695                 700
   Lys Met Leu Glu Arg Phe Phe Pro Ala Tyr Gly Gln Ala Leu Arg Lys
   705                 710                 715                 720
   Ile Glu Ser Ala Thr Glu Val Gly His Ile Thr Met Leu Asn Phe Leu
                   725                 730                 735
   Pro Phe Phe Phe Leu Leu Gly Asp Asn Ile Ile Tyr Lys His Arg Glu
               740                 745                 750
   Trp Thr Val Pro Ile Leu Ile Tyr Thr Asp Gly Val Met Ile Trp Pro
           755                 760                 765
   Ala Gln Val Gly Ala Asn Tyr Asn Arg Phe Gly Phe Cys Gly Phe Leu
       770                 775                 780
   Asn Tyr Met Arg Phe His Ala Gly Ser Gln Leu Arg Ser Lys Val Leu
   785                 790                 795                 800
   Asp Asp Ala Glu Lys Thr Ile Ala Lys Glu Met Leu Lys Tyr Tyr Met
                   805                 810                 815
   Lys Thr Asp Ile Phe Ser Gly Gly Val Gln Lys Asn Ile Arg Val Thr
               820                 825                 830
   Lys Ser Met Gln Met Glu Ile Tyr His Ser Ser Leu Cys Gly Gly Leu
           835                 840                 845
   Ser Asp Ala Val Val Tyr Ala Leu Pro Ile Ser Phe Pro Ile Lys Cys
       850                 855                 860
   Leu Thr Leu Ile Ile Ile Gly Asp Asp Leu Val Glu Pro Gln Leu Arg
   865 870 875 880
   Ile Ser Arg Val Met Asp Tyr Phe Lys His Val Ser Glu His Val Arg
   885 890 895
   Gly Val Ala Ser Ile Ser Ile Ser Arg Asn Gly Asp Val Ser Thr Tyr
   900 905 910
   Ser Arg Gly Ile Val His Val Glu Leu Leu Lys Lys Asn Ile Leu Lys
   915 920 925
   Tyr Gln Phe Gln Val Ala Leu Leu Lys Val Lys Gly Tyr Val Phe Gly
   930 935 940
   Asn Asp Glu Met Leu Ile Lys Leu Leu Asn Val
   945 950 955
<210> 10
   <211> 955
   <212> PRT
   <213> Artificial sequence
<220>
   <223> BTV-6 VP2 consensus sequence
<220>
   <221> misc_feature
   <222> (20)..(20)
   <223> Xaa can be any naturally occurring amino acid
<220>
   <221> misc_feature
   <222> (84)..(84)
   <223> Xaa can be any naturally occurring amino acid
<220>
   <221> misc_feature
   <222> (123)..(123)
   <223> Xaa can be any naturally occurring amino acid
<220>
   <221> misc_feature
   <222> (201)..(201)
   <223> Xaa can be any naturally occurring amino acid
<220>
   <221> misc_feature
   <222> (292)..(292)
   <223> Xaa can be any naturally occurring amino acid
<220>
   <221> misc_feature
   <222> (296)..(296)
   <223> Xaa can be any naturally occurring amino acid
<220>
   <221> misc_feature
   <222> (303)..(303)
   <223> Xaa can be any naturally occurring amino acid
<220>
   <221> misc_feature
   <222> (335)..(335)
   <223> Xaa can be any naturally occurring amino acid
<220>
   <221> misc_feature
   <222> (364)..(364)
   <223> Xaa can be any naturally occurring amino acid
<220>
   <221> misc_feature
   <222> (379)..(379)
   <223> Xaa can be any naturally occurring amino acid
<220>
   <221> misc_feature
   <222> (473)..(473)
   <223> Xaa can be any naturally occurring amino acid
<220>
   <221> misc_feature
   <222> (480)..(480)
   <223> Xaa can be any naturally occurring amino acid
<220>
   <221> misc_feature
   <222> (514)..(514)
   <223> Xaa can be any naturally occurring amino acid
<220>
   <221> misc_feature
   <222> (552)..(552)
   <223> Xaa can be any naturally occurring amino acid
<220>
   <221> misc_feature
   <222> (560)..(560)
   <223> Xaa can be any naturally occurring amino acid
<220>
   <221> misc_feature
   <222> (586)..(586)
   <223> Xaa can be any naturally occurring amino acid
<220>
   <221> misc_feature
   <222> (706)..(706)
   <223> Xaa can be any naturally occurring amino acid
<220>
   <221> misc_feature
   <222> (841)..(841)
   <223> Xaa can be any naturally occurring amino acid
<220>
   <221> misc_feature
   <222> (947)..(947)
   <223> Xaa can be any naturally occurring amino acid
<400> 10
   Met Asp Glu Leu Ala Ile Pro Ile Ile Asn Gly His Phe Pro Lys Glu
   1               5                   10                  15
   Ala Leu Ile Xaa Tyr Asp Cys Ile Val Glu Leu Asn Ala Pro Ser Glu
               20                  25                  30
   Asp Asp Glu Val Asp Val Thr Gln Ile Pro Thr Lys Asn Met Met Asp
           35                  40                  45
   Ile Pro Asp Val Pro Ile Arg Glu Ala Leu Arg Val Leu Pro Gln Arg
       50                  55                  60
   Asn Asp Gly His Val Leu Thr Arg Ile Leu Asp Ile Ser Leu Ser Gly
   65                  70                  75                  80
   Tyr Asp Arg Xaa Lys Tyr Tyr Lys Thr Asn Lys Gly Gly Glu Phe Glu
                   85                  90                  95
   Ser Pro Lys Glu Trp Leu Asn Trp Met Ile His Asp Ser Met Asp Val
               100                 105                 110
   Gln Pro Leu Lys Val Ser Leu Glu Met Arg Xaa Thr Val Lys His Glu
           115                 120                 125
   Leu Phe Ser Ser Asn Val Tyr Leu Ser Val Lys Lys Ala Asp Thr Ser
       130                 135                 140
   Ser Tyr His Ile Glu Ala Ile Gly Asp Thr Gly Lys Ala Cys Asp His
   145                 150                 155                 160
   Ser Tyr Val Ser Met Trp Asn Asn Leu Val Arg His Glu Val Met His
                   165                 170                 175
   Cys Ala Gln Glu Ser Ala Tyr Val Leu Lys Pro Thr Met Glu Ile Ile
               180                 185                 190
   Val His Ser Glu Arg Ala Ser Thr Xaa Val Pro Phe Gln Leu Val Gly
           195                 200                 205
   Gln Asn Tyr Val Ser Ile Gly Arg Asn His Arg Leu Val Leu Gly Asp
       210                 215                 220
   Glu Pro Tyr Lys Lys Thr Leu Gln Gly Leu Thr Lys Leu Arg Val Gln
   225                 230                 235                 240
   Gly Lys Val Pro Pro Gln Ile Gln Asp Glu Ile Gln Gln Leu Asn Arg
                   245                 250                 255
   Ile Arg Asp Ala Trp Ile Gly Asp Ala Tyr Asn Pro Arg His Ile Arg
               260                 265                 270
   Ser Leu Glu Leu Cys Arg Ile Leu Ser Ser Val Gly Arg Lys Val Val
           275                 280                 285
   Cys Ile Glu Xaa Glu Pro Lys Xaa Glu Ser Asp Ile Ser Val Xaa Phe
       290                 295                 300
   Gln Phe Lys Leu Asp Glu Lys Phe Arg Leu Asn Asp Gly Glu Arg Gly
   305                 310                 315                 320
   Val Ile Phe Thr Ser Lys Asn Gln Arg Asn Asp Gln Asp Arg Xaa Phe
                   325                 330                 335
   Val Leu Ile Met Ile Ala Ala Ser Asp Thr Tyr Asn Gly Arg Ile Trp
               340                 345                 350
   Trp Thr Asn Pro Tyr Pro Cys Leu Arg Gly Ala Xaa Ile Ala Ser Glu
           355                 360                 365
   Thr Arg Leu Gly Asp Val Tyr Trp Thr Leu Xaa His Thr Tyr Asp Trp
       370                 375                 380
   Ser Val Arg Met Thr Tyr Ser Pro Arg Met Lys Glu Arg Glu Asp Asp
   385                 390                 395                 400
   Lys Tyr Ile Tyr Gly Arg Val Asn Leu Phe Asp Leu Asp Ala Ser Pro
                   405                 410                 415
   Gly Asp Lys Val Val His Trp Arg Tyr Glu Leu Ile Ser Gln Thr Lys
               420                 425                 430
   Lys Thr Thr Phe His Asp Gly Asn Pro Cys Asp Leu Phe Pro Asp Val
           435                 440                 445
   Glu Gly Ile Val Thr Lys Phe Asn Lys Thr Ala Tyr Ser Asn Met Ile
       450                 455                 460
   Asn Glu Leu Leu Asn Gly Gly Trp Xaa Thr Arg Glu Phe Lys Met Xaa
   465                 470                 475                 480
   Lys Leu Leu Glu Asp Glu Gly Asn Val Leu Thr Ile Asp Phe Glu Lys
   485 490 495
   Asp Thr Lys Leu Asn Ser Arg Ser Glu Phe Ile Leu Pro Asn Tyr Tyr
   500 505 510
   Asp Xaa Trp Ile Tyr Ala Pro Met Phe Asn Ala Lys Leu Lys Ile Thr
   515 520 525
   Glu Thr Glu Ile Ala Gln Ala Lys Asn Asp Asp Pro Tyr Val Ile Arg
   530 535 540
   Thr Leu Ala Pro Ile Gly Ser Xaa Pro Phe Thr Leu Gln Arg Leu Xaa
   545 550 555 560
   Leu Gly Asn Tyr Tyr Asp Val Arg Pro Ala Met His Gly Arg Ala Leu
   565 570 575
   Ser Lys Lys Gln Glu Gln Ser Ala Tyr Xaa Ala Ala Leu Val Lys Glu
   580 585 590
   Lys Asp Tyr Glu Asp Ile Leu Thr Arg Arg Gly Leu Arg Lys Ala Asp
   595 600 605
   Lys Pro Cys Pro Leu Thr Thr Cys Ala Tyr Ile Leu Glu Lys Thr Ala
   610 615 620
   Leu Leu Trp Ile Asp Val Met Glu Lys His Ile Ser Glu Val Thr Asp
   625 630 635 640
   Asp Ser Asn Glu Phe Asp Phe Pro Lys Ala Cys Val Asp Ser Pro Val
   645 650 655
   Gly Thr Asp Arg Ile Val Asp Val Thr Gln Leu Ile Val Leu Leu Ile
   660 665 670
   Asp Trp Ile Tyr Glu Arg Arg Lys Asp Val Arg Ser Val Asp Glu Ser
   675 680 685
   Arg Trp Val Ile Glu Thr Ile Arg Arg Ala Arg Gly Arg Asn Arg Leu
   690 695 700
   Glu Xaa Ile Ser Arg Glu Phe Pro Asn Phe Gly Arg Ala Val Ala Arg
   705 710 715 720
   Leu Gln Ser Pro Asn Thr Val Gly Asp Val Met Val Ile Asn Phe Leu
   725 730 735
   Pro Phe Leu Phe Leu Met Gly Asp Asn Val Ser Tyr Glu His Arg Gln
               740                 745                 750
   Trp Ser Val Pro Val Ile Leu His Ala Asp Val Met Trp Phe Ile Pro
           755                 760                 765
   Val Glu Val Gly Ala Phe Tyr Asn Arg Phe Gly Val Thr Ser Phe Leu
       770                 775                 780
   Glu Tyr Leu Met Phe Phe Pro Ser Phe His Met Arg Thr Val Arg Leu
   785                 790                 795                 800
   Asp Glu Ala Glu Val Lys Val Ser Lys Leu Ile Leu Asp Phe Tyr Leu
                   805                 810                 815
   Asp Thr Thr Ile Phe Ser Gly Gly Ile Gln Ser Ser Val Val Ser Thr
               820                 825                 830
   Lys Gln Ile Leu Tyr Glu Thr Tyr Xaa Ser Ser Leu Cys Gly Gly Phe
           835                 840                 845
   Ser Glu Gly Leu Val Trp Tyr Leu Pro Ile Thr His Pro Thr Lys Cys
       850                 855                 860
   Val Val Ala Ile Glu Phe Ala Asp Asn Lys Val Ser Ala Val Tyr Arg
   865                 870                 875                 880
   Cys Asn Arg Leu Arg Ala Arg Phe Pro Leu Ser Ser Ile His Leu Arg
                   885                 890                 895
   Gly Ile Ala Ile Leu Thr Ile Gly Lys Asn Arg Met Val Asp Ala Tyr
               900                 905                 910
   Thr Glu Gly Ile Val Ser His Arg Ile Cys Lys Lys Asn Val Leu Gly
           915                 920                 925
   Tyr Thr Cys Gln Ile Leu Leu Leu Lys Phe Ser Gly His Val Phe Gly
       930                 935                 940
   Asn Asp Xaa Met Leu Thr Lys Leu Leu Asn Val
   945                 950                 955
<210> 11
   <211> 959
   <212> PRT
   <213> Artificial sequence
<220>
   <223> BTV-7 VP2 consensus sequence
<400> 11
   Met Asp Glu Phe Ala Ile Pro Ile Val His Gly Arg Val Ile Pro Tyr
   1               5                   10                  15
   Asp Leu Val Arg Ser Tyr Asp Ile Val Val Asp Leu Asp Val Arg Ile
               20                  25                  30
   Asp Glu Glu Gly Asp Lys His Asp Thr Thr Lys Ile Pro Glu Ala Tyr
           35                  40                  45
   Met Cys Asp Val Pro Asn Ile Ala Met Gln Asp Ala Asn Ser Ala Ile
       50                  55                  60
   Leu Gly Arg Asn Asp Gly Tyr Ala Leu Pro Arg Ile Leu Asp Val Ser
   65                  70                  75                  80
   Ile Ala Ala Tyr Asp Thr Arg Lys Gly Tyr Trp Lys Tyr Arg Gly Ile
                   85                  90                  95
   Glu Lys Met Thr Asn Gln Thr Trp Leu Lys Trp Ala Ile Lys Asp Phe
               100                 105                 110
   Met Asp Val Gln Pro Leu Lys Val Asp Leu Ala Asp Leu Glu Gly Lys
           115                 120                 125
   Val Arg Tyr Asp Met Phe Asn Cys Ser Ala Arg Ile Asp Ala Lys Lys
       130                 135                 140
   Ala Glu Ala Ile Tyr Tyr Gly Trp Ala Ala Gln Glu Val Val Asn Lys
   145                 150                 155                 160
   Gly Cys Asn His Ala Lys Ser Glu Met Phe Gln His Val Val Arg Cys
                   165                 170                 175
   Glu Leu Leu Asn Cys Ser Gln Glu Ser Ala Tyr Ile Leu Lys Pro Thr
               180                 185                 190
   Gln Lys Ile Glu Pro Leu Ala Glu Lys Asn Asp Leu Gly Glu Arg Leu
           195                 200                 205
   Gly Pro Gly Ser Asn Asn Leu Ile Asn Met Arg Arg Gly Asp Thr Phe
       210                 215                 220
   Arg Met Gly Thr Asp Gln Tyr Asn Arg Met Leu Gln Gly Trp Tyr Arg
   225                 230                 235                 240
   Val Lys Val Lys Gly Gln Ile Pro Asp Met Val Arg Glu Glu Met Ala
                   245                 250                 255
   Glu Leu Val Lys Ile Arg Asp Arg Trp Lys Val Ala Lys Asn Pro Arg
               260                 265                 270
   Glu Ile Lys Ser Leu Glu Leu Cys Lys Leu Leu Ser Thr Ile Gly Arg
           275                 280                 285
   Arg Met Tyr Asn Met Tyr Glu Glu Pro Lys Asp Glu Glu Ala Val Ser
       290                 295                 300
   Leu Arg Phe Gln Trp Asn Leu Asp Glu Ile Phe Lys Thr Asp Asn Arg
   305                 310                 315                 320
   Glu His Val Asn Ile Ala Val Asn Lys Asn Gly Arg Thr Asp Asp Glu
                   325                 330                 335
   Arg Phe Phe Ala Leu Ile Met Ile Ala Ala Thr Asp Thr Asn Arg Gly
               340                 345                 350
   Arg Ile Trp Trp Ser Asn Met Phe Pro Cys Leu Arg Gly Ala Leu Ile
           355                 360                 365
   Ala Ser Glu Thr Gln Phe Gly Asp Val Tyr Lys Met Leu Arg Val Arg
       370                 375                 380
   Phe Glu Trp Ser Val Arg Asn Glu Tyr Ser Pro Gln Ala Asp Val Ala
   385                 390                 395                 400
   Arg Gln Asn Glu Asn Tyr Val Tyr Ala Arg Val Asn Leu Phe Asp Leu
                   405                 410                 415
   Val Ala Asp Pro Ala Thr Ser Val Ile His Trp Glu Tyr Lys Leu Asp
               420                 425                 430
   Lys Pro Phe Lys Thr Cys Tyr Ser Asp Gly His Gln Cys Asp Glu Tyr
           435                 440                 445
   Pro Asp Glu Tyr Asp Leu Ile Cys Ser Phe Asp Glu Ala Lys Tyr Ser
       450                 455                 460
   Glu Met Val Gln Arg Ile Ile Glu Gly Gly Trp Asp His Lys Asn Phe
   465                 470                 475                 480
   Lys His Tyr Lys Ile Leu Lys Glu Ala Ser Asn Val Leu Thr Ile Asp
                   485                 490                 495
   Phe Glu Lys Asp Ala Lys Leu Asn Glu Ser Ser Glu Met Asn Leu Pro
               500                 505                 510
   Tyr Tyr Phe Asn Lys Asp Ile Ala Ala Pro Met Phe Arg Cys Val Leu
           515                 520                 525
   Lys Val Cys Glu Thr Ser Ile Ser Glu Arg His Asn Leu Asp Pro Ala
       530                 535                 540
   Ile Ser Arg Thr Met Lys Pro Met Arg Ala Lys Ala Ala Asp Leu Leu
   545                 550                 555                 560
   Asp Tyr Ser Leu Gly Ser Tyr Tyr Glu Thr Arg Ser Gly Val Met Gly
                   565                 570                 575
   Arg Thr Leu Thr Thr Thr Gln Lys Gln Ser Asp Met Tyr Lys Met Val
               580                 585                 590
   Gln Asp Glu Asp Asp Tyr Leu Ser Leu Met Val Gly Arg Gly Val Thr
           595                 600                 605
   Asp Lys Phe Ala Asn Pro Cys Pro Ile Thr Thr Ala Cys Gln Tyr Ala
       610                 615                 620
   Leu Gln Lys Thr Ser Val Met Ile Phe Ser Arg Val Glu His Tyr Phe
   625                 630                 635                 640
   Glu Glu Tyr Leu Lys Asp Asp Lys Val Glu Tyr Leu Ser Pro Ser Leu
                   645                 650                 655
   Asn Leu Glu Trp Val Glu Ile Thr Ser Arg Ile Thr Asp Ile Ser Gln
               660                 665                 670
   Leu Ile Val Leu Thr Phe Asp Val Phe Phe Glu Gly Arg Arg Gln Val
           675                 680                 685
   Arg Thr Ile Asp Glu Ser Arg Phe Ile Leu Tyr Gln Ile Arg Ala Arg
       690                 695                 700
   Lys Gly Met Asp Arg Leu Ser Tyr Ile Lys Asp Leu Phe Pro Gln Leu
   705                 710                 715                 720
   Tyr Gly Trp Trp Val Ser Pro Glu Pro Thr Tyr Gly His Ala Gln Lys
                   725                 730                 735
   Leu Asn Leu Leu Pro Leu Leu Phe Ile Ile Gly Asp Asn Phe Ile Tyr
               740                 745                 750
   Glu His Arg Gln Trp Ser Tyr Pro Val Ile Leu Val Ser His Lys Leu
   755 760 765
   Arg Phe Val Pro Val Glu Leu Gly Ala Tyr Tyr Asn Arg Phe Gly Leu
   770 775 780
   Thr Ser Leu Phe Ser Tyr Leu Val Phe His Val Gly Lys Ser Lys Tyr
   785 790 795 800
   Gly Leu Glu Leu Asp Glu Ala Glu Lys Lys Val Leu Gln Ala Ala Lys
   805 810 815
   Asp Tyr Tyr Ala Val Ala Thr Ile Ser Glu Gly Gly Ile Asn Thr His
   820 825 830
   Ser Thr Met Thr Lys Asp Pro Ile Tyr Gln Val Tyr Leu Ser Thr Ile
   835 840 845
   Cys Gly Gly Leu Leu Asp Ser Leu Ile Tyr Leu Met Pro Ile Thr His
   850 855 860
   Pro Val Lys Ser Ile Ile Met Val Ile Ile Asn Asp Gly Arg Gly Ser
   865 870 875 880
   Ala Ser Asp Cys Val Arg Arg Ala Thr Arg Arg Leu Arg Tyr Ile Gln
   885 890 895
   Arg His Ile Arg Gly Ser Val Val Val Ser Val Leu Arg Asp Gly Val
   900 905 910
   Ile Asn Thr Tyr Ser Glu Gly Ile Ala Lys Ala Met Leu Cys Glu Lys
   915 920 925
   Ser Val Leu Lys Tyr Asn Tyr Lys Val Val Leu Val Lys Val Lys Gly
   930 935 940
   Tyr Val Phe Gly His Asp Glu Met Leu Thr Lys Leu Leu Asn Val
   945 950 955
<210> 12
   <211> 961
   <212> PRT
   <213> Artificial sequence
<220>
   <223> BTV-8 VP2 consensus sequence
<400> 12
   Met Glu Glu Leu Ala Ile Pro Ile Tyr Thr Asn Val Phe Pro Ala Glu
   1               5                   10                  15
   Leu Leu Asp Gly Tyr Asp Tyr Ile Ile Asp Val Ser Ser Arg Val Glu
               20                  25                  30
   Glu Glu Gly Asp Glu Pro Val Lys Arg His Asp Val Thr Glu Ile Pro
           35                  40                  45
   Arg Asn Ser Met Phe Asp Ile Lys Asp Glu His Ile Arg Asp Ala Ile
       50                  55                  60
   Ile Tyr Lys Pro Val Asn Asn Asp Gly Tyr Val Leu Pro Arg Val Leu
   65                  70                  75                  80
   Asp Ile Thr Leu Lys Ala Phe Asp Asp Arg Lys Arg Val Val Leu Asn
                   85                  90                  95
   Asp Gly His Ser Glu Phe His Thr Lys Thr Asn Trp Val Gln Trp Met
               100                 105                 110
   Ile Asp Asp Ala Met Asp Val Gln Pro Leu Lys Val Asp Ile Ala His
           115                 120                 125
   Thr Arg Ser Arg Ile Ser His Ala Leu Phe Asn Cys Thr Val Arg Leu
       130                 135                 140
   His Ser Lys Lys Ala Asp Thr Ala Ser Tyr His Val Glu Pro Val Glu
   145                 150                 155                 160
   Ile Glu Ser Trp Gly Cys Asn His Thr Trp Leu Ser Arg Ile His His
                   165                 170                 175
   Leu Val Asn Val Glu Leu Phe His Cys Ser Gln Glu Ala Ala Tyr Thr
               180                 185                 190
   Leu Lys Pro Thr Tyr Lys Ile Ile Ser Asn Ala Glu Arg Ala Ser Thr
           195                 200                 205
   Ser Asp Ser Phe Asn Gly Thr Met Ile Glu Leu Gly Arg Asn His Gln
       210                 215                 220
   Ile Gln Met Gly Asp Gln Gly Tyr Gln Lys Leu Lys Glu Gly Leu Val
   225                 230                 235                 240
   Gln Val Arg Ile Glu Gly Lys Thr Pro Leu Val Ile Gln Glu Glu Ile
                   245                 250                 255
   Thr Ala Leu Asn Lys Ile Arg Glu Gln Trp Ile Ala Arg Asn Phe Asp
               260                 265                 270
   Gln Arg Glu Ile Lys Val Leu Asp Leu Cys Arg Leu Leu Ser Thr Ile
           275                 280                 285
   Gly Arg Lys Met Cys Asn Thr Glu Glu Glu Pro Lys Asn Glu Ala Asp
       290                 295                 300
   Leu Ser Val Lys Phe Gln Met Glu Leu Asp Glu Ile Phe Arg Pro Gly
   305                 310                 315                 320
   Asn Asn Glu Arg Thr Asn Ile Met Gly Gly Gly Val His Arg Lys Asn
                   325                 330                 335
   Glu Asp Arg Phe Tyr Val Leu Ile Met Ile Ala Ala Ser Asp Thr Asn
               340                 345                 350
   Lys Gly Arg Ile Trp Trp Ser Asn Pro Tyr Pro Cys Leu Arg Gly Ala
           355                 360                 365
   Leu Ile Ala Ala Glu Val Gln Leu Gly Asp Val Tyr Asn Leu Leu Arg
       370                 375                 380
   Asn Trp Phe Gln Trp Ser Val Arg Pro Thr Tyr Val Pro Tyr Asp Arg
   385                 390                 395                 400
   Asn Arg Glu Ser Asp Lys Tyr Ile Tyr Ser Arg Ile Asn Leu Phe Asp
                   405                 410                 415
   Ser Thr Leu Arg Pro Gly Asp Lys Ile Val His Trp Glu Tyr Lys Leu
               420                 425                 430
   Leu Asn Glu Val Arg Glu Val Ser Ile Asn Lys Gly Asn Glu Cys Asp
           435                 440                 445
   Leu Phe Pro Glu Asp Glu Glu Phe Thr Thr Lys Phe His Glu Ala Arg
       450                 455                 460
   Tyr Thr Glu Met Lys Asn Gln Ile Ile Gln Ser Gly Trp Asn Gln Arg
   465                 470                 475                 480
   Asp Phe Lys Met His Lys Ile Leu Glu Asp Gly Ala Asn Val Leu Thr
                   485                 490                 495
   Ile Asp Phe Glu Lys Asp Ala His Ile Gly Thr Gly Ser Ala Leu Ser
               500                 505                 510
   Leu Pro Asp Tyr Tyr Asn Lys Trp Ile Ile Ala Pro Met Phe Asn Ala
           515                 520                 525
   Lys Leu Arg Ile Thr Glu Val Val Ile Gly Thr Ala His Thr Asp Asp
       530                 535                 540
   Pro Ala Val Gly Arg Ser Ala Lys Ala Phe Thr His Asp Pro Phe Asp
   545                 550                 555                 560
   Leu Gln Arg Tyr Cys Leu Ala Arg Tyr Tyr Asp Val Arg Pro Gly Met
                   565                 570                 575
   Met Gly Arg Ala Leu Ser Lys Gln Gln Asn Met Ser Ser Met Thr Asp
               580                 585                 590
   Lys Leu Ser Lys Gln Glu Asp Tyr Ala Gly Ile Val Ser Arg Arg Leu
           595                 600                 605
   Glu Tyr Lys Glu Arg Glu Asn Arg Cys Leu Thr Glu Thr Ala Gln Tyr
       610                 615                 620
   Val Phe Glu Lys Thr Cys Leu Tyr Val Leu Glu Leu Leu Ser Arg His
   625                 630                 635                 640
   Thr Met Pro Ser Glu Asp Ser Glu Val Thr Phe Glu His Pro Thr Ile
                   645                 650                 655
   Asp Pro Ser Val Asp Ile Glu Thr Trp Lys Ile Ile Asp Val Ser Gln
               660                 665                 670
   Leu Ile Ile Phe Val Phe Asp Tyr Leu Phe Glu Asn Arg Lys Ile Val
           675                 680                 685
   Arg Asp Thr Thr Glu Ala Arg Trp Thr Leu Phe Lys Ile Arg Ser Glu
       690                 695                 700
   Val Gly Arg Ala Arg Ile Asp Ala Ile Glu Met Thr Phe Pro Arg Phe
   705                 710                 715                 720
   Gly Arg Met Leu Arg Asn Ala Ser Gln Ala Lys Ile Asn Gln Asp Ile
                   725                 730                 735
   Ala Cys Leu Asn Phe Leu Pro Leu Leu Phe Ile Ile Gly Asp Asn Ile
               740                 745                 750
   Ser Tyr Ala His Arg Gln Trp Ser Ile Pro Val Leu Leu Tyr Ala His
   755 760 765
   Asp Ile Arg Ile Ile Pro Leu Glu Val Gly Ala Tyr Asn Asn Arg Phe
   770 775 780
   Gly Leu Thr Ser Tyr Leu Glu Tyr Met Ala Phe Phe Pro Ser Tyr Ala
   785 790 795 800
   Thr Arg Val Ala Lys Ile Asp Glu Ser Ile Lys Glu Cys Ala Ile Ala
   805 810 815
   Met Ala Glu Phe Tyr Met Asn Thr Asp Ile His Ser Gly Ser Val Met
   820 825 830
   Ser Asn Val Ile Thr Thr Lys Arg Leu Leu Tyr Glu Thr Tyr Leu Ala
   835 840 845
   Ser Leu Cys Gly Gly Tyr Ser Asp Gly Leu Leu Trp Tyr Leu Pro Ile
   850 855 860
   Thr His Pro Ser Lys Cys Leu Val Ala Phe Glu Val Ala Asp Asp Val
   865 870 875 880
   Val Pro Leu Ser Val Arg Arg Glu Arg Ile Leu Ser Arg Phe Pro Leu
   885 890 895
   Ser Ser Arg His Val Lys Gly Ile Ala Leu Ile Ser Val Asp Arg Asn
   900 905 910
   Gln Lys Val Ser Val Gln Thr Glu Gly Ile Val Thr His Arg Leu Cys
   915 920 925
   Lys Lys Asn Leu Leu Lys Tyr Val Cys Asp Val Ile Leu Phe Lys Phe
   930 935 940
   Ser Gly His Val Phe Gly Asn Asp Glu Met Leu Thr Lys Leu Leu Asn
   945 950 955 960
   Val
<210> 13
   <211> 955
   <212> PRT
   <213> Artificial sequence
<220>
   <223> BTV-9 VP2 consensus sequence
<220>
   <221> misc_feature
   <222> (425)..(425)
   <223> Xaa can be any naturally occurring amino acid
<220>
   <221> misc_feature
   <222> (604)..(604)
   <223> Xaa can be any naturally occurring amino acid
<220>
   <221> misc_feature
   <222> (758)..(758)
   <223> Xaa can be any naturally occurring amino acid
<400> 13
   Met Asp Glu Phe Gly Ile Pro Ile Tyr Leu Ser Ser Ser Val Pro His
   1               5                   10                  15
   Asp Leu Leu Ser Asp Tyr Asp Phe Met Ile Asp Leu Ser Asp Lys Val
               20                  25                  30
   Val His Ala Gly Asp Arg His Asp Pro Lys Lys Leu Lys Glu Arg Lys
           35                  40                  45
   Ala Ile Asp Val Pro Asp Gly Glu Val Arg Thr Asp Leu Asn Tyr Asn
       50                  55                  60
   Pro Thr Asp Asn Glu Gly Asn Gln Leu Pro Arg Ala Leu Asp Ile Ala
   65                  70                  75                  80
   Leu Ser Ala Arg Leu Thr Arg Lys Ser Val Arg Asn Asn Asp Gly Leu
                   85                  90                  95
   Asp Phe Leu Thr Asp Asp Lys Trp Met Glu Trp Met Leu Ser Asp Ala
               100                 105                 110
   Met Asp Val Gln Pro Leu Lys Ile Asp Leu Ser Ala Arg Asn Gly Phe
           115                 120                 125
   Val Lys Cys Asp Ile Phe Asn Ser Ser Ile Tyr Ile Arg Arg Lys Tyr
       130                 135                 140
   Ala Asp Ala Ile Ala Tyr Arg Tyr Thr Ser Leu Glu Asp Glu Ser Lys
   145                 150                 155                 160
   Gly Cys Asn His Thr Arg Val Cys Met Val Asn His Leu Ile Asn Asn
                   165                 170                 175
   Gly Leu Tyr Asn Ala Val Gln Glu Cys Ala Tyr Ala Leu Lys Asp Thr
               180                 185                 190
   Tyr Val Leu Arg Val His Ser Gln Arg Glu Arg Ile Asp Glu Pro Phe
           195                 200                 205
   Glu Pro Gly Arg Pro Lys Val Gly Ser Leu Gly Arg Asn Ala Arg Ile
       210                 215                 220
   Asp Met Ser Asp Pro Gly Tyr Ser Leu Phe Lys Gly Gly Met Leu Gln
   225                 230                 235                 240
   Ile Thr Val Ser Gly Glu Ile Pro Asn Asp Ile Arg Val Glu Met Glu
                   245                 250                 255
   Arg Leu Asn Gln Ile Arg Ala Thr Trp Ile Arg Asp Lys Ser Ser Arg
               260                 265                 270
   Glu Val Arg Ala Ile Glu Leu Cys Thr Leu Leu Ser Arg Ile Gly Arg
           275                 280                 285
   Val Lys Trp Asn Ala Glu Glu Glu Pro Lys Asp Glu Gly Ala Met Ser
       290                 295                 300
   Leu Arg Phe Gln Asn Lys Ile Asp Ser Met Phe Phe Ser Glu Asn Thr
   305                 310                 315                 320
   Glu Lys Thr Asn Ile Met Gln Asn Thr Ser Gly Arg Thr Asp Glu Glu
                   325                 330                 335
   Arg Phe Tyr Ala Leu Leu Leu Ile Cys Ala Thr Asp Ala Tyr Ser Arg
               340                 345                 350
   Arg Ile Trp Arg Ser Asn Pro Tyr Pro Cys Leu Arg Gly Thr Leu Ile
           355                 360                 365
   Ala Ala Glu Cys Val Leu Gly Asp Pro Tyr Lys Thr Leu Arg Arg Lys
       370                 375                 380
   Phe Asn Trp Ser Val Arg His Ala Ala Asp Lys Ala Leu Glu Asn Asn
   385                 390                 395                 400
   Ala Tyr Ile Phe Thr Arg Ile Asn Leu Phe Asp Thr Glu Lys Thr Pro
                   405                 410                 415
   Gly Met Arg Val Val His Trp Thr Xaa Glu Leu Thr Met Glu Ser Gln
               420                 425                 430
   Thr Thr Trp Asp Glu Gly Tyr Pro Leu Lys Asp Glu Ala Pro Asp Asp
           435                 440                 445
   Glu Met His Cys Lys Ile Asp Thr Asn Lys Tyr Lys Glu Met Val Thr
       450                 455                 460
   Arg Val Ile Asp Gly Gly Trp Asp Gln Glu Asn Phe Lys Ile His Lys
   465                 470                 475                 480
   Leu Phe His Asp Asp Gly Asn Ile Phe Leu Met Asp Phe Glu Lys Asp
                   485                 490                 495
   Ala Lys Leu Thr Ala Gln Ser Glu Val Gln Tyr Pro Asp Tyr Tyr Asp
               500                 505                 510
   Lys Trp Ile Tyr Ala Pro Ile Phe Asp Thr Lys Tyr Lys Ile Thr Glu
           515                 520                 525
   Thr Glu Ile Ala Asn Ala Lys Asn Leu Asp Pro Ala Ile Lys Arg Thr
       530                 535                 540
   Met Asp Pro Met Met Asp Asp Pro Val Met Leu Gln Leu Ser Thr Ile
   545                 550                 555                 560
   Gly Asn Leu Tyr Asp Thr Arg Pro Ala Val Met Gly Ser Thr Leu Ser
                   565                 570                 575
   Met Arg Gln Arg Gln Thr Ser Leu His Lys Thr Leu Leu Asp Asp Pro
               580                 585                 590
   Gln Asn Glu Arg Phe Tyr Ser Ser Arg Ser Ile Xaa Lys Val Asp Asp
           595                 600                 605
   Pro Cys Pro Val Tyr Tyr Thr Ala Ser Leu Val Leu Gln Lys Ile Ser
       610                 615                 620
   Glu Leu Val Met Ser Leu Met Ile Tyr His Val Asp Gln Asp Trp Glu
   625                 630                 635                 640
   Ser Leu Lys Gln Tyr Lys His Pro Ser Ile Leu Ser Asp Phe Ser Ser
                   645                 650                 655
   Leu Val Ser Asn Ile Gln Asp Ile Ser Gln Leu Val Val Leu Leu Val
               660                 665                 670
   Asp Phe Phe Phe Glu Arg Arg Ser Val Leu Arg Ser Val Glu Glu Ser
           675                 680                 685
   Arg Tyr Ile Ile His Ile Ile Arg Ser Ser Asn Asp Ala Asn Arg Leu
       690                 695                 700
   Lys Ser Phe Glu Leu Tyr Phe Pro Arg Phe Gly Gly Ile Leu Arg Thr
   705                 710                 715                 720
   Ile Lys Asp Ala Lys Glu Val Ser His Val Thr Ala Leu Asn Phe Leu
                   725                 730                 735
   Pro Tyr Phe Phe Leu Leu Gly Asp Asn Ile Ile Tyr Lys His Lys Glu
               740                 745                 750
   Trp Ser Val Pro Val Xaa Ile Tyr Ser Asp Gly Leu Met Ile Trp Pro
           755                 760                 765
   Ala Gln Val Gly Ala Asn Phe Asn Arg Phe Gly Phe Phe Gly Phe Leu
       770                 775                 780
   Asn Tyr Met Arg Phe His Pro Gly Ile Arg Leu Arg Ser Lys Val Leu
   785                 790                 795                 800
   Asp Asp Ala Glu Lys Val Val Ala Val Glu Met Leu Asn Tyr Tyr Met
                   805                 810                 815
   Arg Thr Asp Ile Phe Lys Gly Gly Ile Gln Lys Asn Val Arg Val Thr
               820                 825                 830
   Lys Leu Ser Gln Met Glu Ile Tyr His Ser Ser Leu Cys Gly Gly Leu
           835                 840                 845
   Ser Asp Ala Leu Ile Tyr Ala Leu Pro Ile Ser Phe Pro Ile Lys Cys
       850                 855                 860
   Leu Thr Leu Ile Ile Val Gly Asp Asp Leu Ala Glu Pro Gln His Arg
   865                 870                 875                 880
   Ile Ser Lys Val Leu Glu Phe Phe Glu His Val Arg Asp His Val Arg
                   885                 890                 895
   Gly Ile Ala Ser Ile Ser Ile Ser Arg Asn Gly Asp Val Thr Thr His
               900                 905                 910
   Ser Gln Gly Ile Val Lys Val Glu Leu Leu Lys Lys Asn Ile Leu Arg
           915                 920                 925
   His Gln Phe Arg Val Ala Leu Leu Lys Val Lys Gly Tyr Val Phe Gly
   930 935 940
   Asn Asp Glu Met Leu Thr Lys Leu Leu Asn Val
   945 950 955
<210> 14
   <211> 956
   <212> PRT
   <213> Artificial sequence
<220>
   <223> BTV-10 VP2 consensus sequence
<220>
   <221> misc_feature
   <222> (579)..(579)
   <223> Xaa can be any naturally occurring amino acid
<400> 14
   Met Glu Glu Phe Val Ile Pro Val Phe Ser Glu Arg Asp Ile Pro Tyr
   1               5                   10                  15
   Ser Leu Leu Asn His Tyr Pro Leu Ala Ile Gln Ile Asp Val Lys Val
               20                  25                  30
   Asp Asp Glu Gly Gly Lys His Asn Leu Ile Lys Ile Pro Glu Ser Asp
           35                  40                  45
   Met Ile Asp Val Pro Arg Leu Ser Ile Ile Glu Ala Leu Asn Tyr Arg
       50                  55                  60
   Pro Lys Arg Asn Asp Gly Val Val Val Pro Arg Leu Leu Asp Ile Thr
   65                  70                  75                  80
   Leu Arg Ala Tyr Asp Asn Arg Lys Ser Ala Lys Asn Ala Lys Gly Val
                   85                  90                  95
   Glu Phe Met Thr Asp Thr Lys Trp Met Lys Trp Ala Ile Asp Asp Lys
               100                 105                 110
   Met Asp Ile Gln Pro Leu Lys Val Thr Leu Asp Asn His Cys Ser Val
           115                 120                 125
   Asn His Gln Leu Phe Asn Cys Ile Val Lys Ala Arg Ser Ala Asn Ala
       130                 135                 140
   Asp Thr Ile Tyr Tyr Asp Tyr Tyr Pro Leu Glu Asn Gly Ala Lys Arg
   145                 150                 155                 160
   Cys Asn His Thr Asn Leu Asp Leu Leu Arg Ser Leu Thr Thr Thr Glu
                   165                 170                 175
   Met Phe His Ile Leu Gln Gly Ala Ala Tyr Ala Leu Lys Thr Thr Tyr
               180                 185                 190
   Glu Leu Val Ala His Ser Glu Arg Glu Asn Met Ser Glu Ser Tyr Gln
           195                 200                 205
   Val Gly Thr Gln Arg Trp Ile Gln Leu Arg Lys Gly Thr Lys Ile Gly
       210                 215                 220
   Tyr Arg Gly Gln Pro Tyr Glu Arg Phe Ile Ser Ser Leu Val Gln Val
   225                 230                 235                 240
   Ile Ile Lys Gly Lys Ile Pro Asp Glu Ile Arg Thr Glu Ile Ala Glu
                   245                 250                 255
   Leu Asn Arg Ile Lys Asp Glu Trp Lys Asn Ala Ala Tyr Asp Arg Thr
               260                 265                 270
   Lys Ile Arg Ala Leu Glu Leu Cys Lys Ile Leu Ser Ala Ile Gly Arg
           275                 280                 285
   Lys Met Leu Asp Val Gln Glu Glu Pro Lys Asp Glu Met Ala Leu Ser
       290                 295                 300
   Thr Arg Phe Gln Phe Lys Leu Asp Glu Lys Phe Ile Arg Thr Asp Gln
   305                 310                 315                 320
   Glu His Val Asn Ile Phe Lys Val Gly Gly Ser Ala Thr Asp Asp Gly
                   325                 330                 335
   Arg Phe Tyr Ala Leu Ile Ala Ile Ala Ala Thr Asp Thr Gln Gln Gly
               340                 345                 350
   Arg Val Trp Arg Thr Asn Pro Tyr Pro Cys Leu Arg Gly Ala Leu Ile
           355                 360                 365
   Ala Ala Glu Cys Glu Leu Gly Asp Val Tyr Phe Thr Leu Arg Gln Thr
       370                 375                 380
   Tyr Lys Trp Ser Leu Arg Pro Glu Tyr Gly Gln Arg Glu Arg Pro Leu
   385                 390                 395                 400
   Glu Asp Asn Lys Tyr Val Phe Ala Arg Leu Asn Leu Phe Asp Thr Asn
                   405                 410                 415
   Leu Ala Val Gly Asp Glu Ile Ile His Trp Arg Tyr Glu Val Tyr Gln
               420                 425                 430
   Pro Lys Glu Thr Thr His Asp Asp Gly Tyr Ile Cys Val Ser Gln Lys
           435                 440                 445
   Gly Asp Asp Glu Leu Leu Cys Glu Val Asp Glu Asp Arg Tyr Lys Glu
       450                 455                 460
   Met Phe Asp Arg Met Ile Gln Gly Gly Trp Asp Gln Glu Arg Phe Lys
   465                 470                 475                 480
   Leu His Asn Ile Leu Thr Glu Pro Asn Leu Leu Thr Ile Asp Phe Glu
                   485                 490                 495
   Lys Asp Ala Tyr Leu Gly Ala Arg Ser Glu Leu Val Phe Pro Pro Tyr
               500                 505                 510
   Tyr Asp Lys Trp Ile Asn Ser Pro Met Phe Asn Ala Arg Leu Lys Ile
           515                 520                 525
   Ala Arg Gly Glu Ile Ala Thr Trp Lys Ala Asp Asp Pro Trp Ser Asn
       530                 535                 540
   Arg Ala Val His Gly Tyr Ile Lys Thr Ser Ala Glu Ser Leu Glu Tyr
   545                 550                 555                 560
   Ala Leu Gly Pro Tyr Tyr Asp Leu Arg Leu Gln Leu Phe Gly Asp Thr
                   565                 570                 575
   Leu Ser Xaa Gly Gln Arg Gln Ser Ala Val Phe Glu His Met Ala Gln
               580                 585                 590
   Gln Asp Asp Phe Ser Thr Leu Thr Asp Tyr Thr Lys Gly Arg Thr Val
           595                 600                 605
   Cys Pro His Ser Gly Gly Thr Phe Tyr Thr Phe Arg Lys Val Ala Leu
       610                 615                 620
   Ile Ile Leu Ser Asn Tyr Glu Arg Leu Asp Pro Ser Leu His Glu Gly
   625                 630                 635                 640
   Arg Glu His Glu Thr Tyr Met His Pro Ala Val Asn Asp Val Phe Arg
                   645                 650                 655
   Arg His Val Leu Glu Met Lys Asp Phe Ser Gln Leu Ile Cys Phe Val
               660                 665                 670
   Phe Asp Tyr Ile Phe Glu Lys His Val Gln Leu Arg Asn Ala Lys Glu
   675 680 685
   Ala Arg Arg Ile Ile Tyr Leu Ile Gln Asn Thr Ser Gly Ala Tyr Arg
   690 695 700
   Leu Asp Val Leu Arg Glu Ala Phe Pro Asn Phe Leu Lys His Val Met
   705 710 715 720
   Asn Leu Arg Asp Val Lys Arg Ile Cys Asp Leu Asn Val Ile Asn Phe
   725 730 735
   Phe Pro Leu Leu Phe Leu Val Gln Asp Asn Ile Ser Tyr Trp His Arg
   740 745 750
   Gln Trp Ser Ile Pro Met Ile Leu Phe Asp Gln Val Ile Arg Leu Ile
   755 760 765
   Pro Val Glu Val Gly Ala Tyr Ala Asn Arg Phe Gly Leu Lys Ser Phe
   770 775 780
   Phe Asn Phe Ile Arg Phe His Pro Gly Asp Ser Lys Lys Arg Gln Asp
   785 790 795 800
   Ala Asp Asp Thr His Lys Glu Phe Gly Ser Ile Cys Phe Glu Tyr Tyr
   805 810 815
   Thr Thr Thr Lys Ile Ser Gln Gly Glu Ile Asp Val Pro Val Val Thr
   820 825 830
   Ser Lys Leu Asp Thr Leu Lys Leu His Val Ala Ser Leu Cys Ala Gly
   835 840 845
   Leu Ala Asp Ser Leu Val Tyr Thr Leu Pro Val Ala His Pro Lys Lys
   850 855 860
   Ser Ile Val Leu Ile Ile Val Gly Asp Asp Lys Leu Glu Pro Gln Ile
   865 870 875 880
   Arg Ser Glu Gln Ile Val Asn Lys Tyr Tyr Tyr Ser Arg Arg His Ile
   885 890 895
   Ser Gly Val Val Ser Ile Cys Val Asn Gln Gly Gly Gln Leu Lys Val
   900 905 910
   His Ser Met Gly Ile Thr Arg His Arg Ile Cys Asp Lys Ser Ile Leu
   915 920 925
   Lys Tyr Lys Cys Lys Val Val Leu Val Arg Met Pro Gly His Val Phe
   930 935 940
   Gly Asn Asp Glu Leu Met Thr Lys Leu Leu Asn Val
   945 950 955
<210> 15
   <211> 956
   <212> PRT
   <213> Artificial sequence
<220>
   <223> BTV-11 VP2 consensus sequence
<400> 15
   Met Glu Glu Phe Val Ile Pro Val Tyr Ser Glu Thr Asp Ile Pro Tyr
   1               5                   10                  15
   Ser Leu Leu Ser His Tyr Pro Leu Ala Ile Arg Thr Asp Val Lys Ile
               20                  25                  30
   Ala Asp Thr Asp Glu Gly His Asp Val Val Lys Ile Pro Glu Ser Asp
           35                  40                  45
   Met Ile Asp Val Pro Arg Val Asp Ile Val Glu Ala Leu Ala Ala Lys
       50                  55                  60
   Pro Met Arg Asn Asp Gly Ile Val Val Pro Arg Leu Leu Asp Ile Thr
   65                  70                  75                  80
   Leu Arg Ala Tyr Asp Asp Arg Lys Ala Ile Lys Ser Ala Arg Gly Val
                   85                  90                  95
   Glu Phe Met Thr Asn Ala Lys Trp Met Lys Trp Ala Ile Asp Asp Arg
               100                 105                 110
   Met Asp Ile Gln Pro Leu Lys Val Ala Ile Asp His Tyr Asn Ala Val
           115                 120                 125
   Asn His Gln Leu Phe Asn Cys Ile Val Lys Ala Arg Ser Ala Asn Ala
       130                 135                 140
   Asp Thr Ile Tyr Tyr Asp Tyr Phe Pro Leu Glu Ser Lys Val Lys Lys
   145                 150                 155                 160
   Cys Asn His Ala Asn Leu Asp Leu Leu Arg Ser Leu Thr Asn Thr Glu
                   165                 170                 175
   Met Phe His Met Leu Gln Gly Ala Ala Tyr Ser Leu Lys Ser Asn Tyr
               180                 185                 190
   Glu Leu Ile Thr Asn Ser Glu Arg Asn Ser Thr Glu Glu Thr Tyr Ala
           195                 200                 205
   Pro Gly Val His Asn Gln Ile Arg Leu Val Arg Gly Thr Arg Ile Gly
       210                 215                 220
   Tyr Lys Gly Glu Ala Tyr Ser Arg Phe Val Ser Ser Leu Val Gln Val
   225                 230                 235                 240
   Arg Ile Gln Gly Arg Thr Pro Pro Glu Ile Val Asp Asp Ile Ala Arg
                   245                 250                 255
   Leu Asn Val Ile Arg Thr Glu Trp Ile Asn Ala Gln Phe Asp Ser Thr
               260                 265                 270
   Lys Ile Arg Ala Leu Glu Leu Cys Lys Ile Leu Ser Ala Ile Gly Arg
           275                 280                 285
   Lys Met Leu Asn Thr His Glu Glu Pro Lys Asp Glu Met Asp Leu Ser
       290                 295                 300
   Thr Lys Phe Gln Phe Lys Leu Asp Asp Lys Phe Lys Lys Thr Asp Ser
   305                 310                 315                 320
   Glu His Ile Asn Ile Phe Asn Val Arg Ala Pro Ala Thr His Glu Gly
                   325                 330                 335
   Arg Phe Tyr Ala Leu Ile Ala Ile Ala Ala Thr Asp Thr Gln Arg Gly
               340                 345                 350
   Arg Ile Trp Arg Thr Asn Pro Tyr Pro Cys Leu Arg Gly Ala Leu Ile
           355                 360                 365
   Ala Ala Glu Cys Glu Leu Gly Asp Val Tyr His Thr Leu Arg Gln Val
       370                 375                 380
   Tyr Lys Trp Ser Leu Arg Gln Asp Tyr Gly Arg Thr Glu Val Pro Leu
   385                 390                 395                 400
   Glu Asn Asn Lys Tyr Val Phe Ser Arg Ile Asn Leu Phe Asp Ser Asn
                   405                 410                 415
   Leu Asp Val Gly Asp Gln Val Val His Trp Met Tyr Glu Val Asp Gly
   420 425 430
   Pro Ala Glu Thr Thr Tyr Asp Asn Gly Tyr Met Cys Lys Thr Glu Arg
   435 440 445
   Glu Asp Glu Glu Leu Val Cys Lys Ile Ser Glu Glu Lys Tyr Lys Thr
   450 455 460
   Met Leu Asp Arg Met Ile Gln Gly Gly Trp Asp Gln Glu Arg Phe Lys
   465 470 475 480
   Leu Tyr Ser Ile Leu Thr Asp Pro Asn Leu Leu Thr Ile Asp Phe Glu
   485 490 495
   Lys Asp Ala His Leu Asn Ile Arg Ser Glu Phe Val Leu Pro Ser Tyr
   500 505 510
   Phe Asp Gln Trp Ile Tyr Ser Pro Met Phe Asn Ala Arg Leu Arg Ile
   515 520 525
   Thr His Gly Glu Ile Ala Thr Arg Lys Ser Ala Asp Pro Trp Asn Lys
   530 535 540
   Arg Val Val Phe Gly Tyr Ile Lys Ala Ser Thr Glu Ser Pro Glu Tyr
   545 550 555 560
   Ala Leu Gly Gln Tyr Phe Asp Met Arg Ile Gln Phe Tyr Gly Asp Ala
   565 570 575
   Leu Ser Ser Lys Gln Asn Gln Ser Ala Val Phe Gln Tyr Leu Ser Gln
   580 585 590
   Gln Glu Asp Phe Pro Thr Leu Thr Ser Tyr Ala Lys Gly Asp Val Val
   595 600 605
   Cys Pro His Ser Gly Gly Ala Leu Tyr Thr Phe Arg Arg Val Ala Leu
   610 615 620
   Met Leu Met Ala Asn Tyr Glu Lys Leu Ser Pro Asp Leu His Glu Gly
   625 630 635 640
   Met Glu Asp Tyr Thr Tyr Thr His Pro Ser Ile Gly Gly Ala Tyr Gln
   645 650 655
   Lys Arg Ile Leu Glu Met Arg Asp Phe Ser Gln Leu Ile Cys Phe Ile
   660 665 670
   Ile Asp Tyr Ile Phe Glu Arg His Asp Gln Leu Arg Asp Ala Lys Glu
           675                 680                 685
   Ala Arg Arg Ile Leu Tyr Leu Ile Gln Asn Leu Asp Glu Pro Gln Arg
       690                 695                 700
   Leu Glu Met Leu Asn Val Thr Phe Pro Asn Phe Phe Arg His Phe Leu
   705                 710                 715                 720
   Lys Leu Lys Asp Val Gln Leu Ile Ser Asp Leu Asn Val Ile Asn Phe
                   725                 730                 735
   Leu Pro Leu Leu Phe Leu Val Gln Asp Asn Ile Ser Tyr Trp His Arg
               740                 745                 750
   Gln Trp Ala Val Pro Met Ile Leu Tyr Asp Asp Val Ile Arg Leu Ile
           755                 760                 765
   Pro Val Glu Val Gly Ala Tyr Ala Asn Arg Phe Gly Ile Lys Ser Phe
       770                 775                 780
   Phe Asn Phe Thr Arg Phe His Pro Gly Asp Ala Lys Lys Arg Gln Lys
   785                 790                 795                 800
   Ala Asp Asp Thr His Lys Glu Phe Gly Ser Ile Ser Phe Asn Tyr Tyr
                   805                 810                 815
   Ala Ser Thr Lys Ile Ala Gln Gly Gly Val His Thr Pro Val Val Thr
               820                 825                 830
   Thr Lys Leu Asp Thr Leu Lys Ile His Leu Ser Ser Leu Cys Ala Gly
           835                 840                 845
   Leu Ala Asp Ser Ile Val Tyr Thr Leu Pro Val Ala His Pro Lys Lys
       850                 855                 860
   Cys Ile Val Leu Ile Ile Val Gly Asp Asp Lys Leu Glu Pro His Val
   865                 870                 875                 880
   Arg Ser Glu Gln Val Val Ser Lys Tyr Tyr Phe Ser Arg Arg His Val
                   885                 890                 895
   Ser Gly Ile Val Ser Ile Cys Val Gly Gln Asp Asn Gln Leu Lys Val
               900                 905                 910
   Tyr Ser Ser Gly Ile Val Arg His Arg Val Cys Glu Lys Phe Ile Leu
           915                 920                 925
   Lys Tyr Lys Cys Lys Val Val Leu Val Lys Met Pro Gly Tyr Val Phe
   930 935 940
   Gly Asn Asp Glu Leu Met Thr Lys Leu Leu Asn Val
   945 950 955
<210> 16
   <211> 950
   <212> PRT
   <213> Artificial sequence
<220>
   <223> BTV-12 VP2 consensus sequence
<400> 16
   Met Glu Asp Phe Pro Ile Cys Val Ile Glu Arg Glu Ser Lys Phe Asp
   1 5 10 15
   Glu Asp Phe Val Val Arg Tyr Pro Val Ile Ile Asp Leu Lys Glu Cys
   20 25 30
   Val Asp Asp Val Asn Gly Lys Thr Asp Val Ser Lys Leu Glu Glu Thr
   35 40 45
   Arg Gly Ile Phe Val Arg Ala Glu Gly Ile Arg Val Ala Leu Asn Ser
   50 55 60
   Arg Pro Asp Asn Leu Ser His Val Ile Thr Pro Ser Ala Leu Asp Val
   65 70 75 80
   Leu Ile Arg Ala Tyr Asp Arg Lys His Lys Ile Ala Leu Asp Lys Arg
   85 90 95
   Thr Asp Asp Ser Gly Arg Met Ala Asp His Asn Ile Asp Val Trp Val
   100 105 110
   Lys Lys Arg Leu Glu Glu Gln Ala Asp Leu Gln Ala Ile Tyr His Asp
   115 120 125
   Val Asp Leu Gly Arg Gly Lys Leu Lys Phe Lys Met Leu Thr Gly Leu
   130 135 140
   Val Lys Leu Asp Ser Asn Tyr Ala Glu Thr Val Gly Tyr Asp Tyr Ala
   145 150 155 160
   Pro Val Pro Lys Val Gly Cys Asp His Gly Asn Val Asp Phe Leu Tyr
   165 170 175
   Asp Phe Ile Leu Ser Gly Ser Leu Gln Val Ser Lys Thr Ala Gly Tyr
               180                 185                 190
   Thr Leu Lys Gln Thr Tyr Arg Leu Thr Val His Ala Glu Ala Asn Ala
           195                 200                 205
   Glu Thr Arg Asp Gln Leu Ile Val Asn Gly Val Tyr Thr Glu Gly Val
       210                 215                 220
   Thr Arg Gly Glu Glu Ile Arg Asp Met Lys Lys Met Cys Tyr Ser Arg
   225                 230                 235                 240
   Phe Arg Asp Gly Trp Ile Arg Ala Thr Ile Gln Pro Lys Ile Val Glu
                   245                 250                 255
   Glu Leu Arg Arg Ser Lys Glu Ala Leu Asp Arg Ile Ser Ser Glu Trp
               260                 265                 270
   Tyr Glu Lys Leu Thr Thr Pro Asp Val Val Glu Ile Cys Lys Ile Val
           275                 280                 285
   Ser Ser Ile Gly Arg Gln Met Trp Asn Ser Glu Glu Leu Pro Val Asp
       290                 295                 300
   Asp Ala Met Arg Ser Lys Val Phe Gln Glu Glu Leu Arg Leu Leu Phe
   305                 310                 315                 320
   Arg Val Gly Asn Ser Glu Tyr Asp Thr Ile Gln Ala Ile Arg Ser Gly
                   325                 330                 335
   Gln Thr Pro Leu Lys Lys Phe Tyr Ala Leu Leu Ala Ile Ala Ala Thr
               340                 345                 350
   Asp Ser Tyr Arg Trp Arg Ile Trp Trp Ser Asn Pro Tyr Pro Cys Leu
           355                 360                 365
   Arg Gly Thr Ile Ile Ala Cys Glu Met Glu Leu Gly Asp Val Tyr Lys
       370                 375                 380
   Thr Leu Arg Ser Thr Phe His Trp Ser Leu Arg Pro Thr Tyr Ala Thr
   385                 390                 395                 400
   Arg Lys Glu Ile Asp Arg Glu Arg Arg Ala Tyr Pro Tyr Gln Arg Ile
                   405                 410                 415
   Asn Leu Phe Glu Ser Arg Leu Asp Pro Gly Thr Arg Ile Ile Asn Trp
               420                 425                 430
   Leu Val Val Arg Glu Pro Leu Ala Glu Pro Asp Val Lys Ser Gly Arg
   435 440 445
   Val Cys Met Thr Ser Thr Glu Ser Glu Tyr Ile Leu Lys Val Asp Asp
   450 455 460
   Thr Lys Tyr Arg Glu Met Ile Gly Gln Ile Ile Glu Arg Gly Trp Glu
   465 470 475 480
   Lys Glu His Leu Lys Leu Glu Lys Ile Val Gln Asp Glu Gly Asn Val
   485 490 495
   Phe Gln Met Glu Phe Glu Lys Asp Ala Ser Leu Asp Glu Arg Ser His
   500 505 510
   Leu Val Met Pro Tyr Tyr Tyr Asp Lys Glu Ile His Cys Pro Met Tyr
   515 520 525
   His Lys Lys Ile Lys Ile Thr Glu Ala Glu Ile Ala Thr Ser Trp Ser
   530 535 540
   Asp Asp Pro Trp Leu Asn Arg Val Leu Asn Gly Phe Leu Gly Asp His
   545 550 555 560
   Val Glu Thr Phe Asn Leu Gly Cys Asn His Ile Phe Asp Met Arg Val
   565 570 575
   Pro Leu Lys Gly Asn Leu Leu Thr Gln Glu Gln Ala Ile Ser Ser Tyr
   580 585 590
   His Glu His Ile Arg Ser Pro Glu Ala Gln Ser Glu Asn Gln Cys Ala
   595 600 605
   Leu Lys Val Tyr Ala Asp Glu Ala Val Ser Lys Leu Phe Thr Thr Leu
   610 615 620
   Tyr His Arg Leu Ala Tyr His Leu Pro Lys Met Glu Tyr Ile Glu Arg
   625 630 635 640
   Glu Glu Glu Val Ser Gln Tyr Glu Gly Leu Val Lys Arg Ile Arg Thr
   645 650 655
   Trp Asp Gln Phe Ile Ile Phe Val Phe Asp Phe Phe Phe Glu Asn Arg
   660 665 670
   Lys Gly Leu Arg Thr Thr Glu Glu Ala Thr Gln Leu Ala Lys Ala Ile
   675 680 685
   Arg Ser Asn Val Gly Ala Thr Arg Trp Thr Met Met Ala Ser Ala Phe
       690                 695                 700
   Pro Thr Ser Val Arg Leu Val Lys Gly Ile Leu Arg Ala Lys Thr Trp
   705                 710                 715                 720
   Gly Glu Cys Tyr Ile Met Asn Phe Leu Pro Leu Leu Ile Cys Gly Gly
                   725                 730                 735
   Ser Ser Ile Ser Tyr Leu His Arg Glu Trp Ser Tyr Pro Met Ile Val
               740                 745                 750
   Val Phe Glu Asp Gly Ile Lys Val Val Pro Ala Met Thr Gly Arg Ser
           755                 760                 765
   Leu Thr Gln Gly Ser Leu Gly Glu Trp Ala Ser Phe Val Cys Phe Phe
       770                 775                 780
   Met Gly Ser Asn His Glu Lys Ile Lys Leu Glu Glu Asp Glu Leu Glu
   785                 790                 795                 800
   Val Leu Arg Lys Cys Val Glu Phe Tyr Glu Gly Val Lys Met Val Asp
                   805                 810                 815
   Ile Ile Lys Asp Thr Pro Val Arg Met Thr Lys Tyr Ile Ala Cys Asp
               820                 825                 830
   Leu Ala Phe Gly Ser Gly Cys Gly Gly Thr Ser Glu Ser Leu Ser Phe
           835                 840                 845
   Ile Leu Pro Val Thr His Pro Asn Arg Ser Leu Ile Ile Phe Val Val
       850                 855                 860
   Ala Asp Asp Lys Ile Ser Pro Ala Gln His Ala Ala Arg Ala Lys Arg
   865                 870                 875                 880
   Arg Phe Arg Gly Thr Arg Glu Tyr Val Asn Asp Val Ile Val Leu Gln
                   885                 890                 895
   Met Lys Arg Pro Gly Thr Val Gly Asn Val Trp Ser Glu Lys His Ser
               900                 905                 910
   Lys Val Arg Ile Cys Arg Arg Asn Phe Leu Lys Tyr Asp His Lys Val
           915                 920                 925
   Val Leu Thr Lys Phe Ala Gly Leu Val Tyr Gly Asn Tyr Glu Leu Ile
   930 935 940
   Thr Lys Leu Ala Asn Leu
   945 950
<210> 17
   <211> 959
   <212> PRT
   <213> Artificial sequence
<220>
   <223> BTV-13 VP2 consensus sequence
<220>
   <221> misc_feature
   <222> (50)..(50)
   <223> Xaa can be any naturally occurring amino acid
<220>
   <221> misc_feature
   <222> (72)..(72)
   <223> Xaa can be any naturally occurring amino acid
<220>
   <221> misc_feature
   <222> (84)..(84)
   <223> Xaa can be any naturally occurring amino acid
<220>
   <221> misc_feature
   <222> (142)..(142)
   <223> Xaa can be any naturally occurring amino acid
<220>
   <221> misc_feature
   <222> (183)..(183)
   <223> Xaa can be any naturally occurring amino acid
<220>
   <221> misc_feature
   <222> (236)..(236)
   <223> Xaa can be any naturally occurring amino acid
<220>
   <221> misc_feature
   <222> (314)..(314)
   <223> Xaa can be any naturally occurring amino acid
<220>
   <221> misc_feature
   <222> (369)..(369)
   <223> Xaa can be any naturally occurring amino acid
<220>
   <221> misc_feature
   <222> (407)..(407)
   <223> Xaa can be any naturally occurring amino acid
<220>
   <221> misc_feature
   <222> (416)..(416)
   <223> Xaa can be any naturally occurring amino acid
<220>
   <221> misc_feature
   <222> (448)..(448)
   <223> Xaa can be any naturally occurring amino acid
<220>
   <221> misc_feature
   <222> (463)..(463)
   <223> Xaa can be any naturally occurring amino acid
<220>
   <221> misc_feature
   <222> (466)..(466)
   <223> Xaa can be any naturally occurring amino acid
<220>
   <221> misc_feature
   <222> (473)..(473)
   <223> Xaa can be any naturally occurring amino acid
<220>
   <221> misc_feature
   <222> (486)..(486)
   <223> Xaa can be any naturally occurring amino acid
<220>
   <221> misc_feature
   <222> (572)..(572)
   <223> Xaa can be any naturally occurring amino acid
<220>
   <221> misc_feature
   <222> (602)..(602)
   <223> Xaa can be any naturally occurring amino acid
<220>
   <221> misc_feature
   <222> (608)..(608)
   <223> Xaa can be any naturally occurring amino acid
<220>
   <221> misc_feature
   <222> (704)..(704)
   <223> Xaa can be any naturally occurring amino acid
<220>
   <221> misc_feature
   <222> (738)..(738)
   <223> Xaa can be any naturally occurring amino acid
<220>
   <221> misc_feature
   <222> (744)..(744)
   <223> Xaa can be any naturally occurring amino acid
<220>
   <221> misc_feature
   <222> (755)..(755)
   <223> Xaa can be any naturally occurring amino acid
<220>
   <221> misc_feature
   <222> (769)..(769)
   <223> Xaa can be any naturally occurring amino acid
<220>
   <221> misc_feature
   <222> (771)..(771)
   <223> Xaa can be any naturally occurring amino acid
<220>
   <221> misc_feature
   <222> (805)..(805)
   <223> Xaa can be any naturally occurring amino acid
<220>
   <221> misc_feature
   <222> (807)..(807)
   <223> Xaa can be any naturally occurring amino acid
<220>
   <221> misc_feature
   <222> (813)..(813)
   <223> Xaa can be any naturally occurring amino acid
<220>
   <221> misc_feature
   <222> (833)..(833)
   <223> Xaa can be any naturally occurring amino acid
<220>
   <221> misc_feature
   <222> (842)..(842)
   <223> Xaa can be any naturally occurring amino acid
<220>
   <221> misc_feature
   <222> (862)..(862)
   <223> Xaa can be any naturally occurring amino acid
<220>
   <221> misc_feature
   <222> (886)..(886)
   <223> Xaa can be any naturally occurring amino acid
<400> 17
   Met Glu Glu Leu Val Ile Pro Val Ile Thr Glu Arg Phe Asp Lys Arg
   1               5                   10                  15
   Leu Val Gly Arg Tyr Asp Tyr Val Ile Glu Leu Ala Arg Pro Glu Gly
               20                  25                  30
   Asp Glu Trp Ser Gly His Asp Val Thr His Ile Pro Asp Lys Arg Met
           35                  40                  45
   Phe Xaa Ile Lys Val Gln Pro Ile Arg Asp Ala Ile Asp Tyr Lys Pro
       50                  55                  60
   Val Glu Asn Asp Gly Glu Val Xaa Pro Arg Ile Leu Asp Met Ser Ile
   65                  70                  75                  80
   Ala Cys Tyr Xaa Met Arg Lys Arg Met Met Lys Arg Asp Gly Val Asp
                   85                  90                  95
   Phe Val Ser Asn Thr Lys Trp Leu Glu Trp Met Ile Gln Asp Ser Met
               100                 105                 110
   Asp Val Gln Pro Leu Arg Val Asp Met Lys Glu Asp His Ser Thr Val
           115                 120                 125
   Gln Tyr Asp Met Phe Ser Ala Lys Leu His Val Asp Ser Xaa Lys Ala
       130                 135                 140
   Asp Thr Thr Ser Tyr Asn Ile Leu Ala Leu Glu Val Lys Lys Gly Ala
   145                 150                 155                 160
   Gln Cys His His Val His Thr Asn Ile Trp Asn His Met Ile Arg Asn
                   165                 170                 175
   His Leu Phe His Ala Val Xaa Glu Ser Cys Tyr Ile Phe Lys Pro Thr
               180                 185                 190
   Tyr Lys Leu Ile Val Asn Ser Glu Arg Arg Thr Pro Asp Glu Asp Phe
           195                 200                 205
   Gln Ile Gly Asn Pro Gln Phe Pro Thr Leu Arg Arg Asn Gln Gln Ile
       210                 215                 220
   Phe Leu Gly Asp Asp Ala Tyr Lys Lys Thr Ala Xaa Gly Leu Val Gln
   225                 230                 235                 240
   Val Leu Val Asn Gly Val Val Pro Asp Ile Ile Arg Asn Glu Ile Ala
                   245                 250                 255
   Ala Leu Asp Ala Ile Arg Asp Lys Trp Ile Gln Gly Asn Tyr Glu Arg
               260                 265                 270
   Thr His Ile Lys Ser Leu Glu Leu Cys Asn Leu Leu Ser Ala Ile Gly
           275                 280                 285
   Arg Lys Met Val Asn Leu Glu Glu Glu Pro Lys Asp Glu Arg Asp Leu
       290                 295                 300
   Ser Leu Arg Phe Gln His Lys Leu Asp Xaa Lys Phe Ala Lys Asn Asp
   305                 310                 315                 320
   Gln Glu Arg Asn Val Ile Phe Ala Gln Lys Ser Gln Arg Asn Asp Gln
   325 330 335
   Asp Arg Phe Tyr Val Leu Met Val Ile Ala Ala Ser Asp Thr Asn Asn
   340 345 350
   Ser Arg Val Trp Trp Ser Asn Pro Tyr Pro Cys Leu Arg Gly Ala Leu
   355 360 365
   Xaa Ala Ala Glu Cys Lys Leu Gly Asp Val Tyr Tyr Lys Leu Arg Ser
   370 375 380
   Trp Tyr Glu Trp Ser Val Arg Glu Gly Tyr Lys Pro Arg Asp Leu Asp
   385 390 395 400
   Arg Gln Tyr Glu Lys Tyr Xaa Val Gly Arg Val Asn Leu Phe Asp Xaa
   405 410 415
   Glu Ala Glu Pro Gly Thr Lys Val Leu His Trp Glu Tyr Glu Leu Ile
   420 425 430
   Ser Lys Leu Tyr Thr Val Ser Asn His Glu Gly Asn Gln Cys Asp Xaa
   435 440 445
   Tyr Pro Asp Glu Gly Glu Ile Val Thr Lys Phe Asp Asp Ala Xaa Tyr
   450 455 460
   Ser Xaa Met Ile Gln Thr Ile Ile Xaa Glu Gly Trp Lys Gln Gly Asp
   465 470 475 480
   Phe Lys Met Phe Lys Xaa Leu Lys Asp Glu Gly Asn Pro Leu Leu Tyr
   485 490 495
   Asp Leu Glu Lys Asp Ile Lys Leu Asp Arg Val Ser Arg Val Val Phe
   500 505 510
   Pro Pro Tyr Phe Asp Gln Trp Thr Tyr Val Pro Met Phe Asn Ala Arg
   515 520 525
   Ile Lys Pro Cys Glu Val Glu Leu Ala Glu Arg Lys Asn Ile Asp Pro
   530 535 540
   Tyr Val Lys Arg Thr Leu Arg Pro Leu Lys Ala Asp Cys Ile Glu Leu
   545 550 555 560
   Met Arg Tyr His Met Ser Gln Tyr Met Asp Leu Xaa Val Ser Leu Gln
   565 570 575
   Gly Thr Ser Leu Ser Ile Lys Gln Val Pro Ser Ser Ile His Gln Ser
               580                 585                 590
   Leu Ala Arg Asp Ala Ser Tyr Ala Gly Xaa Leu Ser Arg Arg Arg Xaa
           595                 600                 605
   Asn Leu Asp Tyr Lys Ser Gln Cys Pro Ile Val Thr Asn Leu Phe Leu
       610                 615                 620
   Leu Glu Lys Phe Phe Leu Leu Ile Phe Thr Thr Met Glu Lys His Tyr
   625                 630                 635                 640
   Trp Glu Met Asp Asp Asp Glu Thr Glu Tyr Glu His Pro Lys Ile Asp
                   645                 650                 655
   Pro Ser Lys Phe Glu Val Glu Gly Thr Leu His Asp Val Ser Gln Val
               660                 665                 670
   Met Val His Leu Phe Asp Arg Phe Phe Glu Lys Arg Arg Phe Leu Arg
           675                 680                 685
   Thr Val Asp Glu Ser Arg Trp Ile Leu His Leu Ile Arg Ser Thr Xaa
       690                 695                 700
   Gly Ala Lys Arg Leu Glu Val Leu Ser Arg Phe Phe Pro Ala Phe Ser
   705                 710                 715                 720
   Asp Gly Leu Arg Ile Arg Glu Phe Lys Lys Val Arg Asp Ile Met Leu
                   725                 730                 735
   Leu Xaa Phe Leu Pro Phe Leu Xaa Leu Thr Gly Asp Asn Ile Ala Tyr
               740                 745                 750
   Glu His Xaa Gln Trp Ala Val Pro Val Ile Phe Tyr Ala Asp Lys Ile
           755                 760                 765
   Xaa Ile Xaa Pro Ala Glu Val Gly Ala Tyr Tyr Asn Arg Phe Gly Leu
       770                 775                 780
   Thr Cys Ile Leu Glu Leu Met Met Phe Phe Pro Ser Tyr Asp Thr Arg
   785                 790                 795                 800
   Asn Glu Asp Leu Xaa Glu Xaa Val Arg Ala Cys Ile Xaa Pro Ile Ile
                   805                 810                 815
   Asn Tyr Tyr Leu Asp Thr Thr Ile Ser Asn Gly Gly Ile Gln Thr Ser
   820 825 830
   Xaa Val Ser Thr Lys Ala Leu Leu Tyr Xaa Thr Tyr Leu Ser Ser Ile
   835 840 845
   Cys Gly Gly Phe Ser Glu Ala Ile Leu Trp Tyr Leu Pro Xaa Thr His
   850 855 860
   Pro Ser Lys Cys Leu Ile Ala Leu Glu Val Ser Asp Ala Leu Thr Ser
   865 870 875 880
   Pro Glu Leu Arg Ile Xaa Lys Ile Lys Arg Arg Phe Pro Leu Ser Ser
   885 890 895
   Asn His Leu Lys Gly Ile Val Gln Ile Ser Val Arg Pro Asp Arg Thr
   900 905 910
   Phe Ser Val Val Thr Gln Gly Ile Val Lys His Arg Val Cys Lys Lys
   915 920 925
   Thr Leu Leu Arg Tyr Arg Cys Asp Val Ile Leu Ile Gln Thr Pro Gly
   930 935 940
   Tyr Val Phe Gly Asn Asp Glu Leu Leu Thr Lys Leu Leu Asn Ile
   945 950 955
<210> 18
   <211> 955
   <212> PRT
   <213> Artificial sequence
<220>
   <223> BTV-14 VP2 consensus sequence
<400> 18
   Met Asp Glu Leu Ala Ile Pro Ile Ile Asn Gly His Phe Pro Lys Glu
   1               5                   10                  15
   Thr Leu Ile Glu Tyr Asp Cys Ile Ile Glu Leu Asn Pro Pro Val Glu
               20                  25                  30
   Asp Asp Ala Ala Asp Val Val Gln Ile Pro Thr Arg Asp Met Met Asp
           35                  40                  45
   Ile Pro Asn Val Pro Ile Arg Asp Ala Leu Lys His Leu Pro Gln Arg
       50                  55                  60
   Asn Asp Gly His Val Leu Thr Arg Val Leu Asp Ile Ser Leu Ser Gly
   65                  70                  75                  80
   Tyr Asp Arg Arg Lys Tyr His Lys Thr Asn Ser Gly Gly Glu Phe Tyr
                   85                  90                  95
   Ser Pro Asn Glu Trp Leu Asn Trp Met Ile Asn Asp Ser Met Asp Val
               100                 105                 110
   Gln Pro Leu Lys Val Ser Leu Gly Leu Pro Pro Thr Ile Arg His Glu
           115                 120                 125
   Leu Phe Ser Ser Ser Val Phe Ile Ser Ala Lys Lys Ala Asp Thr Thr
       130                 135                 140
   Ser Tyr His Ile Glu Pro Ile Cys Glu Lys Asp Lys Ala Cys Asp His
   145                 150                 155                 160
   Thr Arg Val Ser Met Trp Asn Asn Leu Thr Arg His Glu Met Met His
                   165                 170                 175
   Cys Ala Gln Glu Ser Ala Tyr Val Leu Lys Pro Thr Tyr Asp Ile Thr
               180                 185                 190
   Val Tyr Ser Glu Arg Ala Thr Thr Asp Glu Ala Phe Gln Gln Val Gly
           195                 200                 205
   Gln Lys Phe Val Thr Ile Thr Lys Asn His Arg Val Val Leu Gly Asp
       210                 215                 220
   Asp Ala Tyr Lys Lys Thr Leu Gly Gly Leu Thr Arg Leu Arg Val Gln
   225                 230                 235                 240
   Gly Lys Ile Pro Ser Leu Ile Gln Asn Asp Ile Gln Gln Leu Asn Gln
                   245                 250                 255
   Ile Arg Asp Thr Trp Ile Arg Asp Ser Tyr Asn Pro Arg Asn Ile Arg
               260                 265                 270
   Ser Leu Glu Leu Cys Lys Leu Leu Ser Gly Ile Gly Arg Lys Met Val
           275                 280                 285
   Cys Ile Glu Glu Glu Pro Lys Asn Glu Ser Asp Leu Ser Val Lys Phe
       290                 295                 300
   Gln Phe Lys Leu Asp Glu Lys Phe Arg Leu Asp Asp Ser Glu Arg Gly
   305                 310                 315                 320
   Val Ile Phe Thr Asn Lys Gly Gln Arg Asn Asp Gln Asp Arg Phe Tyr
   325 330 335
   Val Leu Ile Met Ile Ala Ala Ser Asp Thr Tyr Asn Ser Arg Ile Trp
   340 345 350
   Trp Thr Asn Pro Tyr Pro Cys Leu Arg Gly Ala Leu Ile Ala Ala Glu
   355 360 365
   Thr Gln Leu Gly Asp Val Tyr Trp Thr Leu Arg His Phe Tyr Glu Trp
   370 375 380
   Ser Val Arg Pro Ala Tyr Ser Pro Arg Leu Arg Glu Arg Glu Asp Asp
   385 390 395 400
   Lys Tyr Ile Tyr Gly Arg Val Asn Leu Phe Asp Leu Asp Ala Ser Pro
   405 410 415
   Gly Thr Lys Ile Ile His Trp Thr Tyr Asp Leu Ile Thr Arg Glu Ser
   420 425 430
   Lys Ile Thr Tyr His Asn Gly Asn Pro Cys Asp Leu Asn Pro Asp Val
   435 440 445
   Asp Gly Ile Ile Thr Lys Phe Asn Lys Thr Ala Tyr Thr Asp Met Ile
   450 455 460
   Gly Glu Leu Leu Asn Gly Gly Trp Asn Thr Arg Glu Phe Lys Met Tyr
   465 470 475 480
   Lys Ile Leu Glu Ser Val Gly Asn Val Leu Thr Ile Asp Phe Glu Lys
   485 490 495
   Asp Ala Lys Leu Asn Ser Arg Ser Glu Phe Ile Leu Pro Asp Tyr Tyr
   500 505 510
   Asp Lys Trp Ile Tyr Ala Pro Met Phe Asn Ala Lys Leu Lys Ile Thr
   515 520 525
   Glu Val Glu Ile Ala Gln Ala Lys Ser Asp Asp Pro Asp Val Val Arg
   530 535 540
   Thr Leu Ala Pro Ile Gly Ser Asp Pro Ile Thr Leu Gln Arg Leu Cys
   545 550 555 560
   Leu Gly Asn Tyr Tyr Asp Ile Arg Pro Ala Met His Gly Arg Ala Leu
   565 570 575
   Met Lys Lys Gln Glu Gln Thr Ser Tyr His Lys Thr Leu Ala Lys Thr
   580 585 590
   Lys Asp Tyr Ser Asp Val Leu Asn Arg Arg Gly Tyr Arg Lys Val Glu
   595 600 605
   Lys Pro Cys Pro Met Val Thr Cys Ser Tyr Val Leu Glu Lys Thr Ala
   610 615 620
   Leu Phe Trp Ile Asp Val Met Glu Lys His Val Gly Asp Ile Glu Asp
   625 630 635 640
   Ser Thr Asp Glu Phe Asp Phe Pro Arg Val Asp Val Thr Leu Pro Val
   645 650 655
   Glu Thr His Lys Val Val Asp Val Ala Gln Leu Ser Val Leu Leu Ile
   660 665 670
   Asp Phe Leu Tyr Glu Lys Arg Arg Thr Val Arg Asp Ile Asp Ser Ala
   675 680 685
   Arg Trp Thr Ile Glu Leu Ile Arg Arg Val Arg Gly Lys Asp Arg Leu
   690 695 700
   Glu Ala Ile Arg Arg Glu Phe Pro Asn Phe Gly Glu Ala Val Thr Ile
   705 710 715 720
   Leu Met Asn Pro Thr Arg Val Lys Asp Val Met Val Ile Asn Phe Leu
   725 730 735
   Pro Phe Leu Ile Leu Leu Gly Asp Asn Ile Ser Tyr Glu His Arg Gln
   740 745 750
   Trp Ser Ile Pro Ile Ile Leu His Ala Asp Ser Met Trp Phe Leu Pro
   755 760 765
   Val Glu Val Gly Ala Phe Tyr Asn Arg Phe Gly Val Ile Ser Phe Leu
   770 775 780
   Glu Tyr Met Met Phe Phe Pro Ser Ala Gln Ser Arg Glu Ser Glu Leu
   785 790 795 800
   Asp Glu Ala Glu Ile Lys Val Ser Lys Leu Ile Leu Glu Phe Tyr Leu
   805 810 815
   Asp Thr Thr Ile Phe Ser Gly Gly Val Gln Arg Ser Val Val Ser Thr
   820 825 830
   Lys Gln Ile Leu Tyr Glu Thr Tyr Leu Ser Ser Leu Cys Gly Gly Tyr
   835 840 845
   Ser Glu Gly Leu Val Trp Tyr Leu Pro Ile Thr His Pro Thr Lys Cys
   850 855 860
   Val Val Ala Ile Glu Phe Ser Asp Asp Arg Val Asp Ala Ala His Arg
   865 870 875 880
   Cys Asn Arg Leu Arg Ala Arg Phe Pro Leu Ser Ala Val His Leu Lys
   885 890 895
   Gly Ile Val Ile Leu Ser Ile Ala Lys Asp Arg Ala Val Lys Ala Tyr
   900 905 910
   Thr Glu Gly Ile Val Thr His Arg Leu Cys Lys Lys Asn Val Leu Gly
   915 920 925
   Phe Thr Cys Gln Ile Leu Leu Leu Lys Phe Ser Gly His Val Phe Gly
   930 935 940
   Asn Asp Glu Met Leu Thr Lys Leu Leu Asn Val
   945 950 955
<210> 19
   <211> 952
   <212> PRT
   <213> Artificial sequence
<220>
   <223> BTV-15 VP2 consensus sequence
<220>
   <221> misc_feature
   <222> (293)..(293)
   <223> Xaa can be any naturally occurring amino acid
<220>
   <221> misc_feature
   <222> (479)..(479)
   <223> Xaa can be any naturally occurring amino acid
<220>
   <221> misc_feature
   <222> (553)..(553)
   <223> Xaa can be any naturally occurring amino acid
<220>
   <221> misc_feature
   <222> (637)..(637)
   <223> Xaa can be any naturally occurring amino acid
<220>
   <221> misc_feature
   <222> (676)..(676)
   <223> Xaa can be any naturally occurring amino acid
<400> 19
   Met Glu Asp Phe Pro Val Ser Val Ile Glu Arg Asn Ser Thr Ile Ala
   1 5 10 15
   Pro Ala Ile Ile Lys Arg Tyr Pro Ile Ile Val Asp Val Arg Asn Met
   20 25 30
   Val Ala Leu Ser Gly Gly Lys Asn Asp Val Thr Asn Ile Pro Arg Val
   35 40 45
   Arg Asn Ile Gly Val Arg Gln Glu Gly Ile Arg Val Gly Leu Lys Tyr
   50 55 60
   Arg Pro Asp Thr Glu Thr Asn Val Ile Phe Pro Lys Asn Leu Asp Val
   65 70 75 80
   Leu Ile Arg Ala Tyr Asp His Lys His Arg Leu Thr Arg Asp Lys Arg
   85 90 95
   Pro Thr Asp His Asp Val Asp Ser Ile Ser Asp Gly Asp Val Thr Val
   100 105 110
   Trp Val Thr Asn Arg Thr Asn Glu Phe Tyr Asp Ile Gln Pro Ile Tyr
   115 120 125
   His Thr Leu Glu Asp Glu Val Cys Lys Val Lys Phe Thr Ser Leu Leu
   130 135 140
   Gly Ser Val Ser Thr Asp Ser Asn Tyr Ala Glu Ser Met Ser Tyr His
   145 150 155 160
   Tyr Ile Gly Val Glu Arg Asp Lys Cys Asp His Ser Lys Trp His Arg
   165 170 175
   Phe Phe Pro Met Leu Leu Ser Asp Ser Ile Asn Leu Gly Lys Glu Val
   180 185 190
   Gly Tyr Ile Leu Lys Glu Thr Tyr Lys Ile Lys Ala Ile Gly Glu Ala
   195 200 205
   Arg Glu Gly Gln Arg Gln Glu Leu Arg Val Asp Val Pro Tyr Thr Pro
   210 215 220
   Gly Ile Gly Arg Asn Glu Arg Ile Ser Asp Met Lys Leu Pro Val Tyr
   225                 230                 235                 240
   Lys Arg Phe Leu Glu Gly Tyr Ile Arg Cys Gln Ile Glu Pro Asn Val
                   245                 250                 255
   Pro Glu Lys Leu Thr Glu Leu Lys Asn Gln Leu Asp Ala Ile Cys Val
               260                 265                 270
   Ala Trp Tyr Gly Gly Gln Val Pro Ile Val Ala Asp Glu Ile Cys Lys
           275                 280                 285
   Ile Thr Ser Arg Xaa Gly Arg Leu Met Trp Asn Thr Glu Glu Glu Pro
       290                 295                 300
   Val Asp Glu Thr Met Arg Ser Arg Ala Phe Gln Glu Ser Ile Arg Leu
   305                 310                 315                 320
   Met Phe Arg Thr Glu Asn Ser Glu Tyr Asn Asn Ile Gln Ala Ile Gly
                   325                 330                 335
   Ser Gly Arg Thr Gln Arg Gln Lys Phe Tyr Ala Leu Leu Ile Ile Ala
               340                 345                 350
   Ala Thr Asp Ser Phe Arg Trp Arg Ile Trp Trp Asn Asn Pro Tyr Pro
           355                 360                 365
   Cys Leu Arg Gly Cys Leu Ile Ala Cys Glu Met Glu Leu Gly Asp Val
       370                 375                 380
   Tyr Lys Ser Leu Lys Ser Ile Tyr Lys Trp Thr Leu Arg Ser Ser Tyr
   385                 390                 395                 400
   Ser Ser Gln Arg Glu Ile Asp Arg Gln Leu Asn Val Phe Pro Tyr Gln
                   405                 410                 415
   Lys Ile Asn Leu Phe Asp Phe Ser Gly Thr Pro Gly Thr Glu Ile Ile
               420                 425                 430
   His Trp Arg Ile Ala His Ile Pro Val Pro Asp Glu Ile Asp Tyr Glu
           435                 440                 445
   His Gly Tyr Leu Cys Pro Glu Ser Gly Asp Tyr Asp Ile Val Met Ala
       450                 455                 460
   Val Asp Asp Asp Leu Tyr Ser Thr Phe Lys His Arg Val Ile Xaa Arg
   465                 470                 475                 480
   Gly Trp Glu His Gln Ala Met Lys Leu Asp Glu Leu Val Pro Gln Glu
   485 490 495
   Asn Asn Ile Phe Lys Met Glu Phe Glu Lys Asp Ala His Leu Asp Asn
   500 505 510
   Arg Asn His Leu Val Leu Pro Pro Tyr Tyr Asn Lys Val Val Tyr Cys
   515 520 525
   Pro Leu Phe His Ala Thr Ala Lys Ile Thr Pro Thr Glu Ile Ser His
   530 535 540
   Arg Gln Ser Asp Asp Pro Trp Cys Xaa Arg Thr Leu Tyr Gly Phe Arg
   545 550 555 560
   Gly Asp His Val Met Thr His Tyr Thr Gly Leu Asn His Ile Met Asn
   565 570 575
   Arg Arg Lys Pro Leu Met Gly Gln Thr Leu Ser Val Ala Gln Ser Met
   580 585 590
   Gly Asp Tyr Thr Lys Tyr Ile Leu Gly Asp Gly Trp Ser Ala Asn Ser
   595 600 605
   Cys Thr Gly Thr Ala Tyr Ser Ser Arg Leu Pro Thr Met Leu Phe Gln
   610 615 620
   Asp Ile Phe Asp Ala Ile Ile Arg His Leu Pro Lys Xaa Ile Leu Lys
   625 630 635 640
   Asp Gln Ala Asp Tyr Phe Ser Glu Ser Arg Lys Arg Val Gly Asp Ile
   645 650 655
   Ile Asp Trp Asp Thr Phe Ile Val Tyr Ser Ile Tyr Ala Cys Phe Glu
   660 665 670
   Arg Lys Asn Xaa Val Leu Arg Glu Glu Asp Ala Thr Gln Ile Val Arg
   675 680 685
   Gly Ile Ser Thr Ala Gly Lys Glu Lys Arg Leu Lys Leu Ile Glu Arg
   690 695 700
   Ser Phe Pro Ile Tyr Tyr Arg Glu Leu Met Lys Ile Lys Asn Ala Lys
   705 710 715 720
   Thr Trp Lys Asp Ala Tyr Ala Phe Asn Phe Leu Pro Leu Leu Leu Cys
   725 730 735
   Ala Gly Thr Asn Val Gln Tyr Val His Arg Gln Trp Ser Tyr Pro Val
               740                 745                 750
   Leu Val Ser Met Glu Lys Gly Pro Arg Ile Ile Pro Ala Met Val Gly
           755                 760                 765
   Arg Asp Leu Ala Asp Gly Asn Leu Gly Asp Trp His Ala Tyr Ile Lys
       770                 775                 780
   Tyr Tyr Met Gly Gln His Met Glu Asp Gln Glu Val Glu Glu Asp Ser
   785                 790                 795                 800
   Leu Glu Ile Leu Lys Ile Ser Leu Glu His Tyr Met Ser His Ser Tyr
                   805                 810                 815
   Ile Ser Asn Val Lys Asp Val Ala Val Arg Met Thr Lys Glu Ile Val
               820                 825                 830
   Leu Asp Leu Tyr Phe Ala Ser Gly Cys Asn Gly Val Ser Glu Met Met
           835                 840                 845
   Thr Phe Leu Leu Pro Ile Ile His Pro Arg Lys Gly Leu Val Met Phe
       850                 855                 860
   Cys Ile Ala Asp Asp Gln Val Gln Pro Gln Val Gln Cys Glu Thr Ala
   865                 870                 875                 880
   Leu Arg Arg Phe Pro Tyr Thr Arg Glu Tyr Ile His Asp Val Ile Val
                   885                 890                 895
   Ile Gln Val Gly Arg Pro Gly Glu Leu Lys Gln Val Trp Lys Arg Gly
               900                 905                 910
   Ser Thr Lys Val Lys Val Cys Arg Arg Asn Phe Leu Arg Tyr Asp His
           915                 920                 925
   Lys Val Ile Leu Ser Arg Leu Cys Gly Leu Val Tyr Gly Asn Tyr Glu
       930                 935                 940
   Leu Met Thr Lys Leu Thr Asn Ile
   945                 950
<210> 20
   <211> 959
   <212> PRT
   <213> Artificial sequence
<220>
   <223> BTV-16 VP2 consensus sequence
<220>
   <221> misc_feature
   <222> (32)..(32)
   <223> Xaa can be any naturally occurring amino acid
<220>
   <221> misc_feature
   <222> (50)..(50)
   <223> Xaa can be any naturally occurring amino acid
<220>
   <221> misc_feature
   <222> (67)..(67)
   <223> Xaa can be any naturally occurring amino acid
<220>
   <221> misc_feature
   <222> (72)..(72)
   <223> Xaa can be any naturally occurring amino acid
<220>
   <221> misc_feature
   <222> (76)..(76)
   <223> Xaa can be any naturally occurring amino acid
<220>
   <221> misc_feature
   <222> (84)..(84)
   <223> Xaa can be any naturally occurring amino acid
<220>
   <221> misc_feature
   <222> (190)..(190)
   <223> Xaa can be any naturally occurring amino acid
<220>
   <221> misc_feature
   <222> (216)..(216)
   <223> Xaa can be any naturally occurring amino acid
<220>
   <221> misc_feature
   <222> (470)..(470)
   <223> Xaa can be any naturally occurring amino acid
<220>
   <221> misc_feature
   <222> (477)..(477)
   <223> Xaa can be any naturally occurring amino acid
<220>
   <221> misc_feature
   <222> (516)..(516)
   <223> Xaa can be any naturally occurring amino acid
<220>
   <221> misc_feature
   <222> (547)..(547)
   <223> Xaa can be any naturally occurring amino acid
<400> 20
   Met Glu Glu Leu Val Ile Pro Val Ile Thr Arg Gln Phe Asp Lys Lys
   1               5                   10                  15
   Leu Val Gly Arg Tyr Asp Tyr Val Ile Glu Leu Thr Glu Pro Glu Xaa
               20                  25                  30
   Gly Glu Trp Ser Gly His Asp Val Thr Glu Ile Pro Asn Arg Arg Met
           35                  40                  45
   Phe Xaa Ile Lys Gln Gln Asn Ile Arg Glu Ala Ile Glu Tyr Lys Pro
       50                  55                  60
   Val Asp Xaa Asp Gly Glu Val Xaa Pro Arg Ile Xaa Asp Met Ser Ile
   65                  70                  75                  80
   Ala Cys Tyr Xaa Met Lys Lys Ser Met Met Lys Lys Asp Gly Val Asp
                   85                  90                  95
   Phe Val Ser Asn Thr Lys Trp Leu Glu Trp Met Ile Gly Asp Ser Met
               100                 105                 110
   Asp Val Gln Pro Leu Arg Val Gln Leu Lys Glu Asp His Ser Thr Ile
           115                 120                 125
   Gln Tyr Gly Met Phe Ser Asn Thr Leu His Ile Asp Ser Arg Lys Ala
       130                 135                 140
   Asp Thr Thr Ser Tyr His Thr Ile Ala Val Glu Ser Lys Gly Glu Arg
   145                 150                 155                 160
   Gly Cys Cys His Val His Thr Ala Ile Trp Asn His Met Val Arg Asn
                   165                 170                 175
   His Leu Phe Asn Ala Val Gln Glu Ala Cys Tyr Val Phe Xaa Pro Thr
               180                 185                 190
   Tyr Asp Leu Ile Val Ile Gly Glu Lys Gln Asn Arg Glu Asp Glu Phe
           195                 200                 205
   Arg Ile Gly Glu His Asn Phe Xaa Thr Ile Thr Arg Asn His His Met
       210                 215                 220
   Arg Leu Gly Asp Asn Ala Tyr Asn Gln Phe Met Lys Gly Leu Val Gln
   225                 230                 235                 240
   Leu Arg Val Ala Gly Val Thr Pro Asn Ala Ile Arg Glu Glu Met Ala
   245 250 255
   Ala Leu Asp Ala Ile Arg Asp Thr Trp Ile Gly Gly Asn Phe Glu Arg
   260 265 270
   Thr His Ile Lys Ser Leu Glu Ile Cys Lys Leu Leu Ser Ser Ile Gly
   275 280 285
   Arg Lys Met Val Asn Met Glu Glu Glu Pro Lys Asp Glu Arg Asp Leu
   290 295 300
   Ser Val Lys Phe Gln Phe Lys Leu Asp Asp Lys Phe Ser Thr Thr Asp
   305 310 315 320
   Pro Glu Arg Asn Val Ile Phe Thr His Lys Thr His Arg Thr Asn Gln
   325 330 335
   Asp Arg Phe Tyr Val Leu Leu Met Ile Ala Ala Ser Asp Thr Asn Asn
   340 345 350
   Gly Arg Val Trp Trp Ser Asn Pro Tyr Pro Cys Leu Arg Gly Ala Leu
   355 360 365
   Ile Ala Ser Glu Cys Lys Leu Gly Asp Val Tyr His Lys Leu Arg Ala
   370 375 380
   Trp Tyr Glu Trp Ser Val Arg Pro Glu Tyr Lys Pro Arg Asp Leu Glu
   385 390 395 400
   Arg Glu Gln Glu Lys Tyr Ile Val Gly Arg Val Asn Leu Phe Asp Leu
   405 410 415
   Glu Gly Glu Pro Ala Thr Lys Val Phe His Trp Glu Tyr Glu Leu Ile
   420 425 430
   Asn Lys Val Tyr Gln Ile Thr Asn His Thr Gly Asn His Cys Asp Leu
   435 440 445
   Tyr Pro Asp Asp Val Glu Ile Thr Ala Lys Phe Asp Glu Glu Lys Tyr
   450 455 460
   Gly Glu Met Ile Gln Xaa Ile Ile Asn Glu Gly Trp Xaa His Gly Asp
   465 470 475 480
   Phe Lys Met Phe Lys Ile Leu Lys Glu Glu Gly Asn Pro Leu Leu Tyr
   485 490 495
   Asp Leu Glu Lys Asp Ile Arg Leu Asp Ser Arg Ser Gln Val Ile Phe
   500 505 510
   Pro Pro Tyr Xaa Asn Lys Trp Thr His Ala Pro Met Phe Asn Ala Lys
   515 520 525
   Val Lys Pro Cys Glu Val Glu Leu Ala Gln Arg Lys Asn Glu Asp Pro
   530 535 540
   Tyr Val Xaa Arg Thr Val Lys Pro Ile Arg Ala Asp Cys Ile Asp Leu
   545 550 555 560
   Leu Arg Tyr His Met Ser His Tyr Met Asp Val Arg Val Ser Met Lys
   565 570 575
   Gly Leu Ser Leu Ala Val Lys Gln Thr Pro Ser Ser Ile His Gln Glu
   580 585 590
   Leu Ala Lys Asp Pro Leu Tyr Pro Gly Phe Leu Gln Arg Arg Asp Glu
   595 600 605
   Asn Leu Asp His Lys Ser Val Cys Pro Ile Val Thr Asn Tyr Phe Leu
   610 615 620
   Leu Glu Lys Phe Tyr Thr Leu Val Leu Thr Ile Met Glu Lys His Tyr
   625 630 635 640
   Trp Asp Leu Asp Asp Ser Asp Val Thr Tyr Glu Phe Pro Thr Leu Asp
   645 650 655
   Asp Ser Ala Tyr Lys Thr Glu Gly Thr Leu Tyr Asp Val Ser Gln Val
   660 665 670
   Val Val His Met Ile Asp Arg Phe Phe Glu Lys Arg Arg Phe Leu Arg
   675 680 685
   Ser Val Asp Glu Cys Arg Trp Ile Leu His Leu Ile Arg Ser Thr Gln
   690 695 700
   Ser Arg Asp Arg Leu Ser Val Phe Lys Arg Phe Phe Pro Thr Phe Gly
   705 710 715 720
   Glu Gly Leu Cys Val Asn Asn Phe Arg Lys Val Lys Asp Ile Met Leu
   725 730 735
   Leu Asn Phe Leu Pro Phe Phe Phe Leu Ile Gly Asp Asn Ile Ala Tyr
   740 745 750
   Glu His Arg Gln Trp Ala Val Pro Leu Leu Phe Tyr Ala Asp Lys Ile
           755                 760                 765
   Arg Val Ile Pro Ala Glu Val Gly Ala Tyr Tyr Asn Arg Phe Gly Leu
       770                 775                 780
   Val Ser Ile Leu Glu Leu Leu Thr Phe Phe Pro Ser Tyr Glu Met Arg
   785                 790                 795                 800
   Asp Asn Lys Val Glu Glu Asp Val His Ala Cys Ala Ser Ala Ile Leu
                   805                 810                 815
   Asp Phe Tyr Leu Thr Thr Thr Ile Ser Asn Gly Gly Ile Gln Ala Ser
               820                 825                 830
   Ile Val Ser Thr Lys Ala Leu Leu Tyr Glu Thr Tyr Leu Ser Ser Val
           835                 840                 845
   Cys Gly Gly Phe Ser Glu Ala Ile Leu Trp Tyr Leu Pro Ile Thr His
       850                 855                 860
   Pro Val Lys Cys Leu Val Ala Leu Glu Val Ser Asp Ser Leu Val Asp
   865                 870                 875                 880
   Ser Asn Val Arg Ile Asp Lys Ile Lys Arg Arg Phe Pro Leu Ser His
                   885                 890                 895
   Arg His Leu Arg Gly Ile Val Gln Ile Ser Val Arg Pro Asp Arg Thr
               900                 905                 910
   Phe Gly Val Thr Thr Cys Gly Ile Val Lys His Lys Ile Cys Lys Lys
           915                 920                 925
   Thr Leu Leu Lys Gln Arg Cys Asp Val Ile Leu Ile Gln Ile Pro Gly
       930                 935                 940
   Tyr Val Phe Gly Asn Asp Glu Leu Leu Thr Lys Leu Leu Asn Ile
   945                 950                 955
<210> 21
   <211> 955
   <212> PRT
   <213> Artificial sequence
<220>
   <223> BTV-17 VP2 consensus sequence
<220>
   <221> misc_feature
   <222> (54)..(54)
   <223> Xaa can be any naturally occurring amino acid
<220>
   <221> misc_feature
   <222> (155)..(155)
   <223> Xaa can be any naturally occurring amino acid
<220>
   <221> misc_feature
   <222> (342)..(342)
   <223> Xaa can be any naturally occurring amino acid
<220>
   <221> misc_feature
   <222> (669)..(669)
   <223> Xaa can be any naturally occurring amino acid
<220>
   <221> misc_feature
   <222> (801)..(801)
   <223> Xaa can be any naturally occurring amino acid
<400> 21
   Met Glu Glu Phe Val Ile Pro Val Tyr Ser Glu Asp Glu Ile Pro Tyr
   1               5                   10                  15
   Ala Leu Leu Ser Arg Tyr Pro Leu Ala Ile Gln Thr Asn Val Lys Ile
               20                  25                  30
   Glu Asp Val Glu Gly Lys His Asn Val Val Lys Ile Pro Glu Ser Asp
           35                  40                  45
   Met Ile Asp Ile Pro Xaa Leu Thr Ile Val Glu Ala Met Asn Tyr Lys
       50                  55                  60
   Pro Ala Arg Asn Asp Gly Ile Val Val Pro Arg Leu Leu Asp Ile Thr
   65                  70                  75                  80
   Leu Arg Ala Tyr Asp Asp Arg Lys Ser Thr Lys Ser Ala Arg Gly Ile
                   85                  90                  95
   Glu Phe Met Thr Asn Ala Arg Trp Met Lys Trp Ala Ile Asp Asp Arg
               100                 105                 110
   Met Asp Ile Gln Pro Leu Lys Val Thr Leu Asp His Tyr Cys Ser Val
           115                 120                 125
   Asn His Gln Leu Phe Asn Cys Val Val Lys Ala Asn Ala Ala Asn Ala
       130                 135                 140
   Asp Thr Ile Tyr Tyr Asp Tyr Phe Pro Leu Xaa Asp His Lys Lys Arg
   145 150 155 160
   Cys Asn His Thr Asn Leu Asp Leu Leu Arg Ser Leu Thr Asn Met Glu
   165 170 175
   Leu Phe His Ala Leu Gln Gly Ala Ala Tyr Ser Ile Lys Ser Ser Tyr
   180 185 190
   Glu Leu Val Ala Asn Ser Glu Arg Glu Ser Leu Glu Glu Thr Tyr Ala
   195 200 205
   Val Gly Gln Pro Lys Trp Ile His Leu Thr Arg Gly Thr Arg Ile Gly
   210 215 220
   Asn Ser Gly Leu Ser Tyr Glu Arg Phe Ile Ser Ser Met Val Gln Val
   225 230 235 240
   Ile Val Asn Gly Lys Ile Pro Asn Glu Ile Ala Asn Glu Ile Ala Gln
   245 250 255
   Leu Asn Arg Ile Arg Ala Glu Trp Ile Ala Ala Thr Tyr Asp Arg Gly
   260 265 270
   Arg Ile Arg Ala Leu Glu Leu Cys Asn Ile Leu Ser Thr Ile Gly Arg
   275 280 285
   Lys Met Leu Asn Thr His Glu Glu Pro Lys Asp Glu Met Asp Leu Ser
   290 295 300
   Thr Arg Phe Gln Phe Lys Leu Asp Glu Lys Phe Asn Arg Ala Asp Ser
   305 310 315 320
   Glu His Val Asn Ile Phe Gly Val Arg Ala Pro Ala Thr Asp Glu Gly
   325 330 335
   Arg Phe Tyr Ala Leu Xaa Ala Ile Ala Ala Thr Asp Thr Gln Lys Gly
   340 345 350
   Arg Val Trp Arg Thr Asn Pro Tyr Pro Cys Leu Arg Gly Ala Leu Val
   355 360 365
   Ala Ala Glu Cys Glu Leu Gly Asp Val Tyr Ser Thr Leu Arg Arg Val
   370 375 380
   Tyr Arg Trp Ser Leu Arg Pro Glu Tyr Gly Gln His Glu Arg Gln Leu
   385 390 395 400
   Glu Asn Asn Lys Tyr Val Phe Asn Arg Ile Asn Leu Phe Asp Ser Asn
   405 410 415
   Leu Ala Val Gly Asp Gln Ile Ile His Trp Arg Tyr Glu Val Lys Ala
   420 425 430
   Ser Ala Glu Thr Thr Tyr Asp Ser Gly Tyr Met Cys Arg His Glu Ala
   435 440 445
   Glu Glu Asp Glu Leu Leu Cys Lys Ile Asn Glu Asp Lys Tyr Lys Glu
   450 455 460
   Met Leu Asp Arg Met Ile Gln Gly Gly Trp Asp Gln Glu Arg Phe Lys
   465 470 475 480
   Leu His Asn Ile Leu Thr Asp Pro Asn Leu Leu Thr Ile Asp Phe Glu
   485 490 495
   Lys Asp Ala Tyr Leu Asn Ser Arg Ser Glu Leu Val Leu Pro Asp Tyr
   500 505 510
   Phe Asp Lys Trp Ile Ser Ser Pro Met Phe Asn Ala Arg Leu Arg Ile
   515 520 525
   Thr Lys Gly Glu Ile Gly Thr Ser Lys Lys Asp Asp Pro Trp Asn Asn
   530 535 540
   Arg Ala Val Arg Gly Tyr Ile Lys Ser Pro Ala Glu Ser Leu Asp Phe
   545 550 555 560
   Val Leu Gly Pro Tyr Tyr Asp Leu Arg Leu Leu Phe Phe Gly Glu Thr
   565 570 575
   Leu Ser Leu Lys Gln Glu Gln Ser Ala Val Phe Gln Tyr Leu Ser Gln
   580 585 590
   Leu Asp Asp Phe Pro Ala Leu Thr Gln Leu Thr Gly Asp Ala Val Cys
   595 600 605
   Pro His Ser Gly Gly Ala Leu Tyr Thr Phe Arg Lys Val Ala Leu Phe
   610 615 620
   Leu Ile Gly Asn Tyr Glu Lys Leu Ser Pro Asp Leu His Glu Gly Met
   625 630 635 640
   Glu His Gln Thr Tyr Val His Pro Ser Thr Gly Gly Thr Tyr Gln Lys
   645 650 655
   Arg Val Leu Glu Met Lys Asp Pro Cys Gln Leu Met Xaa Phe Val Ile
               660                 665                 670
   Asp Tyr Ile Phe Glu Lys Arg Glu Gln Leu Arg Asp Thr Lys Glu Ala
           675                 680                 685
   Arg Tyr Ile Val Tyr Leu Ile Gln Ser Leu Thr Gly Thr Gln Arg Leu
       690                 695                 700
   Asp Val Leu Arg Ser Thr Phe Pro Asn Phe Phe Gln Arg Leu Leu Met
   705                 710                 715                 720
   Leu Lys Glu Ile Lys Phe Val Arg Asp Leu Asn Val Ile Asn Phe Leu
                   725                 730                 735
   Pro Leu Met Phe Leu Val His Asp Asn Ile Ser Tyr Trp His Arg Gln
               740                 745                 750
   Trp Ser Ile Pro Met Val Leu Phe Asp Asp Thr Ile Lys Leu Ile Pro
           755                 760                 765
   Val Glu Val Gly Ala Tyr Ala Asn Arg Phe Gly Phe Lys Ser Phe Met
       770                 775                 780
   Asn Phe Thr Arg Phe His Pro Gly Glu Ser Lys Lys Lys Gln Ile Ala
   785                 790                 795                 800
   Xaa Asp Met His Lys Glu Phe Gly Val Val Ala Phe Glu Tyr Tyr Thr
                   805                 810                 815
   Asn Thr Lys Ile Ser Gln Gly Asn Val His Thr Pro Val Met Thr Thr
               820                 825                 830
   Lys Met Asp Val Leu Lys Val His Leu Ser Ser Leu Cys Ala Gly Leu
           835                 840                 845
   Ala Asp Ser Val Val Tyr Thr Leu Pro Val Ala His Pro Lys Lys Cys
       850                 855                 860
   Ile Val Leu Ile Ile Val Gly Asp Asp Lys Leu Glu Pro His Thr Arg
   865                 870                 875                 880
   Ser Glu Gln Ile Val Ser Arg Tyr Asn Tyr Ser Arg Lys His Ile Cys
                   885                 890                 895
   Gly Ile Val Ser Val Thr Ile Gly Gln Asn Ser Gln Leu Arg Val His
               900                 905                 910
   Thr Ser Gly Ile Val Lys His Arg Val Cys Asp Lys Phe Ile Leu Lys
   915 920 925
   His Lys Cys Lys Val Ile Leu Val Arg Met Pro Gly Tyr Val Phe Gly
   930 935 940
   Asn Asp Glu Leu Met Thr Lys Leu Leu Asn Val
   945 950 955
<210> 22
   <211> 957
   <212> PRT
   <213> Artificial sequence
<220>
   <223> BTV-18 VP2 consensus sequence
<400> 22
   Met Asp Glu Leu Ala Ile Pro Ile Tyr Thr Asp Thr Phe Pro Ala Gly
   1 5 10 15
   Leu Leu Asp Gly Tyr Asp Tyr Ile Ile Asp Val Ser Thr Arg Val Asp
   20 25 30
   Glu Pro Lys Gly Arg His Asp Val Thr Glu Ile Pro Lys Arg Ser Met
   35 40 45
   Phe Asp Ile Lys Asp Glu Pro Ile Arg Glu Ala Ile Ile Tyr Lys Pro
   50 55 60
   Val Ser Asn Asp Gly Cys Val Leu Pro Arg Val Leu Asp Ile Thr Leu
   65 70 75 80
   Arg Ala Phe Asp Asp Arg Lys Arg Val Ile Thr Asn Glu Gly His Ser
   85 90 95
   Glu Phe His Thr Arg Ser Asn Trp Leu Gln Trp Met Ile Asp Asp Ala
   100 105 110
   Met Asp Tyr Gln Pro Leu Lys Val Asp Val Glu His Val Pro Ser Arg
   115 120 125
   Ile Ser His Ala Leu Phe Asn Ser Thr Val Arg Leu His Ala Lys Arg
   130 135 140
   Ala Asp Thr Ala Ser Tyr His Ile Glu Pro Val Glu Val Glu Ala Tyr
   145 150 155 160
   Gly Cys Asn His Val Trp Thr Glu Arg Ile His His Leu Val Asn Ile
                   165                 170                 175
   Glu Met Phe His Ser Ser Gln Glu Ala Ala Tyr Ile Leu Lys Pro Thr
               180                 185                 190
   Tyr Gln Ile Ile Ala His Ala Glu Arg Ala Ala Val Gly Asp Ala Phe
           195                 200                 205
   Asn Gly Arg Thr Leu Glu Leu Asn Arg Asn His Arg Ile Gln Leu Gly
       210                 215                 220
   Asp Gln Gly Tyr Val Lys Leu Lys Lys Gly Leu Val Gln Thr Arg Ile
   225                 230                 235                 240
   Glu Gly Lys Lys Pro Ala Ala Ile Glu Arg Glu Ile Ala Ser Leu Asn
                   245                 250                 255
   Glu Ile Arg Ala Gln Trp Ile Ala Arg Gly His Asp Arg Arg Glu Ile
               260                 265                 270
   Lys Ala Leu Asp Leu Cys Arg Ile Leu Ser Ala Ile Gly Arg Lys Met
           275                 280                 285
   Tyr Asn Ile Glu Glu Glu Pro Lys Asn Glu Ala Asp Met Ser Val Lys
       290                 295                 300
   Phe Gln Met Glu Leu Asp Glu Ile Phe Lys Pro Gly Asn Ser Glu His
   305                 310                 315                 320
   Asp Asn Ile Met Gly Gly Gly Thr His Arg Lys Asp Glu Asp Arg Phe
                   325                 330                 335
   Tyr Val Leu Ile Val Ile Ala Ala Ser Asp Thr Asn Gln Gly Arg Val
               340                 345                 350
   Trp Trp Ser Asn Pro Tyr Pro Cys Leu Arg Gly Ala Leu Ile Ala Ala
           355                 360                 365
   Glu Val Gln Leu Gly Asp Val Tyr Ser Thr Leu Arg Ser Trp Tyr Gln
       370                 375                 380
   Trp Ser Ile Arg Pro Ala Tyr Val Pro Tyr Asp Arg Ser Arg Glu Asn
   385                 390                 395                 400
   Asp Lys Tyr Ile Tyr Thr Arg Thr Asn Leu Phe Asp Ser Ala Leu Gly
                   405                 410                 415
   Pro Gly Asp Lys Ile Ile His Trp Glu Tyr Gln Leu Leu Asn Glu Thr
               420                 425                 430
   Arg Glu Val Ser Met Ser Ser Gly Asn Glu Cys Asp Leu Phe Pro Glu
           435                 440                 445
   Asp Glu Glu Phe Thr Thr Lys Phe Asn Glu Glu Arg Tyr Thr Glu Met
       450                 455                 460
   Lys Gly Gln Ile Ile Gln Ser Gly Trp Asn His Arg Asp Phe Lys Met
   465                 470                 475                 480
   His Lys Ile Leu Glu Asp Gly Ala Asn Val Leu Thr Ile Asp Phe Glu
                   485                 490                 495
   Lys Asp Ala Arg Val Gly Thr Gly Ser Val Leu Thr Leu Pro Asp Tyr
               500                 505                 510
   Tyr Asp Lys Trp Ile Val Ala Pro Met Phe Asn Ala Lys Leu Arg Ile
           515                 520                 525
   Thr Glu Thr Val Ile Ala Asn Ala His Ser Asp Asp Pro Ala Ile Glu
       530                 535                 540
   Arg Ser Val Lys Pro Phe Asp His Asp Pro Ser Thr Leu Gln Arg Phe
   545                 550                 555                 560
   Cys Leu Ala Arg Tyr Tyr Asp Ile Arg Pro Gly Leu Arg Gly Arg Ala
                   565                 570                 575
   Leu Leu Lys Gln Gln Asp Gly Ser Thr Ile Thr Lys Lys Val Ser Ala
               580                 585                 590
   Leu Asp Asp Tyr Gln Asp Val Val Ser Arg Arg Val Asn Tyr Val Gln
           595                 600                 605
   Arg Asp Val Arg Cys Pro Thr Glu Thr Ala Gln Tyr Val Leu Glu Lys
       610                 615                 620
   Thr Ser Leu Tyr Leu Leu Ala Leu Phe Asp Lys His Val Met Pro Ser
   625                 630                 635                 640
   Ala Glu Ser Glu Ala Lys Phe Val His Pro Ser Ile Asp Val Lys His
                   645                 650                 655
   Asp Ile Glu Thr Trp Arg Ile Ala Asp Ile Ser Gln Leu Ile Val Leu
               660                 665                 670
   Ile Phe Asp Tyr Leu Phe Glu Ser Arg Lys Val Val Arg Asp Ala Val
           675                 680                 685
   Glu Ala Arg Trp Ile Leu Tyr Lys Ile Arg Ser Ser Thr Gly Arg Ala
       690                 695                 700
   Arg Met Glu Val Ile Glu Thr Thr Phe Pro Arg Phe Gly Leu Thr Val
   705                 710                 715                 720
   Lys Asn Ile Thr Lys Ala Lys Val Asn Gln Asp Ile Ala Cys Leu Asn
                   725                 730                 735
   Phe Phe Pro Leu Phe Phe Ile Val Gly Asp Asn Met Ala Tyr Ala His
               740                 745                 750
   Arg Gln Trp Ser Ile Pro Ile Leu Leu Tyr Ala His Asp Ile Arg Ile
           755                 760                 765
   Ile Pro Leu Glu Val Gly Ala Tyr Ser Asn Arg Phe Gly Leu Thr Ser
       770                 775                 780
   Tyr Leu Glu Tyr Met Ile Phe Phe Pro Ser Tyr Ala Thr Arg Met Ala
   785                 790                 795                 800
   Met Ile Asp Glu Ala Asn Lys Glu Cys Ala Ala Ala Leu Leu Asp Tyr
                   805                 810                 815
   Tyr Leu Ser Thr Asp Val His Arg Gly Gly Val Met Thr Ser Val Ile
               820                 825                 830
   Thr Thr Lys Arg Leu Leu Tyr Glu Thr Tyr Leu Ser Ser Leu Cys Gly
           835                 840                 845
   Gly Tyr Ser Asp Gly Leu Leu Trp Tyr Ile Pro Ile Thr His Pro Leu
       850                 855                 860
   Lys Cys Leu Val Ala Phe Glu Val Ala Asp Asp Val Val Pro Ala Thr
   865                 870                 875                 880
   Val Arg Lys Glu Arg Ile Leu Met Arg Phe Pro Leu Ser Ala Arg His
                   885                 890                 895
   Leu Arg Gly Ile Ala Leu Ile Thr Ile Asp Arg Asp Arg Lys Val His
               900                 905                 910
   Val Gln Thr Glu Gly Ile Val Ser His Arg Leu Cys Lys Lys Asn Leu
   915 920 925
   Leu Lys Tyr Val Cys Asp Val Ile Leu Phe Lys Phe Ser Gly Tyr Val
   930 935 940
   Phe Gly Asn Asp Glu Met Leu Thr Lys Leu Leu Asn Val
   945 950 955
<210> 23
   <211> 959
   <212> PRT
   <213> Artificial sequence
<220>
   <223> BTV-19 VP2 consensus sequence
<400> 23
   Met Asp Glu Phe Ala Val Pro Ile Val Arg Gly Arg Thr Ile Pro Tyr
   1               5                   10                  15
   Asp Leu Ile Arg Ala Tyr Glu Thr Ile Ile Asp Leu Asp Val Lys Val
               20                  25                  30
   Glu Glu Asn Glu Gly Lys His Asp Ile Thr Lys Ile Pro Glu Ser Tyr
           35                  40                  45
   Met Cys Asp Val Pro Asp Ile Pro Ile Gln Asp Ala Thr Thr Ala Thr
       50                  55                  60
   Leu Ser Arg Asn Asp Gly Tyr Ala Leu Pro Arg Ile Leu Asp Val Ser
   65                  70                  75                  80
   Ile Ala Ala Tyr Asp Thr Arg Lys Gly Tyr Gln Lys Tyr Asn Gly Ile
                   85                  90                  95
   Glu Arg Met Thr Asn Gln Val Trp Leu Lys Trp Ala Ile Lys Asp Phe
               100                 105                 110
   Met Asp Val Gln Pro Leu Lys Val Ser Leu Thr Asp Ala Glu Gly Lys
           115                 120                 125
   Val Arg Tyr Asp Leu Phe Asn Cys Ser Val Arg Met Asp Ala Lys Lys
       130                 135                 140
   Ala Glu Ala Val Tyr Tyr Gly Trp Ala Ala Gln Glu Val Ile Asp Lys
   145                 150                 155                 160
   Gly Cys Asn His Ala Arg Ser Glu Leu Leu Gln His Val Ile Arg Cys
   165 170 175
   Glu Leu Leu Asn Cys Ala Gln Glu Ser Ala Tyr Met Leu Lys Pro Thr
   180 185 190
   Gln Lys Leu Glu Pro Val Ala Glu Lys Thr Ser Leu Ser Glu Arg Leu
   195 200 205
   Leu Pro Gly Ser Ser His Leu Thr Ser Met Arg Arg Gly Glu Val Phe
   210 215 220
   Arg Met Gly Thr Glu Gln Tyr Asn Arg Met Val Gln Gly Trp Tyr Arg
   225 230 235 240
   Val Lys Val Lys Gly His Thr Pro Glu Lys Ile Gln Asp Glu Met Asn
   245 250 255
   Glu Leu Met Lys Ile Arg Asp Arg Trp Lys Val Ala Arg Asp Arg Arg
   260 265 270
   Glu Ile Lys Ser Leu Glu Leu Cys Lys Leu Leu Ser Ser Val Gly Arg
   275 280 285
   Lys Met Tyr Asp Lys Tyr Glu Glu Pro Lys Asp Glu Glu Ala Met Ser
   290 295 300
   Leu Arg Phe Gln Trp Asn Leu Asp Glu Ile Phe Lys Thr Asp Asn Arg
   305 310 315 320
   Glu His Ile Asn Ile Ala Val Glu Lys Ser Gly Arg Thr Asn Asp Glu
   325 330 335
   Arg Phe Phe Ala Leu Ile Met Ile Ala Ala Thr Asp Thr Asn Arg Gly
   340 345 350
   Arg Ile Trp Trp Ser Asn Met Phe Pro Cys Leu Arg Gly Ala Leu Ile
   355 360 365
   Val Gly Glu Thr Gln Phe Gly Asp Val Tyr Lys Met Leu Arg Val Arg
   370 375 380
   Phe Asp Trp Ser Val Arg Asn Glu Tyr Ser Thr Gln Ala Asp Val Ala
   385 390 395 400
   Arg Gln Asn Asp Asn Tyr Thr Phe Thr Arg Val Asn Leu Phe Asp Leu
   405 410 415
   Ile Ser Glu Pro Ala Thr Pro Val Ile His Trp Glu Tyr Asp Leu Asp
               420                 425                 430
   Ala Pro Arg Lys Thr Asn Tyr Leu Asp Gly His Ala Cys Asp Glu Phe
           435                 440                 445
   Pro Asp Asp Tyr Asp Leu Val Cys Ser Phe Asp Glu Thr Lys Tyr Ser
       450                 455                 460
   Glu Met Ile Gln Arg Ile Leu Asp Gly Gly Trp Asp His Lys Asn Phe
   465                 470                 475                 480
   Lys Tyr Tyr Lys Ile Leu Lys Asp Ala Gly Asn Val Leu Thr Ile Asp
                   485                 490                 495
   Phe Glu Lys Asp Ala Arg Leu Asn Glu His Ser Glu Met Val Met Pro
               500                 505                 510
   Ser Tyr Phe Asn Lys Arg Ile Ala Ala Pro Met Phe Arg Cys Ile Leu
           515                 520                 525
   Arg Ile Cys Glu Thr Thr Ile Ser Val Arg Gln Asn Ala Asp Pro Ala
       530                 535                 540
   Val Ser Arg Thr Ile Arg Pro Leu Lys Ala Lys Ala Ala Asp Leu Leu
   545                 550                 555                 560
   Asp Phe Thr Leu Gly Ser Tyr Tyr Asp Thr Arg Phe Gly Val Met Gly
                   565                 570                 575
   Arg Thr Leu Val Met Ser Gln Lys Gln Ser Glu Met Tyr Lys Met Ile
               580                 585                 590
   Gln Glu Ala Glu Asp Tyr Ser Glu Leu Met Lys Ser Arg Gly Val Ile
           595                 600                 605
   Glu Lys Phe Lys Asn Pro Cys Pro Ile Thr Thr Ala Cys Gln Tyr Thr
       610                 615                 620
   Leu Gln Lys Thr Ser Val Leu Ile Leu Ser Arg Ala Gln Arg Tyr Phe
   625                 630                 635                 640
   Arg Glu Phe His Gly Gly Asp Gln Glu Glu Tyr Met Ser Pro Thr Leu
                   645                 650                 655
   Asn Phe Glu Trp Ser Asp Met Thr Ser Asn Ile Tyr Asp Val Ala Gln
               660                 665                 670
   Met Val Val Leu Val Phe Asp Val Leu Phe Glu Arg Arg Arg Val Val
   675 680 685
   Arg Gly Val Asp Glu Ser Arg Phe Ile Leu His Gln Leu Arg Ile Arg
   690 695 700
   Lys Gly Leu Asn Arg Leu Asp Tyr Val Lys Ile His Phe Pro His Leu
   705 710 715 720
   Tyr Gln Trp Trp Lys Ala Ser Glu Ser Thr Tyr Gly Glu Ala Gln Lys
   725 730 735
   Leu Asn Leu Leu Pro Leu Leu Phe Leu Val Gly Asp Asn Phe Ile Tyr
   740 745 750
   Glu His Arg Gln Trp Ala Tyr Pro Ala Val Leu Ile Ser His Lys Leu
   755 760 765
   Arg Phe Ile Pro Val Glu Leu Gly Ala Tyr Tyr Asn Arg Phe Gly Leu
   770 775 780
   Thr Ser Leu Ile Ser His Leu Thr Phe His Val Gly Lys Ala Lys Tyr
   785 790 795 800
   Asp Gln Asn Leu Asp Gln Ala Glu Gln Lys Val Phe Ser Ala Leu Lys
   805 810 815
   Asp Tyr Tyr Ala Glu Ser Thr Ile Thr Asp Gly Gly Val Asn Thr His
   820 825 830
   Ser Thr Met Thr Lys Asp Pro Ile Tyr Gln Val Tyr Leu Ser Thr Ile
   835 840 845
   Cys Gly Gly Leu Leu Asp Ser Leu Val Tyr Leu Met Pro Ile Thr His
   850 855 860
   Pro Ile Lys Ser Ile Val Ala Ile Val Ile Asn Asp Gly Lys Gly Ser
   865 870 875 880
   Ala Ser Asp Cys Val Arg Arg Val Thr Arg Lys Leu Arg Tyr Ile Gln
   885 890 895
   His His Ile Arg Gly Ser Val Val Ile Ser Ile Leu Ser Asp Gly Ser
   900 905 910
   Ile Asn Thr Tyr Ser Glu Gly Ile Ala Lys Ala Thr Leu Cys Asp Lys
   915 920 925
   Ser Val Leu Lys Tyr Ser Tyr Lys Val Val Leu Ile Lys Val Lys Gly
   930 935 940
   His Val Phe Gly His Asp Glu Met Leu Thr Lys Leu Leu Asn Val
   945 950 955
<210> 24
   <211> 956
   <212> PRT
   <213> Artificial sequence
<220>
   <223> BTV-20 VP2 consensus sequence
<220>
   <221> misc_feature
   <222> (192)..(192)
   <223> Xaa can be any naturally occurring amino acid
<400> 24
   Met Glu Glu Phe Val Ile Pro Val Phe Ser Glu Gln Asp Met Pro Tyr
   1 5 10 15
   Ala Leu Ile Asn Gln Tyr Pro Leu Ala Ile Arg Thr Asp Val Lys Ile
   20 25 30
   Pro Asp Val Glu Gly Ile His Asp Leu Ala Lys Ile Pro Glu Ser Asp
   35 40 45
   Met Ile Asp Val Pro Lys Leu Asp Ile Val Ser Ala Leu Asn Tyr Lys
   50 55 60
   Pro Val Arg Asn Asp Gly Ile Val Val Pro Arg Leu Leu Asp Ile Thr
   65 70 75 80
   Leu Lys Ala Tyr Asp Asp Arg Lys Ser Thr Lys Asp Ala Gln Gly Ile
   85 90 95
   Lys Phe Met Thr Asn Ala Gly Trp Met Lys Trp Ala Ile Asp Asp Arg
   100 105 110
   Met Asp Ile Gln Pro Leu Lys Val Lys Leu Asp Gln His Tyr Ser Ile
   115 120 125
   Asp Tyr Gln Leu Phe Asn Cys Val Val Lys Ala Lys Thr Ala Asn Ala
   130 135 140
   Asp Thr Met Tyr Tyr Asp Tyr Phe Pro Ile Glu Asn Lys Thr Lys Lys
   145                 150                 155                 160
   Cys Asn His Thr Asn Leu Asp Leu Leu Arg Ser Leu Thr Ala Val Glu
                   165                 170                 175
   Met Phe His Val Leu Gln Gly Ala Ala Tyr Asn Leu Lys Ala Asn Xaa
               180                 185                 190
   Glu Leu Ile Ala Asn Ser Glu Arg Glu Ser Leu Glu Glu Ser Tyr Pro
           195                 200                 205
   Ile Gly Ser Gln Lys Trp Val His Leu Thr Arg Gly Thr Lys Ile Gly
       210                 215                 220
   Asn Ser Gly Val Pro Tyr Glu Arg Phe Ile Ser Ser Met Val Gln Val
   225                 230                 235                 240
   Val Val Lys Gly Lys Val Pro Val Glu Ile Val Ser Glu Val Val Arg
                   245                 250                 255
   Leu Asn Glu Ile Arg Thr Glu Trp Ile Asn Ala Gly Phe Asp Arg Ser
               260                 265                 270
   Arg Ile Arg Ala Leu Glu Leu Cys Asn Ile Leu Ser Thr Ile Gly Arg
           275                 280                 285
   Lys Met Met Asp Thr Gln Glu Glu Pro Lys Asp Glu Met Asp Leu Ser
       290                 295                 300
   Thr Arg Phe Gln Phe Lys Leu Asp Asp Lys Phe Asn Thr Ala Asp Ser
   305                 310                 315                 320
   Glu Tyr Ile Val Ile Ser Arg Val Gly Gly Ala Ala Thr Asn Glu Gly
                   325                 330                 335
   Arg Phe Tyr Ala Leu Ile Ala Ile Ala Ala Thr Asp Thr Gln Arg Gly
               340                 345                 350
   Arg Val Trp Arg Thr Asn Pro Tyr Pro Cys Leu Arg Gly Ala Leu Ile
           355                 360                 365
   Ala Ala Glu Cys Glu Leu Gly Asp Val Tyr Tyr Ser Leu Arg His Thr
       370                 375                 380
   Tyr Arg Trp Ser Leu Arg Pro Glu Tyr Asp Gln Arg Glu Arg Ser Leu
   385                 390                 395                 400
   Glu Asn Asn Lys Tyr Val Phe Ser Arg Ile Asn Leu Phe Asp Ser Asn
   405 410 415
   Leu Thr Val Gly Asp Lys Val Ile His Trp Arg Tyr Asp Leu Thr Lys
   420 425 430
   Pro Met Asp Thr Thr Phe Asp Asp Gly Tyr Met Cys Val Ser His Glu
   435 440 445
   Ile Asp Gly Glu Leu Leu Cys Arg Ile Asp Asp Asp Arg Tyr Lys Glu
   450 455 460
   Met Leu Glu Arg Met Ile Gln Gly Gly Trp Asp Gln Glu Arg Phe Lys
   465 470 475 480
   Leu His Ser Ile Leu Thr Glu Pro Asn Leu Leu Thr Ile Asp Phe Glu
   485 490 495
   Lys Asp Ala Tyr Leu Asn Ser Arg Ser Glu Leu Val Met Pro Asp Tyr
   500 505 510
   Phe Asp Lys Trp Ile Asn Ser Pro Met Phe Asn Ala Arg Leu Arg Leu
   515 520 525
   Thr His Gly Glu Ile Ala Ser Arg Lys Thr Asp Asp Pro Trp Asn Asn
   530 535 540
   Arg Val Val Phe Gly Tyr Ile Lys Thr Thr Thr Glu Ser Leu Asp Tyr
   545 550 555 560
   Met Leu Gly Arg Tyr Tyr Asp Val Arg Leu Gln Phe Tyr Gly Asp Thr
   565 570 575
   Leu Ser Gln Gly Gln Arg Gln Ser Ser Val Phe Ala Tyr Ile Ser Gln
   580 585 590
   Arg Asp Asp Phe Gln Thr Leu Thr His Tyr Val Lys Asp Asp Ala Ile
   595 600 605
   Cys Pro His Ala Gly Gly Ser Ile Tyr Thr Phe Arg Lys Thr Ala Leu
   610 615 620
   Met Leu Met Ala Asn Tyr Glu Lys Leu Ser Pro Glu Leu His Glu Gly
   625 630 635 640
   Leu Glu His Gln Thr Tyr Thr His Pro Ser Asp Gly Met Asp Tyr Arg
   645 650 655
   Lys Tyr Val Val Asp Met Lys Asp Leu Ser Gln Leu Ile Cys Phe Val
               660                 665                 670
   Phe Asp Tyr Ile Phe Glu Lys Arg Ala Gln Leu Arg Asp Pro Lys Glu
           675                 680                 685
   Ala Arg Arg Ile Leu Tyr Leu Val Gln Asn Asn Val Gly Ser Gln Arg
       690                 695                 700
   Leu Gly Val Leu Ser Ser Ala Phe Pro Asn Leu Ala Asn Arg Leu Leu
   705                 710                 715                 720
   Lys Leu Arg Glu Ile His Phe Ile Tyr Asp Met Asn Val Ile Asn Phe
                   725                 730                 735
   Leu Pro Leu Leu Phe Leu Val Gln Asp Asn Ile Ser Tyr Trp His Arg
               740                 745                 750
   Gln Trp Ala Ile Pro Met Val Leu Phe Asp Glu Thr Ile Lys Leu Ile
           755                 760                 765
   Pro Val Glu Val Gly Ala Tyr Ala Asn Arg Phe Gly Phe Lys Ser Phe
       770                 775                 780
   Phe Asn Phe Thr Arg Phe His Pro Gly Asp Ser Arg Lys Lys Gln Ile
   785                 790                 795                 800
   Ala Asp Asp Val His Lys Glu Phe Gly Thr Ile Cys Tyr Glu Tyr Tyr
                   805                 810                 815
   Met Asn Thr Arg Met Ser Gln Gly Gly Val Asn Ile Ser Val Val Thr
               820                 825                 830
   Thr Lys Leu Asp Val Leu Lys Ile His Leu Ala Ser Leu Cys Ala Gly
           835                 840                 845
   Leu Ala Asp Ser Ile Val Tyr Thr Leu Pro Val Ala His Pro Arg Lys
       850                 855                 860
   Cys Ile Val Leu Ile Val Val Gly Asp Asp Lys Leu Glu Pro Gln Ile
   865                 870                 875                 880
   Arg Ala Glu Gln Val Leu Ser Lys Tyr Tyr Tyr Ser Arg Lys His Val
                   885                 890                 895
   Leu Gly Val Ile Ser Ile Ser Val Ser Gln Asp Gly Lys Leu Lys Val
               900                 905                 910
   Tyr Ser Ser Gly Ile Val Arg His Arg Leu Cys Glu Lys Ser Ile Leu
   915 920 925
   Lys Tyr Lys Cys Lys Thr Ile Leu Ile Arg Met Pro Gly His Val Phe
   930 935 940
   Gly Asn Asp Glu Leu Met Thr Lys Leu Leu Asn Ile
   945 950 955
<210> 25
   <211> 955
   <212> PRT
   <213> Artificial sequence
<220>
   <223> BTV-21 VP2 consensus sequence
<220>
   <221> misc_feature
   <222> (436)..(436)
   <223> Xaa can be any naturally occurring amino acid
<220>
   <221> misc_feature
   <222> (449)..(449)
   <223> Xaa can be any naturally occurring amino acid
<220>
   <221> misc_feature
   <222> (526)..(526)
   <223> Xaa can be any naturally occurring amino acid
<220>
   <221> misc_feature
   <222> (682)..(682)
   <223> Xaa can be any naturally occurring amino acid
<220>
   <221> misc_feature
   <222> (702)..(702)
   <223> Xaa can be any naturally occurring amino acid
<220>
   <221> misc_feature
   <222> (845)..(845)
   <223> Xaa can be any naturally occurring amino acid
<400> 25
   Met Asp Glu Leu Ala Ile Pro Ile Ile Asp Gly Arg Phe Pro Lys Glu
   1 5 10 15
   Ala Leu Leu Glu Tyr Cys Cys Ile Val Glu Leu Asn Pro Ala Ser Glu
   20 25 30
   Glu Asp Asp Val Asp Val Thr Thr Ile Pro Gln Lys Asp Met Met Asp
           35                  40                  45
   Ile Pro Asn Thr Pro Ile Arg Glu Ala Leu Lys Tyr Leu Pro Gln Arg
       50                  55                  60
   Asn Asp Gly His Val Leu Thr Arg Val Val Asp Ile Ser Leu Ser Gly
   65                  70                  75                  80
   Tyr Asp Arg Arg Lys Tyr His Arg Thr Ser Thr Gly Gly Asp Phe Leu
                   85                  90                  95
   Ser Pro Glu Glu Trp Leu Asn Trp Met Ile Arg Asp Ser Met Asp Val
               100                 105                 110
   Gln Pro Leu Lys Val Ser Leu Glu Leu Ser Ser Lys Ile Lys His Ser
           115                 120                 125
   Leu Phe Ser Ser Asn Val Tyr Val Ser Val Lys Lys Ala Asp Thr Ile
       130                 135                 140
   Ser Tyr His Ile Glu Ala Lys Glu Val Glu Thr Lys Ala Cys Asp His
   145                 150                 155                 160
   Ala Arg Val Ala Met Trp Asn Asn Leu Val Arg His Glu Met Met His
                   165                 170                 175
   Ser Ala Gln Glu Pro Ala Tyr Ile Leu Lys Pro Thr Leu Asp Ile Ile
               180                 185                 190
   Val His Ser Glu Arg Ala Ser Thr Asp Val Pro Phe Glu Leu Val Gly
           195                 200                 205
   Gln His Tyr Val Ser Ile Gly Lys Asn His Arg Ile Val Leu Gly Asp
       210                 215                 220
   Asp Ala Tyr Arg Lys Thr Leu Asp Gly Leu Thr Lys Leu Arg Val Gln
   225                 230                 235                 240
   Gly Arg Ile Pro Glu Leu Ile Arg Asn Glu Ile Gln Gln Leu His Gln
                   245                 250                 255
   Ile Arg Asp Ala Trp Ile Ala Asp Ser Tyr Asn Gln Arg His Ile Arg
               260                 265                 270
   Ser Leu Glu Leu Cys Arg Ile Leu Ser His Ile Gly Arg Lys Met Val
           275                 280                 285
   His Ile Glu Glu Glu Pro Lys Asn Glu Ser Asp Leu Ser Val Lys Phe
       290                 295                 300
   Gln Phe Lys Leu Asp Glu Lys Phe Arg Ile Asn Asp Asp Glu Arg Asn
   305                 310                 315                 320
   Val Ile Phe Thr Ser Lys Gly Gln Arg Asn Asp Gln Gln Arg Phe Phe
                   325                 330                 335
   Val Leu Ile Met Ile Ala Ala Ser Asp Thr Tyr Asn Ser Arg Ile Trp
               340                 345                 350
   Trp Thr Asn Pro Tyr Pro Cys Leu Arg Gly Ala Leu Ile Ala Ser Glu
           355                 360                 365
   Thr Ser Leu Gly Asp Val Tyr Trp Thr Leu Arg His Phe Tyr Asp Trp
       370                 375                 380
   Ser Val Arg Pro Ala Tyr Ser Pro Arg Gln Lys Glu Arg Glu Asp Asp
   385                 390                 395                 400
   Lys Tyr Leu Tyr Gly Arg Val Asn Leu Phe Asp Leu Asp Ala Asp Pro
                   405                 410                 415
   Gly Thr Gln Ile Ile His Trp Glu Tyr Lys Pro Ile Ile Ser Thr Val
               420                 425                 430
   Lys Thr Thr Xaa Glu Lys Gly Asn Met Cys Asp Leu Phe Pro Asp Val
           435                 440                 445
   Xaa Gly Ile Thr Thr Lys Phe Asn Lys Thr Ala Tyr Thr Asp Met Ile
       450                 455                 460
   Ser Glu Leu Leu Asn Gly Gly Trp Asn Thr Arg Glu Phe Lys Met His
   465                 470                 475                 480
   Lys Ile Leu Glu Ser Val Gly Asn Val Leu Thr Ile Asp Phe Glu Lys
                   485                 490                 495
   Asp Thr Lys Leu Asn Ser Arg Ser Glu Phe Ser Leu Pro Tyr Tyr Tyr
               500                 505                 510
   Asn Lys Trp Ile Tyr Ala Pro Met Phe Asn Ala Lys Leu Xaa Ile Thr
           515                 520                 525
   Gln Thr Glu Ile Ala Gln Gly Lys Ala Asp Asp Pro Asn Ile Ser Arg
       530                 535                 540
   Thr Leu Ala Pro Ile Ser Ser Asp Pro Ile Thr Leu Gln Arg Met Cys
   545 550 555 560
   Leu Ala Asn Tyr Tyr Asp Val Arg Pro Ala Met His Gly Arg Ala Leu
   565 570 575
   Ser Lys Lys Gln Glu Gln Ser Thr Tyr His Lys Ser Leu Thr Asn Gln
   580 585 590
   Ala Asp Tyr Gln Glu Ile Leu Lys Arg Arg Gly Tyr Arg Lys Val Glu
   595 600 605
   Asn Pro Cys Pro Leu Ile Thr Cys Ser Tyr Ile Leu Glu Lys Ser Ala
   610 615 620
   Leu Leu Trp Ile Asp Ile Met Glu Lys His Val Gly Asp Ala Pro Glu
   625 630 635 640
   Asn Glu Met Glu Phe Asp Phe Pro Arg Val Asp Thr Ser Thr Leu Ile
   645 650 655
   Gly Thr Glu Arg Ile Val Asp Ile Ser Gln Leu Val Val Leu Ile Ile
   660 665 670
   Asp Trp Leu Tyr Glu Arg Arg Gln Thr Xaa Arg Ser Val Asp Asp Ala
   675 680 685
   Arg Trp Thr Val Gln Val Ile Arg Ser Ala Thr Gly Lys Xaa Arg Leu
   690 695 700
   Glu Ala Ile Ser Arg Glu Phe Pro Asn Phe Gly Glu Ala Val Ser Arg
   705 710 715 720
   Leu Ala Cys Pro Lys Thr Val Lys Asp Val Met Val Ile Asn Phe Leu
   725 730 735
   Pro Phe Leu Phe Leu Leu Gly Asp Asn Met Ala Tyr Glu His Arg Gln
   740 745 750
   Trp Ser Val Pro Ile Ile Leu Tyr Ala Asp Thr Leu Trp Phe Leu Pro
   755 760 765
   Val Glu Val Gly Ala Tyr Tyr Asn Arg Phe Gly Ile Val Ser Phe Leu
   770 775 780
   Glu Tyr Leu Ile Phe Phe Pro Ser Asn His Met Arg Thr Val Ala Leu
   785 790 795 800
   Asp Glu Ala Glu Ser Lys Val Gly Lys Leu Ile Leu Glu Phe Tyr Leu
   805 810 815
   Asn Thr Thr Ile His Ser Gly Gly Val Gln Gln Ser Val Val Ser Thr
   820 825 830
   Lys Gln Ile Leu Tyr Glu Thr Tyr Leu Ser Ser Leu Xaa Gly Gly Phe
   835 840 845
   Thr Glu Gly Leu Val Trp Tyr Leu Pro Ile Thr His Pro Gln Lys Cys
   850 855 860
   Val Val Ala Ile Glu Ile Cys Asp Asp Arg Ile Gly Ala Gly Tyr Arg
   865 870 875 880
   Cys Asn Lys Leu Arg Ser Arg Phe Pro Leu Ser Ser Asn His Leu Arg
   885 890 895
   Gly Ile Val Ile Leu Ser Ile Ser Lys Ser Arg Ala Ile Ser Val Tyr
   900 905 910
   Ser Glu Gly Ile Val Ser His Arg Leu Cys Lys Lys Asn Val Leu Gly
   915 920 925
   Phe Thr Cys Arg Val Leu Leu Leu Lys Phe Ser Gly His Val Phe Gly
   930 935 940
   Asn Asp Glu Met Leu Thr Lys Leu Leu Asn Val
   945 950 955
<210> 26
   <211> 951
   <212> PRT
   <213> Artificial sequence
<220>
   <223> BTV-22 VP2 consensus sequence
<400> 26
   Met Glu Asp Phe Pro Ile Cys Ala Ile Glu Arg Glu Ser Val Tyr Asp
   1               5                   10                  15
   Glu Asp Phe Ile Met Arg Tyr Pro Val Ile Val Asp Leu Lys Glu Cys
               20                  25                  30
   Val Ala Asp Arg Arg Asn Lys Thr Asp Val Ser Lys Leu Glu Glu Ile
           35                  40                  45
   Lys Gly Val Cys Val Arg Ser Glu Gly Val Arg Val Ala Leu Thr Ser
       50                  55                  60
   Arg Pro Asp Pro Leu Ser His Val Ile Thr Pro Ser Ala Leu Asn Val
   65                  70                  75                  80
   Leu Ile Gln Ala Tyr Asp Arg Lys His Lys Val Ser Leu Arg Lys Arg
                   85                  90                  95
   Phe Glu Asp Gly Glu Gly Val Thr Glu Tyr Asp Leu Asp Thr Trp Val
               100                 105                 110
   Lys Lys Arg Leu Glu Glu Gln Ala Asp Leu Gln Ala Val Tyr His Thr
           115                 120                 125
   Ile Asp Ala Asn Arg Gly Lys Leu Lys Phe Lys Thr Leu Met Gly Thr
       130                 135                 140
   Ile Arg Leu Asp Ser Asn Tyr Ala Glu Thr Val Gly Tyr Asp Tyr Ala
   145                 150                 155                 160
   Pro Val Ser Lys Ala Gly Cys Asn His Gly Asn Glu Asp Pro Leu Tyr
                   165                 170                 175
   Asp Phe Ile Leu Ser Gly Ala Phe Gln Val Ser Lys Thr Ala Gly Tyr
               180                 185                 190
   Thr Leu Lys Gln Thr Tyr Arg Leu Thr Val Arg Thr Glu Val Arg Glu
           195                 200                 205
   Asn Asn Lys Asn Glu Leu Val Val Asn Glu Ile Tyr Thr Pro Gly Ile
       210                 215                 220
   Val Arg Gly Glu Gln Ile Arg Asp Met Thr Ser Val Ala Tyr Met Arg
   225                 230                 235                 240
   Phe Arg Asp Gly Trp Ile Arg Ala Ile Ile Gln Pro Gln Ile Val Glu
                   245                 250                 255
   Glu Leu Arg Ala Ser Lys Asp Ala Leu Asp Arg Ile Ser Asn Glu Trp
               260                 265                 270
   Tyr Ser Ser Arg Asn Thr Pro Asp Val Val Glu Ile Cys Arg Ile Val
           275                 280                 285
   Ser Ser Ile Gly Arg Arg Met Trp Asn Ser Glu Glu Thr Pro Val Asp
   290 295 300
   Asp Ala Met Arg Ser Arg Val Phe Gln Glu Lys Leu Arg Leu Leu Phe
   305 310 315 320
   Arg Val Gly Asn Ser Glu Tyr Asp Thr Ile Gln Ser Ile Ser Ser Gly
   325 330 335
   Gln Thr Asp Leu Lys Lys Phe Tyr Ala Leu Leu Ala Ile Ala Ala Thr
   340 345 350
   Asp Ser Tyr Arg Trp Arg Ile Trp Trp Ser Asn Pro Tyr Pro Cys Leu
   355 360 365
   Arg Gly Thr Ile Ile Ala Cys Glu Met Glu Leu Gly Asp Val Tyr Lys
   370 375 380
   Thr Leu Arg Ser Thr Phe Arg Trp Ser Leu Arg Pro Glu Tyr Ala Asn
   385 390 395 400
   Arg Ser Glu Ile Asp Arg Glu Lys Lys Ala Tyr Pro Tyr Gln Arg Ile
   405 410 415
   Asn Leu Phe Glu Ser Lys Leu Thr Pro Gly Thr Arg Ile Ile Asn Trp
   420 425 430
   Leu Val Thr Arg Val Pro Leu Glu Lys Pro Asp Ile Ser Thr Gly Arg
   435 440 445
   Val Cys Met Thr Ser Thr Glu Ser Asn Tyr Ile Leu Lys Ile Asp Asp
   450 455 460
   Ala Arg Tyr Lys Gln Met Ile Ser Gln Ile Ile Glu Arg Gly Trp Glu
   465 470 475 480
   Lys Glu Gln Leu Lys Phe Glu Lys Ile Val Gln Asp Asp Gly Asn Val
   485 490 495
   Phe Lys Met Glu Phe Glu Lys Asp Ala Cys Met Asp Glu Lys Ser His
   500 505 510
   Leu Val Met Pro Tyr Tyr Tyr Asp Lys Glu Ile His Cys Pro Ile Tyr
   515 520 525
   His Gln Lys Ile Lys Ile Thr Glu Ala Gln Met Ala Asn Glu Trp Ser
   530 535 540
   Asp Asp Pro Trp Val Asn Arg Val Val Ser Gly Tyr Leu Gly Asp His
   545 550 555 560
   Val Glu Thr Tyr Gly Leu Gly Tyr Asn His Ile Phe Asp Thr Arg Val
   565 570 575
   Pro Leu Lys Gly Asn Leu Leu Glu Gln Glu Gln Glu Ile Ser Ser Tyr
   580 585 590
   His Glu His Leu Arg Ser Asp Glu Ser Lys Glu Asp Asp Thr His Cys
   595 600 605
   Ala Leu Arg Ile Phe Ser Asp Lys Ala Ile Arg Met Leu Phe Ile Ala
   610 615 620
   Ile Tyr Lys Lys Leu Ile His His Ile Pro Lys Glu Glu Tyr Ala Thr
   625 630 635 640
   Arg Glu Glu Glu Leu Ile Gln Tyr Ile Ser Met Val Glu Arg Val Arg
   645 650 655
   Ser Trp Asp Gln Phe Ile Val Phe Ile Phe Asp Phe Phe Phe Glu Asn
   660 665 670
   Arg Lys Gly Ile Arg Thr Met Glu Glu Ala Thr Gln Leu Ala Lys Ser
   675 680 685
   Ile Arg Gly Ser Val Gly Ala Met Arg Trp Thr Ile Phe Ser Ser Ser
   690 695 700
   Phe Pro Thr Ser Tyr Arg Leu Met Lys Gln Ile Leu Val Ala Lys Arg
   705 710 715 720
   Trp Gly Asp Cys Tyr Ile Met Asn Phe Leu Pro Leu Leu Ile Cys Gly
   725 730 735
   Gly Thr Ser Ile Ser Tyr Leu His Arg Glu Trp Ser Tyr Pro Met Ile
   740 745 750
   Val Thr Phe Glu Asp Gly Ile Lys Ile Val Pro Thr Met Val Gly Arg
   755 760 765
   Ser Leu Pro Gln Gly Ser Leu Gly Glu Trp Val Ser Phe Ile Ser Phe
   770 775 780
   Phe Met Gly Thr Val His Glu Lys Ile Lys Val Glu Glu Asp Glu Leu
   785 790 795 800
   Glu Val Leu Arg Arg Cys Val Glu Phe Tyr Arg Asp Val Lys Met Val
   805 810 815
   Asp Val Ile Lys Asp Thr Pro Val Arg Met Thr Lys Tyr Ile Thr Cys
   820 825 830
   Asp Leu Ala Phe Gly Ser Gly Cys Gly Gly Ile Ser Glu Ser Ile Ser
   835 840 845
   Phe Ile Leu Pro Val Thr His Pro Asn Arg Ser Leu Val Val Phe Val
   850 855 860
   Ile Ser Asp Asp Lys Leu Ser Pro Ser Gln His Ala Ala Arg Ala Lys
   865 870 875 880
   Arg Arg Phe Lys Gly Thr Cys Gln Tyr Ile Asp Asp Val Ile Val Leu
   885 890 895
   Gln Met Lys Arg Pro Gly Leu Val Gly Ser Val Trp Ser Glu Lys His
   900 905 910
   Ser Lys Ile Lys Val Cys Arg Arg Asn Phe Leu Asn Tyr Asp His Lys
   915 920 925
   Val Ile Leu Thr Lys Phe Ala Gly Leu Val Tyr Gly Asn Tyr Glu Leu
   930 935 940
   Leu Thr Lys Leu Ala Asn Leu
   945 950
<210> 27
   <211> 957
   <212> PRT
   <213> Artificial sequence
<220>
   <223> BTV-23 VP2 consensus sequence
<220>
   <221> misc_feature
   <222> (25)..(25)
   <223> Xaa can be any naturally occurring amino acid
<220>
   <221> misc_feature
   <222> (41)..(41)
   <223> Xaa can be any naturally occurring amino acid
<220>
   <221> misc_feature
   <222> (49)..(49)
   <223> Xaa can be any naturally occurring amino acid
<220>
   <221> misc_feature
   <222> (97)..(97)
   <223> Xaa can be any naturally occurring amino acid
<220>
   <221> misc_feature
   <222> (141)..(141)
   <223> Xaa can be any naturally occurring amino acid
<220>
   <221> misc_feature
   <222> (156)..(156)
   <223> Xaa can be any naturally occurring amino acid
<220>
   <221> misc_feature
   <222> (160)..(160)
   <223> Xaa can be any naturally occurring amino acid
<220>
   <221> misc_feature
   <222> (163)..(163)
   <223> Xaa can be any naturally occurring amino acid
<220>
   <221> misc_feature
   <222> (223)..(223)
   <223> Xaa can be any naturally occurring amino acid
<220>
   <221> misc_feature
   <222> (294)..(294)
   <223> Xaa can be any naturally occurring amino acid
<220>
   <221> misc_feature
   <222> (310)..(311)
   <223> Xaa can be any naturally occurring amino acid
<220>
   <221> misc_feature
   <222> (335)..(335)
   <223> Xaa can be any naturally occurring amino acid
<220>
   <221> misc_feature
   <222> (361)..(361)
   <223> Xaa can be any naturally occurring amino acid
<220>
   <221> misc_feature
   <222> (399)..(399)
   <223> Xaa can be any naturally occurring amino acid
<220>
   <221> misc_feature
   <222> (414)..(414)
   <223> Xaa can be any naturally occurring amino acid
<220>
   <221> misc_feature
   <222> (456)..(456)
   <223> Xaa can be any naturally occurring amino acid
<220>
   <221> misc_feature
   <222> (469)..(469)
   <223> Xaa can be any naturally occurring amino acid
<220>
   <221> misc_feature
   <222> (508)..(509)
   <223> Xaa can be any naturally occurring amino acid
<220>
   <221> misc_feature
   <222> (526)..(526)
   <223> Xaa can be any naturally occurring amino acid
<220>
   <221> misc_feature
   <222> (537)..(537)
   <223> Xaa can be any naturally occurring amino acid
<220>
   <221> misc_feature
   <222> (583)..(583)
   <223> Xaa can be any naturally occurring amino acid
<220>
   <221> misc_feature
   <222> (613)..(613)
   <223> Xaa can be any naturally occurring amino acid
<220>
   <221> misc_feature
   <222> (641)..(641)
   <223> Xaa can be any naturally occurring amino acid
<220>
   <221> misc_feature
   <222> (655)..(656)
   <223> Xaa can be any naturally occurring amino acid
<220>
   <221> misc_feature
   <222> (673)..(673)
   <223> Xaa can be any naturally occurring amino acid
<220>
   <221> misc_feature
   <222> (742)..(742)
   <223> Xaa can be any naturally occurring amino acid
<220>
   <221> misc_feature
   <222> (782)..(782)
   <223> Xaa can be any naturally occurring amino acid
<220>
   <221> misc_feature
   <222> (795)..(795)
   <223> Xaa can be any naturally occurring amino acid
<220>
   <221> misc_feature
   <222> (818)..(818)
   <223> Xaa can be any naturally occurring amino acid
<220>
   <221> misc_feature
   <222> (906).. (906)
   <223> Xaa can be any naturally occurring amino acid
<220>
   <221> misc_feature
   <222> (938)..(938)
   <223> Xaa can be any naturally occurring amino acid
<220>
   <221> misc_feature
   <222> (943)..(943)
   <223> Xaa can be any naturally occurring amino acid
<400> 27
   Met Asp Glu Leu Ala Ile Pro Ile Tyr Thr Asn Val Phe Pro Ala Glu
   1               5                   10                  15
   Leu Leu Asp Gly Tyr Asp Tyr Ile Xaa Asp Val Ser Thr Arg Ile Asp
               20                  25                  30
   Glu Pro Ala Gly Arg His Asp Val Xaa Gln Ile Pro Asn Ser Ala Met
           35                  40                  45
   Xaa Asp Val Gln Asn Glu His Ile Arg Asp Ala Ile Ile Ser Lys Pro
       50                  55                  60
   Val Ser Asn Asp Gly Tyr Val Leu Pro Arg Ile Leu Asp Ile Ser Leu
   65                  70                  75                  80
   Arg Ala Phe Asp Asp Arg Lys Arg Ile Ile Thr Ser Gly Gly Gln Ser
                   85                  90                  95
   Xaa Tyr His Thr Lys Ala Asp Trp Leu Gln Trp Met Ile Asp Asp Ala
               100                 105                 110
   Met Asp Val Gln Pro Leu Lys Val Asp Thr Ala His Thr Arg Thr Arg
           115                 120                 125
   Val Ser His Ala Leu Phe Asn Cys Thr Val Arg Leu Xaa Ala Lys Lys
       130                 135                 140
   Ala Asp Thr Ala Ser Tyr His Val Glu Ser Val Xaa Ile Glu Pro Xaa
   145                 150                 155                 160
   Gly Cys Xaa His Ala Trp Ile Gln Arg Val His His Leu Val Asn Ile
                   165                 170                 175
   Glu Met Leu His Cys Ser Gln Glu Val Ala Tyr Met Leu Lys Pro Thr
               180                 185                 190
   Tyr Glu Thr Ile Val His Ala Glu Arg Ala Ser Thr Gly Asp Thr Phe
           195                 200                 205
   Asn Gly Arg Thr Ile Glu Leu Ser Arg Gly His Arg Val Gln Xaa Gly
       210                 215                 220
   Asp Ala Ala Tyr Glu Lys Leu Lys Asn Gly Leu Val Gln Thr Arg Ile
   225                 230                 235                 240
   Glu Gly Lys Lys Pro Gln Leu Val Glu Gly Glu Ile Asn Ser Leu Asn
                   245                 250                 255
   Asp Ile Arg Gln Arg Trp Ile Asn Ser Gly His Asp Arg Arg Glu Ile
               260                 265                 270
   Lys Ala Leu Glu Leu Cys Lys Ile Leu Ser Thr Ile Gly Arg Lys Met
           275                 280                 285
   Leu Asn Thr Glu Glu Xaa Pro Lys Asn Glu Ala Asp Leu Ser Val Lys
       290                 295                 300
   Phe Gln Met Glu Leu Xaa Xaa Ile Phe Arg Pro Gly Asn Asn Glu Arg
   305                 310                 315                 320
   Thr Asn Ile Phe Gly Gly Gly Asn His Arg Lys Asp Glu Asp Xaa Phe
                   325                 330                 335
   Tyr Val Leu Ile Met Ile Ala Ala Ser Asp Thr Tyr Lys Gly Arg Ile
               340                 345                 350
   Trp Trp Ser Asn Pro Tyr Pro Cys Xaa Arg Gly Ser Leu Ile Ala Ala
           355                 360                 365
   Glu Ala Gln Leu Gly Asp Val Tyr Asn Thr Met Arg Asn Trp Tyr Gln
       370                 375                 380
   Trp Ser Val Arg Pro Thr Tyr Thr Pro Tyr Asp Arg Ser Arg Xaa Ser
   385                 390                 395                 400
   Asp Lys Tyr Ile Tyr Ser Arg Val Asn Leu Phe Asp Ser Xaa Leu Arg
                   405                 410                 415
   Pro Gly Asp Lys Ile Ile His Trp Glu Tyr Lys Leu Leu Asn Glu Val
               420                 425                 430
   Arg Glu Val Cys Ile Lys Asp Gly Asn Glu Cys Asp Leu Phe Pro Glu
           435                 440                 445
   Asp Glu Glu Met Thr Thr Lys Xaa Asn Glu Glu Lys Tyr Thr Glu Met
       450                 455                 460
   Lys Gly Gln Ile Xaa Gln Asp Gly Trp Asn His Arg Asp Phe Lys Met
   465                 470                 475                 480
   His Lys Ile Leu Gln Asp Gly Ala Asn Val Leu Thr Ile Asp Phe Glu
                   485                 490                 495
   Lys Asp Ala Arg Ile Gly Asn Gly Ser Val Leu Xaa Xaa Pro Glu Tyr
               500                 505                 510
   Tyr Asn Lys Trp Ile Val Ala Pro Met Phe Asn Ala Lys Xaa Arg Ile
           515                 520                 525
   Ser Glu Thr Val Ile Ala Lys Ala Xaa Ser Asp Asp Pro Ala Val Ala
       530                 535                 540
   Arg Thr Ala Lys Ser Phe Asp His Asp Pro Phe Asn Leu Gln Arg Tyr
   545                 550                 555                 560
   Cys Leu Ala Arg Tyr Tyr Asp Val Arg Pro Gly Leu Met Gly Arg Ala
                   565                 570                 575
   Leu Ser Lys Glu Gln Glu Xaa Ser Ala Ile Ala Asn Lys Val Ala Ala
               580                 585                 590
   Leu Glu Asp Tyr Arg Asp Val Ile Ser Arg Arg Leu Gly Tyr Val Glu
           595                 600                 605
   Arg Glu Lys Arg Xaa Leu Thr Glu Thr Ala Gln Tyr Ile Leu Glu Lys
       610                 615                 620
   Thr Ser Leu Tyr Leu Leu Asn Val Leu Ser Met His Val Lys Pro Thr
   625                 630                 635                 640
   Xaa Asp Met Asp Ile Lys Phe Lys His Pro Ser Ile Asp Thr Xaa Xaa
                   645                 650                 655
   Asp Ile Asp Thr Trp Lys Ile Ala Asp Ile Ser Gln Leu Ile Val Leu
               660                 665                 670
   Xaa Phe Asp Tyr Leu Phe Glu Ser Arg Lys Glu Ile Arg Ser Val Thr
   675 680 685
   Glu Ala Arg Trp Ile Leu Phe Lys Ile Arg Ser Val Ser Gly Gln Glu
   690 695 700
   Arg Leu Asp Met Ile Gln Ser Phe Phe Pro Arg Phe Gly Arg Val Val
   705 710 715 720
   Arg Asp Ile Ala Lys Ala Lys Ile Thr Gln Asp Val Ala His Leu Asn
   725 730 735
   Phe Phe Pro Leu Phe Xaa Ile Ile Gly Asp Asn Met Ala Tyr Ala His
   740 745 750
   Arg Gln Trp Ser Ile Pro Val Leu Leu Tyr Ala His Asp Ile Arg Ile
   755 760 765
   Ile Pro Leu Glu Ile Gly Ala Tyr Asn Asn Arg Phe Gly Xaa Thr Ser
   770 775 780
   Tyr Leu Glu Tyr Met Thr Phe Phe Pro Ser Xaa Ala Thr Arg Glu Ala
   785 790 795 800
   Val Leu Asp Glu Ser Ile Lys Glu Cys Ser Leu Ala Leu Thr Asn Phe
   805 810 815
   Tyr Xaa Asn Thr Asp Ile His Ser Gly Glu Ile Met Arg Asn Val Val
   820 825 830
   Thr Thr Lys Arg Leu Leu Tyr Glu Thr His Leu Ala Ser Leu Cys Gly
   835 840 845
   Gly Tyr Ser Asp Gly Leu Leu Trp Tyr Val Pro Ile Thr His Pro Ser
   850 855 860
   Lys Cys Leu Val Ala Phe Glu Val Ala Asp Asp Val Val Pro Tyr Ser
   865 870 875 880
   Val Arg Arg Glu Arg Ile Ile Ser Arg Phe Pro Leu Ser Ser Lys His
   885 890 895
   Leu Arg Gly Ile Ala Leu Ile Ala Val Xaa Arg Asn Gln Lys Val Ser
   900 905 910
   Val Gln Thr Glu Gly Ile Val Thr His Arg Leu Cys Lys Lys Asn Leu
   915 920 925
   Leu Lys Tyr Val Cys Asp Val Val Leu Xaa Lys Phe Ser Gly Xaa Val
   930 935 940
   Phe Gly Asn Asp Glu Met Leu Thr Lys Leu Leu Asn Val
   945 950 955
<210> 28
   <211> 955
   <212> PRT
   <213> Artificial sequence
<220>
   <223> BTV-24 VP2 consensus sequence
<220>
   <221> misc_feature
   <222> (19)..(19)
   <223> Xaa can be any naturally occurring amino acid
<220>
   <221> misc_feature
   <222> (23)..(23)
   <223> Xaa can be any naturally occurring amino acid
<220>
   <221> misc_feature
   <222> (33)..(33)
   <223> Xaa can be any naturally occurring amino acid
<220>
   <221> misc_feature
   <222> (37)..(37)
   <223> Xaa can be any naturally occurring amino acid
<220>
   <221> misc_feature
   <222> (55)..(56)
   <223> Xaa can be any naturally occurring amino acid
<220>
   <221> misc_feature
   <222> (58)..(58)
   <223> Xaa can be any naturally occurring amino acid
<220>
   <221> misc_feature
   <222> (64)..(64)
   <223> Xaa can be any naturally occurring amino acid
<220>
   <221> misc_feature
   <222> (76)..(76)
   <223> Xaa can be any naturally occurring amino acid
<220>
   <221> misc_feature
   <222> (81)..(81)
   <223> Xaa can be any naturally occurring amino acid
<220>
   <221> misc_feature
   <222> (88)..(88)
   <223> Xaa can be any naturally occurring amino acid
<220>
   <221> misc_feature
   <222> (90)..(90)
   <223> Xaa can be any naturally occurring amino acid
<220>
   <221> misc_feature
   <222> (119)..(119)
   <223> Xaa can be any naturally occurring amino acid
<220>
   <221> misc_feature
   <222> (122)..(122)
   <223> Xaa can be any naturally occurring amino acid
<220>
   <221> misc_feature
   <222> (124)..(125)
   <223> Xaa can be any naturally occurring amino acid
<220>
   <221> misc_feature
   <222> (138)..(138)
   <223> Xaa can be any naturally occurring amino acid
<220>
   <221> misc_feature
   <222> (164)..(164)
   <223> Xaa can be any naturally occurring amino acid
<220>
   <221> misc_feature
   <222> (182)..(182)
   <223> Xaa can be any naturally occurring amino acid
<220>
   <221> misc_feature
   <222> (193)..(193)
   <223> Xaa can be any naturally occurring amino acid
<220>
   <221> misc_feature
   <222> (259)..(259)
   <223> Xaa can be any naturally occurring amino acid
<220>
   <221> misc_feature
   <222> (261)..(261)
   <223> Xaa can be any naturally occurring amino acid
<220>
   <221> misc_feature
   <222> (330)..(330)
   <223> Xaa can be any naturally occurring amino acid
<220>
   <221> misc_feature
   <222> (372)..(372)
   <223> Xaa can be any naturally occurring amino acid
<220>
   <221> misc_feature
   <222> (392)..(392)
   <223> Xaa can be any naturally occurring amino acid
<220>
   <221> misc_feature
   <222> (399)..(399)
   <223> Xaa can be any naturally occurring amino acid
<220>
   <221> misc_feature
   <222> (446)..(446)
   <223> Xaa can be any naturally occurring amino acid
<220>
   <221> misc_feature
   <222> (452)..(452)
   <223> Xaa can be any naturally occurring amino acid
<220>
   <221> misc_feature
   <222> (458)..(458)
   <223> Xaa can be any naturally occurring amino acid
<220>
   <221> misc_feature
   <222> (498)..(498)
   <223> Xaa can be any naturally occurring amino acid
<220>
   <221> misc_feature
   <222> (502)..(502)
   <223> Xaa can be any naturally occurring amino acid
<220>
   <221> misc_feature
   <222> (544)..(544)
   <223> Xaa can be any naturally occurring amino acid
<220>
   <221> misc_feature
   <222> (597)..(597)
   <223> Xaa can be any naturally occurring amino acid
<220>
   <221> misc_feature
   <222> (602)..(602)
   <223> Xaa can be any naturally occurring amino acid
<220>
   <221> misc_feature
   <222> (604)..(604)
   <223> Xaa can be any naturally occurring amino acid
<220>
   <221> misc_feature
   <222> (623)..(623)
   <223> Xaa can be any naturally occurring amino acid
<220>
   <221> misc_feature
   <222> (638)..(638)
   <223> Xaa can be any naturally occurring amino acid
<220>
   <221> misc_feature
   <222> (645)..(645)
   <223> Xaa can be any naturally occurring amino acid
<220>
   <221> misc_feature
   <222> (667)..(667)
   <223> Xaa can be any naturally occurring amino acid
<220>
   <221> misc_feature
   <222> (671)..(671)
   <223> Xaa can be any naturally occurring amino acid
<220>
   <221> misc_feature
   <222> (673)..(673)
   <223> Xaa can be any naturally occurring amino acid
<220>
   <221> misc_feature
   <222> (695)..(695)
   <223> Xaa can be any naturally occurring amino acid
<220>
   <221> misc_feature
   <222> (697)..(697)
   <223> Xaa can be any naturally occurring amino acid
<220>
   <221> misc_feature
   <222> (723)..(723)
   <223> Xaa can be any naturally occurring amino acid
<220>
   <221> misc_feature
   <222> (728)..(728)
   <223> Xaa can be any naturally occurring amino acid
<220>
   <221> misc_feature
   <222> (793)..(793)
   <223> Xaa can be any naturally occurring amino acid
<220>
   <221> misc_feature
   <222> (795)..(795)
   <223> Xaa can be any naturally occurring amino acid
<220>
   <221> misc_feature
   <222> (805)..(805)
   <223> Xaa can be any naturally occurring amino acid
<220>
   <221> misc_feature
   <222> (860)..(860)
   <223> Xaa can be any naturally occurring amino acid
<220>
   <221> misc_feature
   <222> (869)..(869)
   <223> Xaa can be any naturally occurring amino acid
<220>
   <221> misc_feature
   <222> (874)..(874)
   <223> Xaa can be any naturally occurring amino acid
<220>
   <221> misc_feature
   <222> (904)..(904)
   <223> Xaa can be any naturally occurring amino acid
<220>
   <221> misc_feature
   <222> (912)..(912)
   <223> Xaa can be any naturally occurring amino acid
<220>
   <221> misc_feature
   <222> (930)..(930)
   <223> Xaa can be any naturally occurring amino acid
<220>
   <221> misc_feature
   <222> (946)..(946)
   <223> Xaa can be any naturally occurring amino acid
<400> 28
   Met Glu Glu Phe Val Ile Pro Val Tyr Ser Glu Asn Glu Ile Pro Tyr
   1               5                   10                  15
   Thr Leu Xaa Asn Arg Tyr Xaa Leu Ala Ile Arg Thr Asp Val Lys Ile
               20                  25                  30
   Xaa Asp Val Glu Xaa Lys His Asn Val Val Lys Ile Pro Asp Ser Asp
           35                  40                  45
   Met Ile Asp Ile Pro Lys Xaa Xaa Ile Xaa Asp Ala Leu Ser Tyr Xaa
       50                  55                  60
   Pro Thr Arg Asn Asp Gly Ile Val Val Pro Arg Xaa Leu Asp Ile Thr
   65                  70                  75                  80
   Xaa Arg Ala Tyr Asp Asp Arg Xaa Ala Xaa Lys Asn Ala Lys Gly Ile
                   85                  90                  95
   Glu Phe Met Thr Asn Ala Lys Trp Met Lys Trp Ala Ile Asp Asp Arg
               100                 105                 110
   Met Asp Ile Gln Pro Leu Xaa Val Thr Xaa Asp Xaa Xaa Tyr Ser Val
           115                 120                 125
   Asn His Gln Leu Phe Asn Cys Val Val Xaa Ala Lys Ser Ala Asn Ala
       130                 135                 140
   Asp Thr Ile Tyr Tyr Asp Tyr Tyr Pro Leu Glu Ser Lys Lys Lys Lys
   145                 150                 155                 160
   Cys Asn His Xaa Asn Leu Asp Leu Leu Arg Ser Leu Thr Thr Met Glu
                   165                 170                 175
   Met Tyr His Ile Leu Xaa Gly Ala Ala Tyr Gly Leu Lys Ser Thr Tyr
               180                 185                 190
   Xaa Leu Lys Ala His Ala Glu Arg Glu Asn Thr Gly Glu Asn Tyr Pro
           195                 200                 205
   Ile Gly Glu Arg Leu Leu Gln Ile Pro Arg Gly Thr Lys Ile Gly Ser
       210                 215                 220
   Arg Gly Glu Pro Tyr Asn Lys Phe Ile Ala Gly Leu Val Lys Val Thr
   225                 230                 235                 240
   Ile Lys Gly Lys Val Pro Thr Glu Ile Arg Glu Glu Met Asn Gln Leu
                   245                 250                 255
   Asn Arg Xaa Lys Xaa Glu Trp Lys Ser Ala Ser His Asp Arg Ser Arg
               260                 265                 270
   Ile Arg Ala Leu Glu Leu Cys Lys Ile Leu Ser Thr Ile Gly Arg Lys
           275                 280                 285
   Met Leu Asp Ala Lys Glu Glu Pro Lys Asp Glu Met Asp Leu Ser Thr
       290                 295                 300
   Arg Phe Gln Phe Lys Leu Asp Glu Lys Phe Thr Lys Thr Asp Ser Glu
   305                 310                 315                 320
   His Ile Asn Ile Phe Arg Ala Gly Gly Xaa Ala Thr Asp Asp Gly Arg
                   325                 330                 335
   Phe Tyr Ala Leu Ile Ala Ile Ala Ala Thr Asp Thr Gln Lys Gly Arg
               340                 345                 350
   Val Trp Arg Met Asn Pro Tyr Pro Cys Leu Arg Gly Ala Ile Ile Ala
           355                 360                 365
   Ala Glu Cys Xaa Leu Gly Asp Val Tyr Phe Thr Leu Arg Gln Val Tyr
       370                 375                 380
   Ser Trp Ser Leu Arg Pro Glu Xaa Gly Gln Lys Glu Lys Pro Xaa Glu
   385                 390                 395                 400
   Val Asn Lys Tyr Ile Phe Asn Arg Ile Asn Leu Phe Asp Ser Asp Leu
                   405                 410                 415
   Thr Val Gly Asp Gln Ile Val His Trp His Tyr Glu Leu Tyr Pro Pro
               420                 425                 430
   Ala Glu Thr Thr Tyr Asp Asp Gly Tyr Leu Cys Gly Ser Xaa Lys Gly
           435                 440                 445
   Asp Asp Glu Xaa Leu Cys Glu Val Asp Xaa Glu Lys Tyr Lys Gly Met
       450                 455                 460
   Phe Glu Ser Met Ile Glu Gly Gly Trp Asp Glu Glu Arg Phe Lys Leu
   465                 470                 475                 480
   His Ser Ile Leu Thr Asp Pro Asn Leu Leu Thr Ile Asp Phe Glu Lys
                   485                 490                 495
   Asp Xaa Tyr Leu Asn Xaa Arg Ser Glu Leu Val Leu Pro Glu Tyr Phe
               500                 505                 510
   Asp Lys Trp Ile Asn Ser Pro Met Phe Asn Ala Lys Leu Arg Leu Ala
           515                 520                 525
   His Gly Glu Ile Ala Thr Arg Arg Thr Asn Asp Pro Trp Asn Lys Xaa
       530                 535                 540
   Ala Val His Gly Tyr Ile Arg Thr Ser Thr Glu Ser Leu Gly Tyr Ala
   545                 550                 555                 560
   Leu Gly Ser Tyr Tyr Asp Leu Arg Leu Gln Leu Phe Gly Asp Ala Leu
                   565                 570                 575
   Ser Leu Glu Gln Gln Gln Ser Ala Val Phe Glu His Leu Ser Lys Gln
               580                 585                 590
   Asp Asp Phe Phe Xaa Leu Thr Gly Tyr Xaa Lys Xaa Gln Val Val Cys
           595                 600                 605
   Pro His Ser Gly Gly Ala Phe Tyr Thr Phe Arg Arg Val Ala Xaa Glu
       610                 615                 620
   Ile Leu Ser Asn Tyr Glu Lys Leu Val Pro Asp Leu His Xaa Gly Leu
   625 630 635 640
   Glu His Gln Thr Xaa Thr His Pro Lys Ile Asn Glu Tyr Phe Asn Arg
   645 650 655
   Phe Val Leu Asp Met Lys Asp Phe Ser Gln Xaa Ile Cys Phe Xaa Phe
   660 665 670
   Xaa His Phe Phe Glu Arg His Glu Gln Leu Arg Asp Val Ala Glu Ala
   675 680 685
   Arg Arg Leu Val Tyr Leu Xaa Gln Xaa Ser Asp Gly Pro Glu Arg Leu
   690 695 700
   Asp Val Leu Gln Lys Asn Phe Pro Asn Phe Phe Arg Arg Phe Met Asn
   705 710 715 720
   Leu Lys Xaa Val Lys Tyr Ile Xaa Asp Leu Asn Val Val Asn Phe Leu
   725 730 735
   Pro Leu Leu Phe Leu Ile Gln Asp Asn Ile Ser Tyr Trp His Arg Gln
   740 745 750
   Trp Ser Val Pro Val Ile Leu Tyr Gly Glu Ile Ile Arg Leu Ile Pro
   755 760 765
   Val Glu Val Gly Ala Tyr Ala Asn Arg Phe Gly Met Lys Ser Phe Phe
   770 775 780
   Asn Phe Ile Arg Phe His Pro Gly Xaa Ser Xaa Lys Lys Gln Lys Ala
   785 790 795 800
   Asp Asp Thr His Xaa Glu Phe Gly Leu Val Cys Phe Glu Tyr Tyr Thr
   805 810 815
   Lys Thr Lys Ile Ser Gln Gly Gly Val Asp Ile Ser Val Val Thr Thr
   820 825 830
   Lys Met Asp Thr Leu Lys Ile His Leu Ala Ser Leu Cys Ser Gly Ile
   835 840 845
   Ala Asp Ser Ile Val Tyr Thr Leu Pro Ile Ala Xaa Pro Lys Lys Cys
   850 855 860
   Val Val Leu Ile Xaa Ile Gly Asp Asp Xaa Val Asp Met Gln Ile Arg
   865                 870                 875                 880
   Ser Glu Gln Ala Val Ser Arg Tyr Tyr Tyr Ser Arg Arg His Ile Ala
                   885                 890                 895
   Gly Val Val Ser Ile Cys Ile Xaa Gln Asp Gly Gly Leu Lys Val Xaa
               900                 905                 910
   Ser Thr Gly Ile Val Lys His Arg Ile Cys Glu Lys Ser Ile Leu Lys
           915                 920                 925
   Tyr Xaa Cys Lys Ala Ile Leu Val Arg Met Pro Gly Tyr Val Phe Gly
       930                 935                 940
   Asn Xaa Glu Leu Met Thr Lys Leu Leu Asn Val
   945                 950                 955
<210> 29
   <211> 959
   <212> PRT
   <213> Artificial sequence
<220>
   <223> BTV-25 VP2 consensus sequence
<400> 29
   Met Asp Glu Leu Val Ile Pro Ile Phe Ser Ala Asp Glu Met Pro Tyr
   1               5                   10                  15
   Ala Leu Ile Ser His Tyr Pro Leu Ala Ile Gln Thr Asn Arg Lys Ile
               20                  25                  30
   Gln Asn Asp Arg Glu Phe His Asp Leu Thr Lys Ile Pro Gln Ser Asn
           35                  40                  45
   Met Ile Asp Ile Ser Gln His Gly Val Ile Arg Ala Leu Asp Tyr Arg
       50                  55                  60
   Pro Ser Arg Asn Asp Gly Ile Val Ile Pro Arg Leu Leu Asp Ile Thr
   65                  70                  75                  80
   Leu His Ala Tyr Asp Lys Arg Arg Ser Met Lys Asp Ile Cys Gly Leu
                   85                  90                  95
   Glu Phe Lys Thr Asp Val Lys Trp Met Gln Trp Ala Ile Asn Asp Arg
               100                 105                 110
   Met Asp Ile Gln Pro Leu Lys Val Glu Ile Asp Asn Glu His Thr Val
           115                 120                 125
   Asn His Gln Leu Phe Asn Cys Asn Ile Lys Ile Arg Ser Lys Asn Val
       130                 135                 140
   Asp Thr Val Tyr Tyr Asp Tyr His Pro Leu Glu Ser Glu Ala Thr Lys
   145                 150                 155                 160
   Cys Asn His Ala Asn Ile Glu Leu Met Arg Ser Leu Thr Met Val Glu
                   165                 170                 175
   Leu Tyr His Val Leu Gln Gly Ala Ala Tyr Ala Leu Lys Thr Asn Phe
               180                 185                 190
   Glu Leu Ile Ala Thr Ala Glu Arg Gly Asp Val Arg Gln Arg Tyr Asp
           195                 200                 205
   Ala Ala Gly Val Arg Trp Ser Arg Leu Ser Arg Asp Gln Arg Ile Gly
       210                 215                 220
   Tyr Ser Gly Asn Ala Tyr Asp Met Phe Met Ala Asn Leu Val Gln Val
   225                 230                 235                 240
   Glu Val Lys Gly Lys Cys Ala Asp Glu Ile Ala Gly Glu Met Gly Arg
                   245                 250                 255
   Leu Ala Lys Ile Lys Asp Lys Trp Val Ser Asn Gly Phe Ser Gly Thr
               260                 265                 270
   Val Ile Phe Val Ser Glu Leu Cys Thr Glu Phe Ser Ala Ile Gly Arg
           275                 280                 285
   Lys Met Met Asp Thr His Glu Glu Pro Lys Asp Glu Glu Gly Ile Ser
       290                 295                 300
   Arg Arg Phe Gln Ile Glu Leu Asp Gly Met Phe Ser Ala Ala Arg Gln
   305                 310                 315                 320
   Glu Asn Lys Asn Val Leu Ser Gln Arg Gly Ser Lys Val Thr Asp Glu
                   325                 330                 335
   Ser Lys Phe Tyr Ala Leu Ile Met Ile Ala Ala Ser Asp Thr Gln Gln
               340                 345                 350
   Gly Arg Val Trp Arg Thr Asn Leu Tyr Pro Cys Leu Arg Gly Ala Leu
           355                 360                 365
   Val Ala Ala Glu Cys Val Met Gly Asp Val Tyr His Thr Leu Arg Thr
   370 375 380
   Val Phe Asn Trp Ser Val Arg Gly Thr Tyr Gly Gly Thr Glu Arg Asn
   385 390 395 400
   Leu Glu Asn Asn Arg Tyr Ile Phe Ser Arg Ile Asn Leu Phe Glu Thr
   405 410 415
   Asn Leu Pro Ser Gly Ser Lys Ile Val His Trp Cys Tyr Glu Glu Val
   420 425 430
   Ala Pro Ile Glu Thr Thr Tyr Asp Asn Gly Tyr Ile Cys Lys Ser Gln
   435 440 445
   Glu Gly Val Gln Asn Asp Glu Lys Leu Cys Lys Ile Asp Asp Asp Leu
   450 455 460
   Tyr Ala Arg Met Arg Asp Glu Ala Ile Thr Gly Gly Trp Arg Gln Glu
   465 470 475 480
   Asp Phe Lys Leu His Lys Ile Leu Thr Asn Pro Asn Leu Leu Thr Ile
   485 490 495
   Asp Phe Glu Lys Glu Ala Tyr Ile Thr Ser Thr Ser Asp Leu Ile Leu
   500 505 510
   Pro Asp Tyr Phe Asp Lys Trp Ile Lys Ala Pro Ile Phe Asn Ala Arg
   515 520 525
   Leu Arg Ile Thr Asn Gly Val Ile Glu Ser Glu Lys Lys Ser Asp Pro
   530 535 540
   Trp Asn Lys Arg Val Ala Asn Gly Thr Met Lys Thr Ser Ala Glu Ser
   545 550 555 560
   Leu Gly Tyr Val Leu Gly Arg Phe Tyr Asp Leu Arg Met Gln Phe Val
   565 570 575
   Glu Asp Val Leu Asn Thr Arg Gln Gln Lys Ser Leu Ala Leu Gln His
   580 585 590
   Ile Arg Asn Leu Glu Asp Phe Thr Arg Leu Thr Asp Tyr Gly Glu Glu
   595 600 605
   Gln Asn Ile Cys Pro His Ala Gly Gly Ala Met Tyr Thr Phe Arg Arg
   610 615 620
   Val Ala Leu Glu Ile Ile Thr Asn Tyr Glu Arg Leu Asn Pro Met Asp
   625 630 635 640
   His Glu Gly Asn Glu Tyr Gln Thr Tyr Arg His Pro Ala Ile Asp Tyr
   645 650 655
   Ser Lys Leu Gly Asp Val Leu Lys Met Lys Asp Leu Ser Gln Leu Ile
   660 665 670
   Cys Phe Val Phe Asp Ser Ile Phe Glu Arg Arg Asp Gln Leu Arg Glu
   675 680 685
   Val Gly Glu Ala Arg Met Ile Ile Tyr Gln Ile Gln Asn Ala Thr Gly
   690 695 700
   Glu Arg Arg Leu Asp Val Leu Lys Arg Glu Phe Pro Thr Phe Tyr Val
   705 710 715 720
   Trp Phe His Arg Leu Arg Asp Val Glu Leu Ile Arg Asp Leu Asn Val
   725 730 735
   Ile Asn Phe Leu Pro Leu Leu Phe Leu Val Arg Asp Asn Ala Ala Tyr
   740 745 750
   Arg His Arg Gln Trp Ser Ile Pro Met Ile Leu Tyr Asp Glu Glu Ile
   755 760 765
   Lys Leu Ile Pro Val Glu Val Gly Ala Tyr Ala Asn Arg Phe Gly Phe
   770 775 780
   Lys Ser Phe Ile Asn Phe Thr Arg Phe His Pro Gly Asp Ala Arg Lys
   785 790 795 800
   Arg Gln Glu Val Ser Ala Phe His Lys Ala Tyr Gly Ser Ile Cys Tyr
   805 810 815
   Arg Tyr Tyr Thr Asp Thr Thr Ile Ser Gln Gly Glu Val Asn Val Pro
   820 825 830
   Val Ile Thr Thr Lys Leu Asp Thr Leu Arg Val His Leu Ala Ala Leu
   835 840 845
   Cys Gly Gly Leu Ser Asp Ser Leu Val Tyr Thr Leu Pro Ile Thr His
   850 855 860
   Pro Glu Lys Cys Leu Val Leu Val Ile Val Gly Asp Asn Lys Leu Asn
   865 870 875 880
   Pro Gln Ile Arg Ser Glu Leu Val Ile Ser Arg Tyr Arg Tyr Ser Arg
   885 890 895
   Arg His Ile Arg Gly Val Val Ser Val Cys Ile Asp Gln Glu Gly Leu
   900 905 910
   Pro Asn Val Tyr Ser Thr Gly Ile Ala Arg His Lys Ile Tyr Glu Lys
   915 920 925
   Thr Val Leu Arg Tyr Arg Cys Lys Ile Ile Leu Val Lys Ile Ser Gly
   930 935 940
   Tyr Val Phe Gly Asn Asp Glu Leu Met Thr Lys Leu Leu Asn Val
   945 950 955
<210> 30
   <211> 959
   <212> PRT
   <213> Artificial sequence
<220>
   <223> BTV-26 VP2 consensus sequence
<400> 30
   Met Asp Glu Leu Val Ile Pro Ile Phe Ser Ala Asp Glu Met Pro Tyr
   1 5 10 15
   Ala Leu Ile Ser His Tyr Pro Leu Ala Ile Gln Thr Asn Arg Lys Ile
   20 25 30
   Gln Asn Asp Arg Glu Phe His Asp Leu Thr Lys Ile Pro Gln Ser Asn
   35 40 45
   Met Ile Asp Ile Ser Gln His Gly Val Ile Arg Ala Leu Asp Tyr Arg
   50 55 60
   Pro Ser Arg Asn Asp Gly Ile Val Ile Pro Arg Leu Leu Asp Ile Thr
   65 70 75 80
   Leu His Ala Tyr Asp Lys Arg Arg Ser Met Lys Asp Ile Cys Gly Leu
   85 90 95
   Glu Phe Lys Thr Asp Val Lys Trp Met Gln Trp Ala Ile Asn Asp Arg
   100 105 110
   Met Asp Ile Gln Pro Leu Lys Val Glu Ile Asp Asn Glu His Thr Val
   115 120 125
   Asn His Gln Leu Phe Asn Cys Asn Ile Lys Ile Arg Ser Lys Asn Val
       130                 135                 140
   Asp Thr Val Tyr Tyr Asp Tyr His Pro Leu Glu Ser Glu Ala Thr Lys
   145                 150                 155                 160
   Cys Asn His Ala Asn Ile Glu Leu Met Arg Ser Leu Thr Met Val Glu
                   165                 170                 175
   Leu Tyr His Val Leu Gln Gly Ala Ala Tyr Ala Leu Lys Thr Asn Phe
               180                 185                 190
   Glu Leu Ile Ala Thr Ala Glu Arg Gly Asp Val Arg Gln Arg Tyr Asp
           195                 200                 205
   Ala Ala Gly Val Arg Trp Ser Arg Leu Ser Arg Asp Gln Arg Ile Gly
       210                 215                 220
   Tyr Ser Gly Asn Ala Tyr Asp Met Phe Met Ala Asn Leu Val Gln Val
   225                 230                 235                 240
   Glu Val Lys Gly Lys Cys Ala Asp Glu Ile Ala Gly Glu Met Gly Arg
                   245                 250                 255
   Leu Ala Lys Ile Lys Asp Lys Trp Val Ser Asn Gly Phe Ser Gly Thr
               260                 265                 270
   Val Ile Phe Val Ser Glu Leu Cys Thr Glu Phe Ser Ala Ile Gly Arg
           275                 280                 285
   Lys Met Met Asp Thr His Glu Glu Pro Lys Asp Glu Glu Gly Ile Ser
       290                 295                 300
   Arg Arg Phe Gln Ile Glu Leu Asp Gly Met Phe Ser Ala Ala Arg Gln
   305                 310                 315                 320
   Glu Asn Lys Asn Val Leu Ser Gln Arg Gly Ser Lys Val Thr Asp Glu
                   325                 330                 335
   Ser Lys Phe Tyr Ala Leu Ile Met Ile Ala Ala Ser Asp Thr Gln Gln
               340                 345                 350
   Gly Arg Val Trp Arg Thr Asn Leu Tyr Pro Cys Leu Arg Gly Ala Leu
           355                 360                 365
   Val Ala Ala Glu Cys Val Met Gly Asp Val Tyr His Thr Leu Arg Thr
       370                 375                 380
   Val Phe Asn Trp Ser Val Arg Gly Thr Tyr Gly Gly Thr Glu Arg Asn
   385                 390                 395                 400
   Leu Glu Asn Asn Arg Tyr Ile Phe Ser Arg Ile Asn Leu Phe Glu Thr
                   405                 410                 415
   Asn Leu Pro Ser Gly Ser Lys Ile Val His Trp Cys Tyr Glu Glu Val
               420                 425                 430
   Ala Pro Ile Glu Thr Thr Tyr Asp Asn Gly Tyr Ile Cys Lys Ser Gln
           435                 440                 445
   Glu Gly Val Gln Asn Asp Glu Lys Leu Cys Lys Ile Asp Asp Asp Leu
       450                 455                 460
   Tyr Ala Arg Met Arg Asp Glu Ala Ile Thr Gly Gly Trp Arg Gln Glu
   465                 470                 475                 480
   Asp Phe Lys Leu His Lys Ile Leu Thr Asn Pro Asn Leu Leu Thr Ile
                   485                 490                 495
   Asp Phe Glu Lys Glu Ala Tyr Ile Thr Ser Thr Ser Asp Leu Ile Leu
               500                 505                 510
   Pro Asp Tyr Phe Asp Lys Trp Ile Lys Ala Pro Ile Phe Asn Ala Arg
           515                 520                 525
   Leu Arg Ile Thr Asn Gly Val Ile Glu Ser Glu Lys Lys Ser Asp Pro
       530                 535                 540
   Trp Asn Lys Arg Val Ala Asn Gly Thr Met Lys Thr Ser Ala Glu Ser
   545                 550                 555                 560
   Leu Gly Tyr Val Leu Gly Arg Phe Tyr Asp Leu Arg Met Gln Phe Val
                   565                 570                 575
   Glu Asp Val Leu Asn Thr Arg Gln Gln Lys Ser Leu Ala Leu Gln His
               580                 585                 590
   Ile Arg Asn Leu Glu Asp Phe Thr Arg Leu Thr Asp Tyr Gly Glu Glu
           595                 600                 605
   Gln Asn Ile Cys Pro His Ala Gly Gly Ala Met Tyr Thr Phe Arg Arg
       610                 615                 620
   Val Ala Leu Glu Ile Ile Thr Asn Tyr Glu Arg Leu Asn Pro Met Asp
   625                 630                 635                 640
   His Glu Gly Asn Glu Tyr Gln Thr Tyr Arg His Pro Ala Ile Asp Tyr
                   645                 650                 655
   Ser Lys Leu Gly Asp Val Leu Lys Met Lys Asp Leu Ser Gln Leu Ile
               660                 665                 670
   Cys Phe Val Phe Asp Ser Ile Phe Glu Arg Arg Asp Gln Leu Arg Glu
           675                 680                 685
   Val Gly Glu Ala Arg Met Ile Ile Tyr Gln Ile Gln Asn Ala Thr Gly
       690                 695                 700
   Glu Arg Arg Leu Asp Val Leu Lys Arg Glu Phe Pro Thr Phe Tyr Val
   705                 710                 715                 720
   Trp Phe His Arg Leu Arg Asp Val Glu Leu Ile Arg Asp Leu Asn Val
                   725                 730                 735
   Ile Asn Phe Leu Pro Leu Leu Phe Leu Val Arg Asp Asn Ala Ala Tyr
               740                 745                 750
   Arg His Arg Gln Trp Ser Ile Pro Met Ile Leu Tyr Asp Glu Glu Ile
           755                 760                 765
   Lys Leu Ile Pro Val Glu Val Gly Ala Tyr Ala Asn Arg Phe Gly Phe
       770                 775                 780
   Lys Ser Phe Ile Asn Phe Thr Arg Phe His Pro Gly Asp Ala Arg Lys
   785                 790                 795                 800
   Arg Gln Glu Val Ser Ala Phe His Lys Ala Tyr Gly Ser Ile Cys Tyr
                   805                 810                 815
   Arg Tyr Tyr Thr Asp Thr Thr Ile Ser Gln Gly Glu Val Asn Val Pro
               820                 825                 830
   Val Ile Thr Thr Lys Leu Asp Thr Leu Arg Val His Leu Ala Ala Leu
           835                 840                 845
   Cys Gly Gly Leu Ser Asp Ser Leu Val Tyr Thr Leu Pro Ile Thr His
       850                 855                 860
   Pro Glu Lys Cys Leu Val Leu Val Ile Val Gly Asp Asn Lys Leu Asn
   865                 870                 875                 880
   Pro Gln Ile Arg Ser Glu Leu Val Ile Ser Arg Tyr Arg Tyr Ser Arg
   885 890 895
   Arg His Ile Arg Gly Val Val Ser Val Cys Ile Asp Gln Glu Gly Leu
   900 905 910
   Pro Asn Val Tyr Ser Thr Gly Ile Ala Arg His Lys Ile Tyr Glu Lys
   915 920 925
   Thr Val Leu Arg Tyr Arg Cys Lys Ile Ile Leu Val Lys Ile Ser Gly
   930 935 940
   Tyr Val Phe Gly Asn Asp Glu Leu Met Thr Lys Leu Leu Asn Val
   945 950 955
<210> 31
   <211> 3252
   <212> DNA
   <213> Artificial Sequence
<220>
   <223> Zera-BTV2 VP2
<400> 31
   atgagggtgt tgctcgttgc cctcgctctc ctggctctcg ctgcgagcgc cacctccacg      60
   catacaagcg gcggctgcgg ctgccagcca ccgccgccgg ttcatctacc gccgccggtg     120
   catctgccac ctccggttca cctgccacct ccggtgcatc tcccaccgcc ggtccacctg     180
   ccgccgccgg tccacctgcc accgccggtc catgtgccgc cgccggttca tctgccgccg     240
   ccaccatgcc actaccctac tcaaccgccc cggcctcagc ctcatcccca gccacaccca     300
   tgcccgtgcc aacagccgca tccaagcccg tgccagacca tggaagcggc ggcgaaaacc     360
   ggtatggatg aattaggaat accaatctac ggaaggaatt acccagaaca cttactcaaa     420
   ggatacgaat ttttaatcaa tacaggagtt aggtatccat ctcaaggagg tagacatgat     480
   gtttcaaaga ttcctgaaat gttcgcttat gatattaagg atgagggaat aagagaagca     540
   ttgaaataca tgccaactag gaatgatgga gttgtgttgc ctagaatcgt ggatattagt     600
   ttaaagggtt acgatatcag gaaatccgtt attgaagcta ataagagtaa ctccttccac     660
   actgatacaa attgggttca gtggatgatt aaggattcta tggatcaaca gccattgaaa     720
   atctcaattg atgaagagca tagtagagtt gtgcactccc tttttaactg tcaggttaag     780
   atcgatgcta agaaagcaga tactctctct tatcatcttg aagctattga ggatgctgaa     840
   aaagcatgtt tgcatacaag gggtcaattg tgcaatcatc ttgctaggat ggatcttttg      900
   catgctgcac aagaaattgc ttatgctatt aagcctactt accagcttat tgtgcactca     960
   gagagggctt ctacatcaga taactttgaa ctcggaagac aagatgttat tactcttaga    1020
   aggggacata ggatacagat gggagatgag gcatatacta agttgatgga aagacttgtt    1080
   aggttgacag tgcagggtaa cgttccaaga aaaatccaat ctgaaattga gcagcttgaa 1140
   gctattagaa ctaggtgggc aacaggaaga tacgatcctg ctcacatcaa cagtcaagat 1200
   ctctgtagga ttctttccag aattggtagg atcatgcttg atcaagaagc tgagccagtt 1260
   gatgaagata gtctttcctt gagattccag agggcactcg atgagaagtt taggcttaac 1320
   gattctgaga gaaacaaaat cttcgaacct aagtcacata gaaaggatga agataggttt 1380
   tatgttttac tcgctatagc tgcatctgat acttataatt caagaatctg gtggagtaac 1440
   ccataccctt gcttgagggg aactttaatt gctgcagaga caaagctcgg agatgtttat 1500
   tttactctta ggtcctggta cgattggtct gtgagatctt catatgttcc aagagaaagg 1560
   gagagagaaa cagagaagta catattctct aaaatcaatt tgttcgatta cgaagctgga 1620
   cctagttcca aggttattca ttgggagtat cagctttaca aaagggagag agttgtgact 1680
   ttggaaagag gtaacccatg tgatctttat cctgatgagg atgatgaagt gattataaca 1740
   aagttcgatg atgcaaagta ctcagagatg gttggagaaa tcattgatgg aggttggaat 1800
   gatgaagagt tcaagatgta taagcttttg caagagaagg gtaatgtttt gactatagat 1860
   ttcgaaaagg atactaagct caactctaca tcagaggttg tgcttccaga ttattacgga 1920
   aagtggattg ttgctcctat gttcaactct aaaatgagaa ttattgaaac tgagattgca 1980
   acaaacaggt cagatgatcc aatgataaag agaactttga aaccaatgac agatgatcct 2040
   gttgaattgc aaagatatac tttagctagg tactacgata ttagacctgg actcatgggt 2100
   agatctctta ataggactca aacacagtca acattcgatg caaaggttag tgagctttcc 2160
   gattacgaaa aagttgtgtc aagatttgga gtgataaaga aaccaactag gccttgcgtt 2220
   actttgacag gtagatacat cttagaaaag tacagtctcc tccttatcga tatcttgaag 2280
   taccacactg aggttgaaga gaacccacaa gaagagttca cacatcctag aatagatctt 2340
   caattcaagt ttaatggtaa cactttatct gatctcaacc aaacagttgt gtttattgtt 2400
   gattatcttc acgaaaagag aaactacgtt aggagtgtgt atgaggctag atacattata 2460
   tccaggataa gatcttcaac aggtgctgca aggatgagta tcttggaatt ctacttccca 2520
   acttttgcta ggctcatatc taatgcaaga gagcctacat acgtgaaaga tcttatggca 2580
   ttgaatttct taccattgct ttttattgtt ggagataaca tgatctataa gcatagacaa 2640
   tggtcaattc cactcctttt gtacactgat agggttaaag tgataccttt ggaagtggga 2700
   agttccaata acagacaggg tctcgttagt tatcttgagt atatgttttt ctttccttcc 2760
   cttgctgata ggacatcaaa ggtggatgag agtatgataa aggtttccaa ggaagttgtg 2820
   aactactaca tgaagactac aatctctgaa ggaggtgtta atttgaacgt tgtgtctact 2880
   aagtcactcc tctacgatat atatctttct tctgtgtgtg gaggtgtttc agatggtgtt 2940
   gtgtggtatt tgccaatcac acacccttac aaatgtgttg tggctattga agtgtgcgat 3000
   gatagagttc cagcaaggct tagatgcgat aggttaagac tcaggttccc tttgtctgct 3060
   caacatctta agggaatagt tgtgattcaa attaatgagg agggaggttt tgatgtttat 3120
   actgagggta ttgtgacaca cagggtttgt aagaaatcac ttttgaagca tgtttgtgat 3180
   atcgttttac tcaagttcca cggtcacgtt ttcggtaatg atgagatgct tacaaagtta 3240
   ttaaatgtgt ga 3252
<210> 32
   <211> 1083
   <212> PRT
   <213> Artificial sequence
<220>
   <223> Zera-BTV2 VP2
<400> 32
   Met Arg Val Leu Leu Val Ala Leu Ala Leu Leu Ala Leu Ala Ala Ser
   1 5 10 15
   Ala Thr Ser Thr His Thr Ser Gly Gly Cys Gly Cys Gln Pro Pro Pro
   20 25 30
   Pro Val His Leu Pro Pro Pro Val His Leu Pro Pro Pro Val His Leu
   35 40 45
   Pro Pro Pro Val His Leu Pro Pro Pro Val His Leu Pro Pro Pro Val
   50 55 60
   His Leu Pro Pro Pro Val His Val Pro Pro Pro Val His Leu Pro Pro
   65 70 75 80
   Pro Pro Cys His Tyr Pro Thr Gln Pro Pro Arg Pro Gln Pro His Pro
   85 90 95
   Gln Pro His Pro Cys Pro Cys Gln Gln Pro His Pro Ser Pro Cys Gln
   100 105 110
   Thr Met Glu Ala Ala Ala Lys Thr Gly Met Asp Glu Leu Gly Ile Pro
   115 120 125
   Ile Tyr Gly Arg Asn Tyr Pro Glu His Leu Leu Lys Gly Tyr Glu Phe
   130 135 140
   Leu Ile Asn Thr Gly Val Arg Tyr Pro Ser Gln Gly Gly Arg His Asp
   145 150 155 160
   Val Ser Lys Ile Pro Glu Met Phe Ala Tyr Asp Ile Lys Asp Glu Gly
   165 170 175
   Ile Arg Glu Ala Leu Lys Tyr Met Pro Thr Arg Asn Asp Gly Val Val
   180 185 190
   Leu Pro Arg Ile Val Asp Ile Ser Leu Lys Gly Tyr Asp Ile Arg Lys
   195 200 205
   Ser Val Ile Glu Ala Asn Lys Ser Asn Ser Phe His Thr Asp Thr Asn
   210 215 220
   Trp Val Gln Trp Met Ile Lys Asp Ser Met Asp Gln Gln Pro Leu Lys
   225 230 235 240
   Ile Ser Ile Asp Glu Glu His Ser Arg Val Val His Ser Leu Phe Asn
   245 250 255
   Cys Gln Val Lys Ile Asp Ala Lys Lys Ala Asp Thr Leu Ser Tyr His
   260 265 270
   Leu Glu Ala Ile Glu Asp Ala Glu Lys Ala Cys Leu His Thr Arg Gly
   275 280 285
   Gln Leu Cys Asn His Leu Ala Arg Met Asp Leu Leu His Ala Ala Gln
   290 295 300
   Glu Ile Ala Tyr Ala Ile Lys Pro Thr Tyr Gln Leu Ile Val His Ser
   305 310 315 320
   Glu Arg Ala Ser Thr Ser Asp Asn Phe Glu Leu Gly Arg Gln Asp Val
   325 330 335
   Ile Thr Leu Arg Arg Gly His Arg Ile Gln Met Gly Asp Glu Ala Tyr
   340 345 350
   Thr Lys Leu Met Glu Arg Leu Val Arg Leu Thr Val Gln Gly Asn Val
   355 360 365
   Pro Arg Lys Ile Gln Ser Glu Ile Glu Gln Leu Glu Ala Ile Arg Thr
   370 375 380
   Arg Trp Ala Thr Gly Arg Tyr Asp Pro Ala His Ile Asn Ser Gln Asp
   385 390 395 400
   Leu Cys Arg Ile Leu Ser Arg Ile Gly Arg Ile Met Leu Asp Gln Glu
   405 410 415
   Ala Glu Pro Val Asp Glu Asp Ser Leu Ser Leu Arg Phe Gln Arg Ala
               420                 425                 430
   Leu Asp Glu Lys Phe Arg Leu Asn Asp Ser Glu Arg Asn Lys Ile Phe
           435                 440                 445
   Glu Pro Lys Ser His Arg Lys Asp Glu Asp Arg Phe Tyr Val Leu Leu
       450                 455                 460
   Ala Ile Ala Ala Ser Asp Thr Tyr Asn Ser Arg Ile Trp Trp Ser Asn
   465                 470                 475                 480
   Pro Tyr Pro Cys Leu Arg Gly Thr Leu Ile Ala Ala Glu Thr Lys Leu
                   485                 490                 495
   Gly Asp Val Tyr Phe Thr Leu Arg Ser Trp Tyr Asp Trp Ser Val Arg
               500                 505                 510
   Ser Ser Tyr Val Pro Arg Glu Arg Glu Arg Glu Thr Glu Lys Tyr Ile
           515                 520                 525
   Phe Ser Lys Ile Asn Leu Phe Asp Tyr Glu Ala Gly Pro Ser Ser Lys
       530                 535                 540
   Val Ile His Trp Glu Tyr Gln Leu Tyr Lys Arg Glu Arg Val Val Thr
   545                 550                 555                 560
   Leu Glu Arg Gly Asn Pro Cys Asp Leu Tyr Pro Asp Glu Asp Asp Glu
                   565                 570                 575
   Val Ile Ile Thr Lys Phe Asp Asp Ala Lys Tyr Ser Glu Met Val Gly
               580                 585                 590
   Glu Ile Ile Asp Gly Gly Trp Asn Asp Glu Glu Phe Lys Met Tyr Lys
           595                 600                 605
   Leu Leu Gln Glu Lys Gly Asn Val Leu Thr Ile Asp Phe Glu Lys Asp
       610                 615                 620
   Thr Lys Leu Asn Ser Thr Ser Glu Val Val Leu Pro Asp Tyr Tyr Gly
   625                 630                 635                 640
   Lys Trp Ile Val Ala Pro Met Phe Asn Ser Lys Met Arg Ile Ile Glu
                   645                 650                 655
   Thr Glu Ile Ala Thr Asn Arg Ser Asp Asp Pro Met Ile Lys Arg Thr
               660                 665                 670
   Leu Lys Pro Met Thr Asp Asp Pro Val Glu Leu Gln Arg Tyr Thr Leu
   675 680 685
   Ala Arg Tyr Tyr Asp Ile Arg Pro Gly Leu Met Gly Arg Ser Leu Asn
   690 695 700
   Arg Thr Gln Thr Gln Ser Thr Phe Asp Ala Lys Val Ser Glu Leu Ser
   705 710 715 720
   Asp Tyr Glu Lys Val Val Ser Arg Phe Gly Val Ile Lys Lys Pro Thr
   725 730 735
   Arg Pro Cys Val Thr Leu Thr Gly Arg Tyr Ile Leu Glu Lys Tyr Ser
   740 745 750
   Leu Leu Leu Ile Asp Ile Leu Lys Tyr His Thr Glu Val Glu Glu Asn
   755 760 765
   Pro Gln Glu Glu Phe Thr His Pro Arg Ile Asp Leu Gln Phe Lys Phe
   770 775 780
   Asn Gly Asn Thr Leu Ser Asp Leu Asn Gln Thr Val Val Phe Ile Val
   785 790 795 800
   Asp Tyr Leu His Glu Lys Arg Asn Tyr Val Arg Ser Val Tyr Glu Ala
   805 810 815
   Arg Tyr Ile Ile Ser Arg Ile Arg Ser Ser Thr Gly Ala Ala Arg Met
   820 825 830
   Ser Ile Leu Glu Phe Tyr Phe Pro Thr Phe Ala Arg Leu Ile Ser Asn
   835 840 845
   Ala Arg Glu Pro Thr Tyr Val Lys Asp Leu Met Ala Leu Asn Phe Leu
   850 855 860
   Pro Leu Leu Phe Ile Val Gly Asp Asn Met Ile Tyr Lys His Arg Gln
   865 870 875 880
   Trp Ser Ile Pro Leu Leu Leu Tyr Thr Asp Arg Val Lys Val Ile Pro
   885 890 895
   Leu Glu Val Gly Ser Ser Asn Asn Arg Gln Gly Leu Val Ser Tyr Leu
   900 905 910
   Glu Tyr Met Phe Phe Phe Pro Ser Leu Ala Asp Arg Thr Ser Lys Val
   915 920 925
   Asp Glu Ser Met Ile Lys Val Ser Lys Glu Val Val Asn Tyr Tyr Met
   930 935 940
   Lys Thr Thr Ile Ser Glu Gly Gly Val Asn Leu Asn Val Val Ser Thr
   945 950 955 960
   Lys Ser Leu Leu Tyr Asp Ile Tyr Leu Ser Ser Val Cys Gly Gly Val
   965 970 975
   Ser Asp Gly Val Val Trp Tyr Leu Pro Ile Thr His Pro Tyr Lys Cys
   980 985 990
   Val Val Ala Ile Glu Val Cys Asp Asp Arg Val Pro Ala Arg Leu Arg
   995 1000 1005
   Cys Asp Arg Leu Arg Leu Arg Phe Pro Leu Ser Ala Gln His Leu
   1010 1015 1020
   Lys Gly Ile Val Val Ile Gln Ile Asn Glu Glu Gly Gly Phe Asp
   1025 1030 1035
   Val Tyr Thr Glu Gly Ile Val Thr His Arg Val Cys Lys Lys Ser
   1040 1045 1050
   Leu Leu Lys His Val Cys Asp Ile Val Leu Leu Lys Phe His Gly
   1055 1060 1065
   His Val Phe Gly Asn Asp Glu Met Leu Thr Lys Leu Leu Asn Val
   1070 1075 1080
<210> 33
   <211> 3234
   <212> DNA
   <213> Artificial sequence
<220>
   <223> Zera-BTV4 VP2
<400> 33
   atgagggtgt tgctcgttgc cctcgctctc ctggctctcg ctgcgagcgc cacctccacg     60
   catacaagcg gcggctgcgg ctgccagcca ccgccgccgg ttcatctacc gccgccggtg    120
   catctgccac ctccggttca cctgccacct ccggtgcatc tcccaccgcc ggtccacctg    180
   ccgccgccgg tccacctgcc accgccggtc catgtgccgc cgccggttca tctgccgccg    240
   ccaccatgcc actaccctac tcaaccgccc cggcctcagc ctcatcccca gccacaccca    300
   tgcccgtgcc aacagccgca tccaagcccg tgccagacca tggaagcggc ggcgaaaacc    360
   ggtatggaag agttcgttat acctgtgttc tccgaaaggg aaatacctta cgcattaatc 420
   aatagatatc ctttagcaat acaaactgat gttaagatag tggatgttga tggataccat 480
   aatcttgcta aaatcccaga aagtgatatg gtggatgttc ctaagttaga tattgtttcc 540
   gcactcaatt acaaaccaac aagaaacgat ggaattgttg tgcctaggct tttggatatc 600
   actttgaagg cttacgatga tagaaagtct gtgaaaaatg caaggggtgt tgattttatg 660
   acagatgcta agtggatgaa atgggcaatt gatgatagga tggatatcca accacttaag 720
   atcactttgg atgagcatta cgctgtgaac caccagcttt ttaactgtat cgttaaagca 780
   aagactgcta acgcagatac aatctattac gattacttcc ctcttgaaga tagagctaag 840
   aaatgcaatc acacaaacct tgagttactc aggtctttga ctacaataga agcattccat 900
   gttttgcaag gagctgcata cagtctcaag tccaactacg atctcatagc taactctgaa 960
   agagagtcac ttgaagaaag ttacccaatt ggttcagaaa agtgggttca tcttactaga 1020
   agggctaaga tcggaaactc tggtttgtca tacaacaggt tcatttcttc aatggtgcag 1080
   gttgtggtta gaggaaaagt tcctgatatt ataaggggtg agattacaca acttaataga 1140
   atcaggactg agtggattaa cgcttcttat gatagaacaa ggattagagc attagagctc 1200
   tgtaacatac tttcagctat cggaagaaag atgatggatg cacacgaaga gccaaaagat 1260
   gagatggatc ttagtacaag gttccagttc aaattggatg agaagtttaa tactagtgat 1320
   tccgaacatg ttaacatttt cagaacttca ggagctgcaa caaatgaggg aaggttttac 1380
   gctttgattg caatagctgc aactgataca caaaagggaa gggtttggag aacaaaccca 1440
   tatccttgtt taagaggagc tctcattgca tcagaatgcg agcttggaga tgtgtattac 1500
   actttgaggc acgtttacag atggagtctt aggcctgaat atggtcaaag ggagagacag 1560
   cttgaagata ataagtatgt tttcggaaga gttaacttat tcgattctga tctcgctgtg 1620
   ggagatcagg ttattcattg gagatatgaa ataactgagc cagttaaaac tacatacgat 1680
   aatggttaca tatgtaaccc tgaagagaaa gatgatgagc ttttgtgcaa gattgatgat 1740
   gaaagatata aggaaatgat ggagaggctt atagagggag gttgggatca agaaaggttt 1800
   aagcttcaca atatcttgac tgagccaaac ttactcacaa tcgatttcga aaaggatgct 1860
   taccttaatt ctaggtctga attggttttc ccaaactact tcgataagtg gattaactca 1920
   cctatgttta acgctaggct tagaataaca catggagaga tcggtagttc caaaactgca 1980
   gatccttgga atagaagggt ggtttacgga tatatcaaga caagtatcga atccctcgat 2040
   tacactctcg gaaaatatta cgatatcaga cttcagttgt ttggagatac tttgagtagg 2100
   aagcaaacac agtccgcagt ttttacttac cttagtggtc aagatgattt cccaactctt 2160
   acaaactatt tgaagtccga ggctgtgtgt cctcacgctg gaggtgcagt ttacacattc 2220
   agaaagatgg ctctctctct cattgcaaat tacgaaaagt taaacccaga aatgcatgag 2280
   ggactcgaac accaaactta cgtgcatcct agtgttaaca ctacatacca aaagcaggtt 2340
   aaagatatga aggatttctc ccaattgatc tgtttcatca tcgattgcat cttcgagaag 2400
   agaatccaga ttaggggagt gggtgaagct agaaggataa tctaccttat ccaaaactct 2460
   atcggtctcc aaagacagga ggttctcaag aggacttttc caaattttct tatgagactt 2520
   tttaaattga gagaagtgaa gaggatctgt gatctttcag ttattaattt ccttcctctt 2580
   ttgtttttgg ttcaagataa catatcttat tggcatagac agtggtcagt tcctatgata 2640
   ttattcgatg atgctgttag gctcatccct gtggaggttg gagcttacgc aaatagattt 2700
   ggtttcaagt ctttctacaa cttcataagg tttcacccag gagattcaaa gaaaaagcag 2760
   gatgctgatg atatgcataa ggagtacggt gttgcatgct tcgaatacta catgaacaca 2820
   aagatatctc aaggaggtgt gaacgttcct gtggttactt caaaattaga tacactcaag 2880
   atccaccttg ctagtctttg tttgggatta gcagattcca ttgtgtatac tttgccagtt 2940
   gcacatccta aaaagtgcat agttcttatc gtggttggag atgataagtt ggatccacaa 3000
   gtgagatccg aacaggttct ttctaaatat tactattcaa gaaggcacat ttgcggagtg 3060
   gttgctgtgt ctgttggaca agagggtcaa ttgcaggtgt actcttcagg tatagttagg 3120
   catagaatct gtgaaaagtc aatcctcaag tataagtgca aggtggttct tgttaggatg 3180
   ccaggacacg tttttggaaa tgatgaactt atgactaaat tattgaatgt gtga 3234
<210> 34
   <211> 1077
   <212> PRT
   <213> Artificial Sequence
<220>
   <223> Zera-BTV4 VP2
<400> 34
   Met Arg Val Leu Leu Val Ala Leu Ala Leu Leu Ala Leu Ala Ala Ser
   1               5                   10                  15
   Ala Thr Ser Thr His Thr Ser Gly Gly Cys Gly Cys Gln Pro Pro Pro
               20                  25                  30
   Pro Val His Leu Pro Pro Pro Val His Leu Pro Pro Pro Val His Leu
           35                  40                  45
   Pro Pro Pro Val His Leu Pro Pro Pro Val His Leu Pro Pro Pro Val
       50                  55                  60
   His Leu Pro Pro Pro Val His Val Pro Pro Pro Val His Leu Pro Pro
   65                  70                  75                  80
   Pro Pro Cys His Tyr Pro Thr Gln Pro Pro Arg Pro Gln Pro His Pro
                   85                  90                  95
   Gln Pro His Pro Cys Pro Cys Gln Gln Pro His Pro Ser Pro Cys Gln
               100                 105                 110
   Thr Met Glu Ala Ala Ala Lys Thr Gly Met Glu Glu Phe Val Ile Pro
           115                 120                 125
   Val Phe Ser Glu Arg Glu Ile Pro Tyr Ala Leu Ile Asn Arg Tyr Pro
       130                 135                 140
   Leu Ala Ile Gln Thr Asp Val Lys Ile Val Asp Val Asp Gly Tyr His
   145                 150                 155                 160
   Asn Leu Ala Lys Ile Pro Glu Ser Asp Met Val Asp Val Pro Lys Leu
                   165                 170                 175
   Asp Ile Val Ser Ala Leu Asn Tyr Lys Pro Thr Arg Asn Asp Gly Ile
               180                 185                 190
   Val Val Pro Arg Leu Leu Asp Ile Thr Leu Lys Ala Tyr Asp Asp Arg
           195                 200                 205
   Lys Ser Val Lys Asn Ala Arg Gly Val Asp Phe Met Thr Asp Ala Lys
       210                 215                 220
   Trp Met Lys Trp Ala Ile Asp Asp Arg Met Asp Ile Gln Pro Leu Lys
   225                 230                 235                 240
   Ile Thr Leu Asp Glu His Tyr Ala Val Asn His Gln Leu Phe Asn Cys
                   245                 250                 255
   Ile Val Lys Ala Lys Thr Ala Asn Ala Asp Thr Ile Tyr Tyr Asp Tyr
               260                 265                 270
   Phe Pro Leu Glu Asp Arg Ala Lys Lys Cys Asn His Thr Asn Leu Glu
           275                 280                 285
   Leu Leu Arg Ser Leu Thr Thr Ile Glu Ala Phe His Val Leu Gln Gly
       290                 295                 300
   Ala Ala Tyr Ser Leu Lys Ser Asn Tyr Asp Leu Ile Ala Asn Ser Glu
   305                 310                 315                 320
   Arg Glu Ser Leu Glu Glu Ser Tyr Pro Ile Gly Ser Glu Lys Trp Val
   325 330 335
   His Leu Thr Arg Arg Ala Lys Ile Gly Asn Ser Gly Leu Ser Tyr Asn
   340 345 350
   Arg Phe Ile Ser Ser Met Val Gln Val Val Val Arg Gly Lys Val Pro
   355 360 365
   Asp Ile Ile Arg Gly Glu Ile Thr Gln Leu Asn Arg Ile Arg Thr Glu
   370 375 380
   Trp Ile Asn Ala Ser Tyr Asp Arg Thr Arg Ile Arg Ala Leu Glu Leu
   385 390 395 400
   Cys Asn Ile Leu Ser Ala Ile Gly Arg Lys Met Met Asp Ala His Glu
   405 410 415
   Glu Pro Lys Asp Glu Met Asp Leu Ser Thr Arg Phe Gln Phe Lys Leu
   420 425 430
   Asp Glu Lys Phe Asn Thr Ser Asp Ser Glu His Val Asn Ile Phe Arg
   435 440 445
   Thr Ser Gly Ala Ala Thr Asn Glu Gly Arg Phe Tyr Ala Leu Ile Ala
   450 455 460
   Ile Ala Ala Thr Asp Thr Gln Lys Gly Arg Val Trp Arg Thr Asn Pro
   465 470 475 480
   Tyr Pro Cys Leu Arg Gly Ala Leu Ile Ala Ser Glu Cys Glu Leu Gly
   485 490 495
   Asp Val Tyr Tyr Thr Leu Arg His Val Tyr Arg Trp Ser Leu Arg Pro
   500 505 510
   Glu Tyr Gly Gln Arg Glu Arg Gln Leu Glu Asp Asn Lys Tyr Val Phe
   515 520 525
   Gly Arg Val Asn Leu Phe Asp Ser Asp Leu Ala Val Gly Asp Gln Val
   530 535 540
   Ile His Trp Arg Tyr Glu Ile Thr Glu Pro Val Lys Thr Thr Tyr Asp
   545 550 555 560
   Asn Gly Tyr Ile Cys Asn Pro Glu Glu Lys Asp Asp Glu Leu Leu Cys
   565 570 575
   Lys Ile Asp Asp Glu Arg Tyr Lys Glu Met Met Glu Arg Leu Ile Glu
               580                 585                 590
   Gly Gly Trp Asp Gln Glu Arg Phe Lys Leu His Asn Ile Leu Thr Glu
           595                 600                 605
   Pro Asn Leu Leu Thr Ile Asp Phe Glu Lys Asp Ala Tyr Leu Asn Ser
       610                 615                 620
   Arg Ser Glu Leu Val Phe Pro Asn Tyr Phe Asp Lys Trp Ile Asn Ser
   625                 630                 635                 640
   Pro Met Phe Asn Ala Arg Leu Arg Ile Thr His Gly Glu Ile Gly Ser
                   645                 650                 655
   Ser Lys Thr Ala Asp Pro Trp Asn Arg Arg Val Val Tyr Gly Tyr Ile
               660                 665                 670
   Lys Thr Ser Ile Glu Ser Leu Asp Tyr Thr Leu Gly Lys Tyr Tyr Asp
           675                 680                 685
   Ile Arg Leu Gln Leu Phe Gly Asp Thr Leu Ser Arg Lys Gln Thr Gln
       690                 695                 700
   Ser Ala Val Phe Thr Tyr Leu Ser Gly Gln Asp Asp Phe Pro Thr Leu
   705                 710                 715                 720
   Thr Asn Tyr Leu Lys Ser Glu Ala Val Cys Pro His Ala Gly Gly Ala
                   725                 730                 735
   Val Tyr Thr Phe Arg Lys Met Ala Leu Ser Leu Ile Ala Asn Tyr Glu
               740                 745                 750
   Lys Leu Asn Pro Glu Met His Glu Gly Leu Glu His Gln Thr Tyr Val
           755                 760                 765
   His Pro Ser Val Asn Thr Thr Tyr Gln Lys Gln Val Lys Asp Met Lys
       770                 775                 780
   Asp Phe Ser Gln Leu Ile Cys Phe Ile Ile Asp Cys Ile Phe Glu Lys
   785                 790                 795                 800
   Arg Ile Gln Ile Arg Gly Val Gly Glu Ala Arg Arg Ile Ile Tyr Leu
                   805                 810                 815
   Ile Gln Asn Ser Ile Gly Leu Gln Arg Gln Glu Val Leu Lys Arg Thr
               820                 825                 830
   Phe Pro Asn Phe Leu Met Arg Leu Phe Lys Leu Arg Glu Val Lys Arg
   835 840 845
   Ile Cys Asp Leu Ser Val Ile Asn Phe Leu Pro Leu Leu Phe Leu Val
   850 855 860
   Gln Asp Asn Ile Ser Tyr Trp His Arg Gln Trp Ser Val Pro Met Ile
   865 870 875 880
   Leu Phe Asp Asp Ala Val Arg Leu Ile Pro Val Glu Val Gly Ala Tyr
   885 890 895
   Ala Asn Arg Phe Gly Phe Lys Ser Phe Tyr Asn Phe Ile Arg Phe His
   900 905 910
   Pro Gly Asp Ser Lys Lys Lys Gln Asp Ala Asp Asp Met His Lys Glu
   915 920 925
   Tyr Gly Val Ala Cys Phe Glu Tyr Tyr Met Asn Thr Lys Ile Ser Gln
   930 935 940
   Gly Gly Val Asn Val Pro Val Val Thr Ser Lys Leu Asp Thr Leu Lys
   945 950 955 960
   Ile His Leu Ala Ser Leu Cys Leu Gly Leu Ala Asp Ser Ile Val Tyr
   965 970 975
   Thr Leu Pro Val Ala His Pro Lys Lys Cys Ile Val Leu Ile Val Val
   980 985 990
   Gly Asp Asp Lys Leu Asp Pro Gln Val Arg Ser Glu Gln Val Leu Ser
   995 1000 1005
   Lys Tyr Tyr Tyr Ser Arg Arg His Ile Cys Gly Val Val Ala Val
   1010 1015 1020
   Ser Val Gly Gln Glu Gly Gln Leu Gln Val Tyr Ser Ser Gly Ile
   1025 1030 1035
   Val Arg His Arg Ile Cys Glu Lys Ser Ile Leu Lys Tyr Lys Cys
   1040 1045 1050
   Lys Val Val Leu Val Arg Met Pro Gly His Val Phe Gly Asn Asp
   1055 1060 1065
   Glu Leu Met Thr Lys Leu Leu Asn Val
   1070 1075
<210> 35
   <211> 957
   <212> PRT
   <213> Artificial sequence
<220>
   <223> BTV-27 VP2 consensus sequence
<400> 35
   Met Asp Glu Leu Val Ile Pro Ile Phe Ser Arg Gln Asp Met Pro Tyr
   1               5                   10                  15
   Ala Leu Ile Ser His Tyr Pro Leu Ala Val Gln Ile Asp Lys Lys Ile
               20                  25                  30
   Gln Asn Asp Arg Glu Phe His Asp Leu Thr Lys Ile Pro Gln Ser Asn
           35                  40                  45
   Met Ile Asp Val Ser Glu Tyr Gly Ile Val Lys Ala Leu Asp Tyr Arg
       50                  55                  60
   Pro Thr Arg Asn Asp Gly Ile Val Val Pro Arg Leu Leu Asp Ile Ala
   65                  70                  75                  80
   Leu Cys Ala Tyr Asp Lys Arg Arg Ser Ile Lys Asn Pro Arg Gly Leu
                   85                  90                  95
   Glu Phe Met Thr Asp Ala Glu Trp Met Arg Trp Ala Ile Asp Asp Arg
               100                 105                 110
   Met Asp Ile Gln Pro Leu Lys Ile Asp Ile Asp Asp Glu Cys Thr Val
           115                 120                 125
   Asn His Gln Leu Phe Asn Cys Asn Ile Lys Ile Arg Ser Arg Asn Val
       130                 135                 140
   Asp Thr Val Tyr Tyr Asp Tyr His Pro Leu Glu Lys Gly Ser Thr Lys
   145                 150                 155                 160
   Cys Ile His Ala Asn Ile Glu Leu Met Arg Ser Leu Thr Leu Val Glu
                   165                 170                 175
   Met Tyr His Val Ile Gln Gly Thr Ala Tyr Ala Leu Lys Thr Asn Phe
               180                 185                 190
   Arg Leu Val Ala Thr Ala Glu Arg Ser Asp Arg Asn Ala Arg Tyr Asn
           195                 200                 205
   Asp Val Gly Leu Arg Trp Arg Ala Leu Arg Gln Asn Gln Glu Ile Gly
       210                 215                 220
   Tyr Glu Gly Val Ala Tyr Asn Lys Phe Met Asp Gly Leu Val Gln Val
   225                 230                 235                 240
   Glu Val Gln Gly Lys Cys Ser Glu Asp Ile Ala Ser Glu Met Gly Asn
                   245                 250                 255
   Leu Ser Arg Ile Lys Glu Lys Trp Ile Glu Ser Gly Arg Ser Gly Ser
               260                 265                 270
   Val Ile Tyr Ala Ser Asp Leu Cys Thr Gln Phe Ser Ser Ile Gly Arg
           275                 280                 285
   Lys Met Thr Asp Met His Glu Glu Arg Lys Asp Glu Glu Gly Ile Ser
       290                 295                 300
   Arg Arg Phe Gln Ile Glu Leu Asp Asp Ile Phe Ser Thr Ala Arg Ala
   305                 310                 315                 320
   Glu Asn Arg Asn Val Leu Asn Gln Asn His Ala Lys Ala Asn Asp Ala
                   325                 330                 335
   Asn Lys Phe Tyr Ala Leu Ile Met Ile Ala Ala Ser Asp Thr Gln Lys
               340                 345                 350
   Gly Arg Val Trp Arg Thr Asn Pro Tyr Pro Cys Leu Arg Gly Ala Leu
           355                 360                 365
   Val Ala Ala Glu Cys Val Met Gly Asp Val Tyr Tyr Thr Leu Arg Thr
       370                 375                 380
   Val Phe Gln Trp Ser Val Arg Ala Thr Tyr Ser Thr Asn Glu Arg Ser
   385                 390                 395                 400
   Leu Glu Asn Asn Arg Tyr Val Phe Ser Arg Val Asn Leu Phe Asp Thr
                   405                 410                 415
   Asp Leu Pro Ile Arg Ser Asn Val Ile Phe Trp Cys Tyr Glu Glu Thr
               420                 425                 430
   Glu Pro Val Glu Thr Thr Tyr Asp Asn Gly Tyr Ile Cys Lys Ser Gln
           435                 440                 445
   Glu Lys Glu Gly Glu Glu Leu Cys Lys Ile Asp Asp Asn Leu Tyr Asn
       450                 455                 460
   Gly Met Arg Asp Asn Ile Ile Gln His Gly Trp Arg Gln Glu Ser Leu
   465                 470                 475                 480
   Lys Leu His Asn Ile Leu Thr Gln Pro Asn Leu Leu Thr Ile Asp Phe
                   485                 490                 495
   Glu Lys Asp Ala Tyr Ile Thr Ser Ala Ser Asp Leu Val Leu Pro Ser
               500                 505                 510
   Tyr Phe Asn Lys Trp Ile Asn Ser Pro Met Phe Asn Ala Arg Leu Arg
           515                 520                 525
   Ile Thr Asn Gly Glu Ile Gln Ser Asp Lys Thr Ser Asp Pro Trp Asn
       530                 535                 540
   Lys Arg Val Ala Glu Gly Thr Ile Arg Ile Ser Ala Glu Ser Leu Gly
   545                 550                 555                 560
   Tyr Ala Leu Gly Arg Phe Tyr Asp Gln Arg Leu Gln Phe Val Asp Asp
                   565                 570                 575
   Val Leu Ser Thr Arg Gln Ile Lys Ser Ser Ala Leu Arg Tyr Met Cys
               580                 585                 590
   Asn Leu Lys Asp Phe Ser Ala Leu Thr His Tyr Ala Ala Gly Gln Asp
           595                 600                 605
   Ile Cys Pro His Ala Gly Gly Ala Met Tyr Thr Phe Arg Arg Ala Ala
       610                 615                 620
   Leu Glu Val Ile Thr Asn Tyr Glu Lys Leu Ser Pro Met Asp His Asp
   625                 630                 635                 640
   Gly Tyr Glu His Gln Val Tyr Arg His Pro Val Val Asp Tyr Ala Arg
                   645                 650                 655
   Leu Asp Asp Val Phe Lys Met Glu Asp Leu Ser Gln Leu Ile Cys Phe
               660                 665                 670
   Ile Phe Asp Ser Ile Phe Glu Arg Arg Asn Ser Ile Arg Glu Ile Gly
           675                 680                 685
   Glu Ala Arg Met Ile Ile Tyr Gln Ile Gln Ser Thr Val Gly Glu Arg
       690                 695                 700
   Arg Cys Asp Val Leu Ala Arg Glu Phe Pro Thr Phe Tyr Lys Trp Phe
   705                 710                 715                 720
   Phe Lys Leu Arg Asn Ile Lys Phe Ile Arg Asp Leu Asn Val Ile Asn
                   725                 730                 735
   Phe Leu Pro Leu Leu Leu Leu Val Arg Asp Asn Ala Ser Tyr Arg His
               740                 745                 750
   Arg Gln Trp Ser Ile Pro Met Ile Phe Tyr Asp Glu Glu Ile Lys Leu
           755                 760                 765
   Ile Pro Val Glu Val Gly Ala Tyr Ala Asn Arg Phe Gly Phe Lys Ser
       770                 775                 780
   Phe Phe Asn Phe Ile Arg Phe His Pro Gly Asp Ala Arg Lys Lys Gln
   785                 790                 795                 800
   Glu Ala Asn Met Tyr His Arg Ala Tyr Gly Ser Val Cys Tyr Asp Tyr
                   805                 810                 815
   Tyr Ile Asn Thr Thr Ile Ser Gln Gly Glu Val Ser Gly Pro Ile Ile
               820                 825                 830
   Thr Thr Lys Leu Asp Thr Leu Lys Val His Leu Ala Ala Leu Cys Gly
           835                 840                 845
   Gly Leu Ser Asp Ser Leu Val Tyr Thr Leu Pro Ile Thr His Pro Arg
       850                 855                 860
   Lys Cys Leu Ala Leu Ile Ile Ile Gly Asp Asp Lys Met Asp Pro Gln
   865                 870                 875                 880
   Thr Arg Ser Glu Leu Val Thr Ser Arg Tyr Arg Tyr Ser Arg Leu His
                   885                 890                 895
   Ile Arg Gly Val Val Ser Ile Cys Ile Asn Gln Asp Gly Gln Phe Asn
               900                 905                 910
   Val Tyr Ser Ser Gly Ile Ala Arg His Lys Val Cys Glu Lys Ala Val
           915                 920                 925
   Leu Arg Tyr Arg Cys Lys Val Val Leu Val Lys Ile Pro Gly Tyr Val
       930                 935                 940
   Phe Gly Asn Asp Glu Leu Met Thr Lys Leu Leu Asn Val
   945                 950                 955